# EUROPEAN PATENT SPECIFICATION

(11) **EP 0 481 522 B1**
(45) Date of publication and mention of the grant of the patent: **29.12.1997**
(21) Application number: 91117849.9
(22) Date of filing: 18.10.1991
(51) Int. Cl.: C07D 471/04, C07D 487/04, C07K 5/06, A61K 38/55

(54) **Novel mercaptoacetylamide derivatives useful as inhibitors of enkephalinase and ace**
Neue Mercaptoacetylamid-Derivate zur Verwendung als Enkephalinase- und ACE-Hemmer
Nouveaux dérivés de mercaptoacétylamides utiles comme inhibiteurs de l'enképhalinase et de l'enzyme de conversion de l'angiotensine

(30) Priority: 18.10.1990 US 600052; 25.07.1991 US 735496
(43) Date of publication of application: 22.04.1992
(73) Proprietor: MERRELL PHARMACEUTICALS INC., Cincinnati, Ohio 45215-6300 (US)
(72) Inventor: Flynn, Gary A., Cincinnati, Ohio 45243 (US); Warshawsky, Alan M., Cincinnati, Ohio 45236 (US); Mehdi, Shujaath, Cincinnati, Ohio 45213 (US); Bey, Philippe, Cincinnati, Ohio 45242 (US); Beight, Douglas W., Cincinnati, Ohio 45246 (US); Giroux, Eugene L., Cincinnati, Ohio 45249 (US); Burkholder, Timothy P., Fairfield, Ohio 45104 (US)
(74) Representative: VOSSIUS & PARTNER

(56) References cited:
- EP-A- 0 249 223
- EP-A- 0 249 224
- US-A- 4 584 294
- US-A- 4 824 832
- TETRAHEDRON LETTERS. vol. 31, no. 6, 1990, OXFORD GB pages 815 - 818; G.A. FLYNN ET AL.: 'The conversion of a diazolactam to an alpha-methylenelactam; an entrance to new conformationally restricted inhibitors of angiotensin-converting enzyme'

## Description

Enkephalinase or, more specifically, endopeptidase-24.11, is a mammalian ectoenzyme which is involved in the metabolic degradation of certain circulating regulatory peptides. This enzyme, which is a Zn⁺²-metallopeptidase, exerts its effect by cleaving the extracellular peptides at the amino group of hydrophobic residues and thus inactivates the peptides as regulatory messengers.

Enkephalinase is involved in the metabolic degradation of a variety of circulating regulatory peptides including endorphins, such as β-endorphin and the enkephalins, atrial natriuretic peptide (ANP), and other circulating regulatory peptides.

Endorphins are naturally-occurring polypeptides which bind to opiate receptors in various areas of the brain and thereby provide an analgesic effect by raising the pain threshold. Endorphins occur in various forms including α-endorphin, β-endorphin, γ-endorphin as well as the enkephalins. The enkephalins, i.e., Met-enkephalin and Leu-enkephalin, are pentapeptides which occur in nerve endings of brain tissue, spinal cord and the gastrointestinal tract. Like the other endorphins, the enkephalins provide an analgesic effect by binding to the opiate receptors in the brain. By inhibiting enkephalinase, the metabolic degradation of the naturally-occurring endorphins and enkephalins are inhibited, thereby providing a potent endorphin- or enkephalin-mediated analgesic effect. Inhibition of enkephalinase would therefore be useful in a patient suffering from acute or chronic pain. Inhibition of enkephalinase would also be useful in providing an antidepressant effect and in providing a reduction in severity of withdrawal symptoms associated with termination of opiate or morphine administration.

ANP refers to a family of naturally-occurring peptides which are involved in the homeostatic regulation of blood pressure, as well as sodium and water levels. ANP have been found to vary in length from about 21 to about 126 amino acids with a common structural feature being one or more disulfide-looped sequences of 17 amino acids with various amino- and carboxy-terminal sequences attached to the cystine moiety. ANP have been found to bind to specific binding sites in various tissues including kidney, adrenal, aorta, and vascular smooth muscle with affinities ranging from about 50 pico-molar (pM) to about 500 nano-molar (nM) [Needleman, *Hypertension* 7, 469 (1985)]. In addition, it is believed that ANP binds to specific receptors in the brain and possibly serves as a neuromodulator as well as a conventional peripheral hormone.

The biological properties of ANP involve potent diuretic/natriuretic and vasodilatory/hypotensive effects as well as an inhibitory effect on renin and aldosterone secretion [deBold, *Science* 230, 767 (1985)]. By inhibiting enkephalinase, the metabolic degradation of the naturally-occurring ANP is inhibited, thereby providing potent ANP-mediated diuretic, natriuretic, hypotensive, hypoaldosteronemic effects. Inhibition of enkephalinase would therefore be useful in a patient suffering from disease states characterized by abnormalities in fluid, electrolyte, blood pressure, intraocular pressure, renin, or aldosterone homeostasis, such as, but not limited to, hypertension, renal diseases, hyperaldosteronemia, cardiac hypertrophy, glaucoma and congestive heart failure.

In addition, the compounds of the present invention are inhibitors of Angiotensin-Converting Enzyme (ACE). ACE is a peptidyl dipeptidase which catalyzes the conversion of angiotensin I to angiotensin II. Angiotensin II is a vasoconstrictor which also stimulates aldosterone secretion by the adrenal cortex. Inhibition of ACE would therefore be useful in a patient suffering from disease states such as hypertension and congestive heart failure [See William W. Douglas, "Polypeptides - Angiotensin, Plasma Kinins, and Others", Chapter 27, in GOODMAN AND GILLMAN'S THE PHARMACOLOGICAL BASIS OF THERAPEUTICS, 7th edition, 1985, pp. 652-3, MacMillan Publishing Co., New York, New York]. In addition, it has been discovered that ACE inhibitors are useful in treating cognitive disorders [German Application No. 3901-291-A, published August 3, 1989].

In EP-A-249 223, US-A-4 824 832, US-A-4 584 294, EP-A-0 249 224 and Tet. Lett. 1990, 31(6), 815-8 fused tricyclic lactams are disclosed which are reported to be ACE inhibitors.

The present invention provides novel compounds of the formula (I) 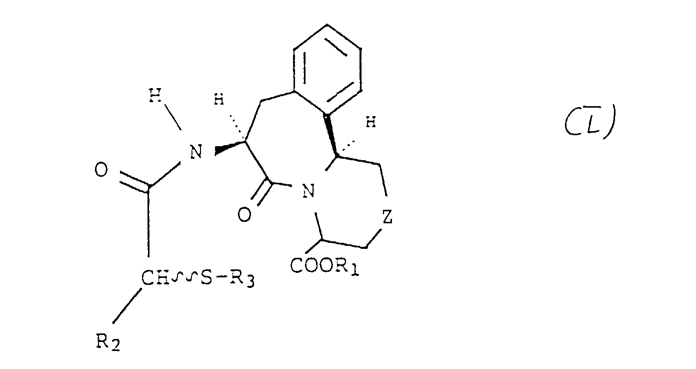
wherein R₁ is hydrogen; -CH₂O-C(O)C(CH₃)₃; a C₁-C₄ alkyl; an Ar-Y-group wherein Ar is aryl and Y is a single bond or a C₁-C₄ alkyl; or diphenylmethyl;
R₂ is hydrogen, C₁-C₈ alkyl, -CH₂OCH₂CH₂OCH₃ or an Ar-Y-group;
R₃ is hydrogen, acetyl, -CH₂O-C(O)C(CH₃)₃ or benzoyl;
Z is a group (CH₂)ₙ, -O-, S, NR₆ or N-C(O)R₇ wherein n is an integer 0 or 1, R₆ is hydrogen, a C₁-C₄ alkyl or an Ar-Y-group and R₇ is -CF₃, C₁-C₁₀ alkyl or an Ar-Y-group.

The compounds of Formula (I) can be used as pharmaceutical agents, especially for the inhibition of enkephalinase and ACE.

In addition, the present invention provides a composition comprising an assayable amount of a compound of Formula (I) in admixture or otherwise in association with an inert carrier. The present invention also provides a pharmaceutical composition comprising an effective enkephalinase or ACE inhibiting amount of a compound of Formula (I) in admixture or otherwise in association with one or more pharmaceutically acceptable carriers or excipients.

As used herein, the term "C₁-C₄ alkyl" refers to a saturated straight or branched chain hydrocarbyl radical of one to four carbon atoms and includes methyl, ethyl, propyl, isopropyl, n-butyl, isobutyl, tertiary butyl. The terms "C₁-C₈ alkyl" and "C₁-C₁₀ alkyl" refer to saturated straight or branched chain hydrocarbyl radicals of one to eight and one to ten carbon atoms, respectively, including methyl, ethyl, propyl, isopropyl, n-butyl, isobutyl, tertiary butyl, pentyl, isopentyl, hexyl, 2,3-dimethyl-2-butyl, heptyl, 2,2-dimethyl-3-pentyl, 2-methyl-2-hexyl, octyl, 4-methyl-3-heptyl. The term "halogen", "halo", "halide" or "Hal" refers to a chlorine, bromine, or iodine atom.

As used herein, the term "Ar-Y-" refers to a radical wherein Ar is an aryl group and Y is a C₀-C₄ alkyl. The term "Ar" refers to a phenyl or naphthyl group unsubstituted or substituted with from one to three substituents selected from the group consisting of methylenedioxy, hydroxy, C₁-C₄ alkoxy, fluoro and chloro. The term "C₀-C₄ alkyl" refers to a saturated straight or branched chain hydrocarbyl radical of zero to four carbon atoms and includes a bond, methyl, ethyl, propyl, isopropyl, n-butyl, isobutyl, tertiary butyl. Specifically included within the scope of the term "Ar-Y-" are phenyl, naphthyl, phenylmethyl or benzyl, phenylethyl, p-methoxybenzyl, p-fluorobenzyl and p-chlorobenzyl.

As used herein, the designation " 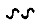 " refers to a bond to a chiral atom for which the stereochemistry is not designated.

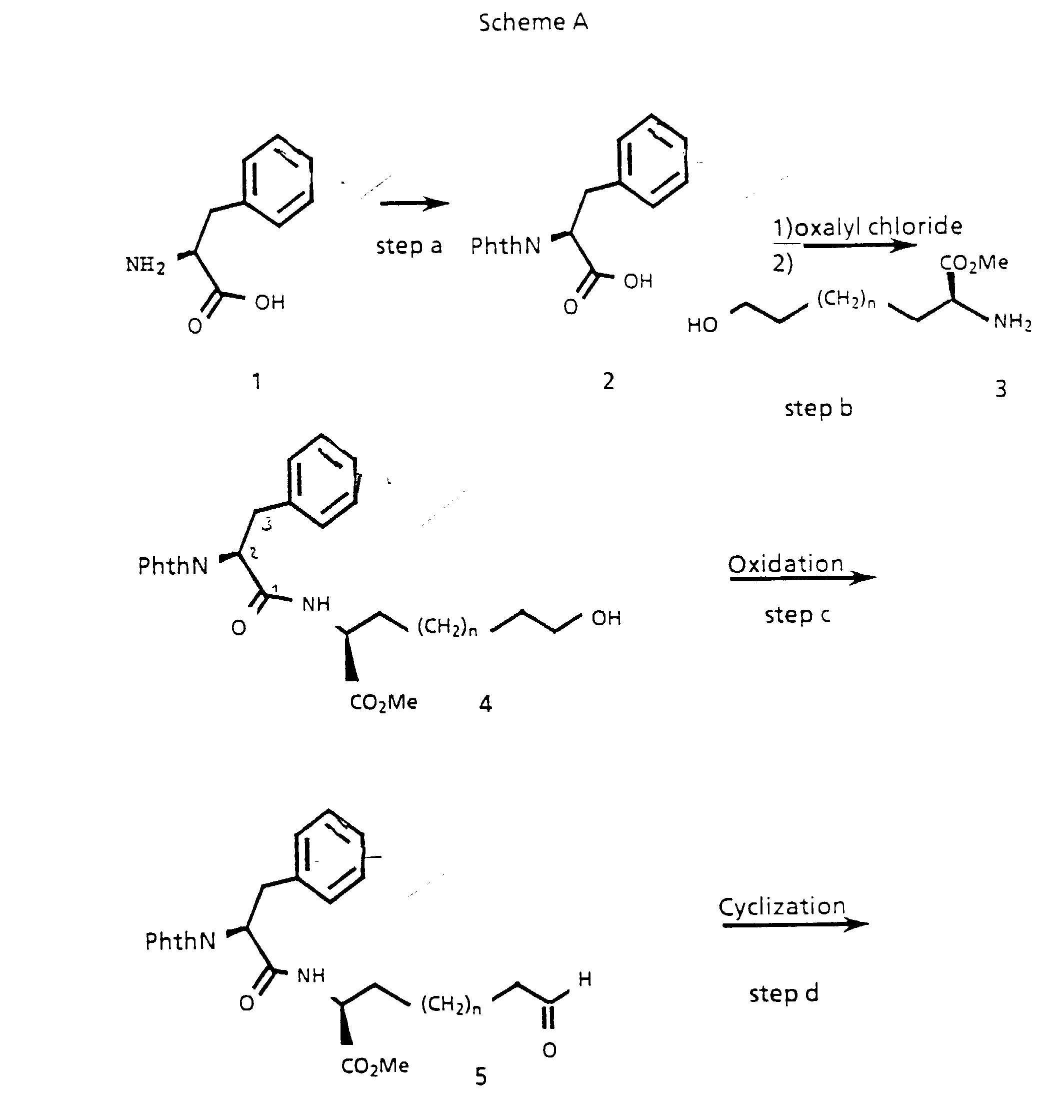

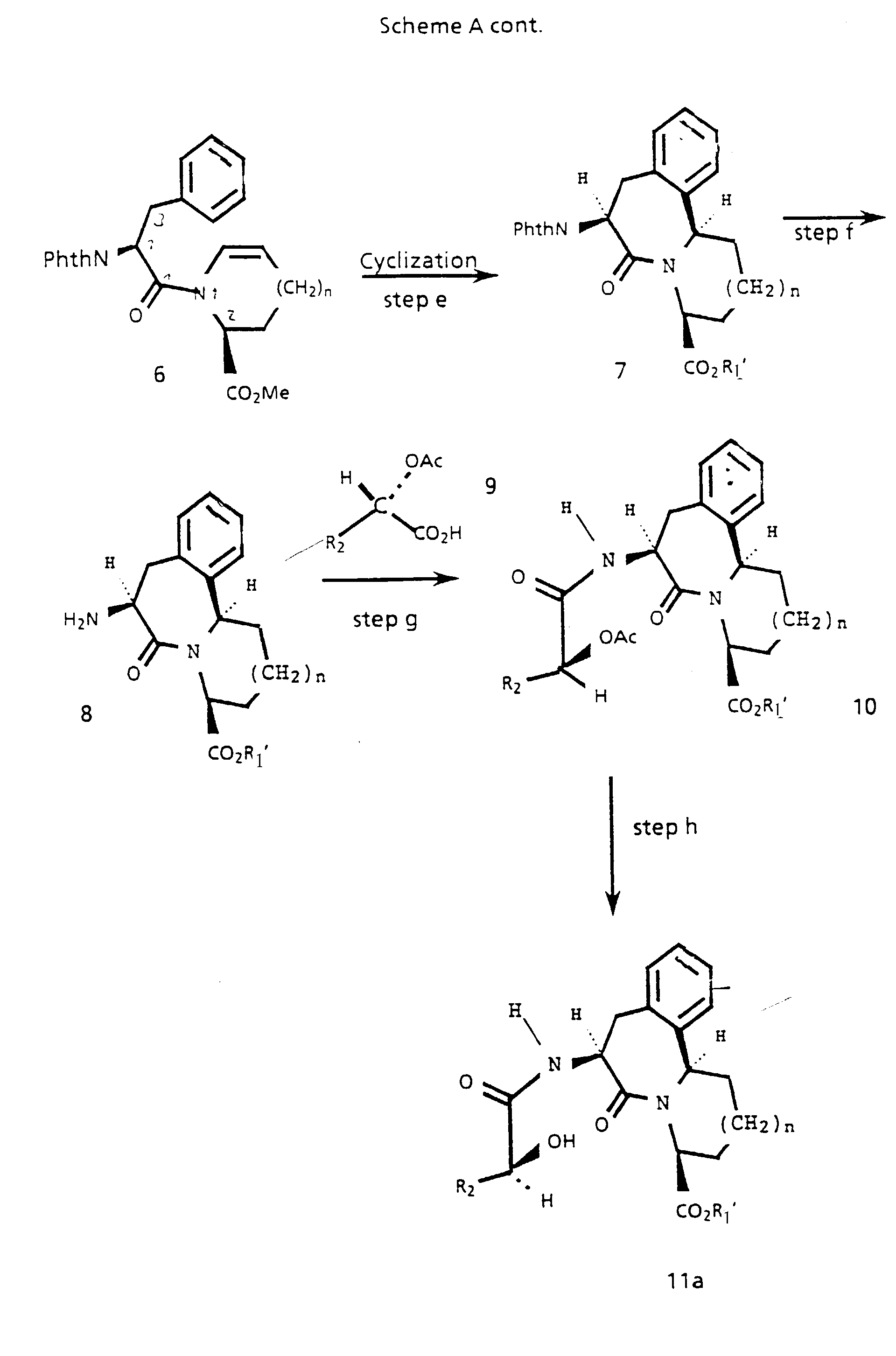

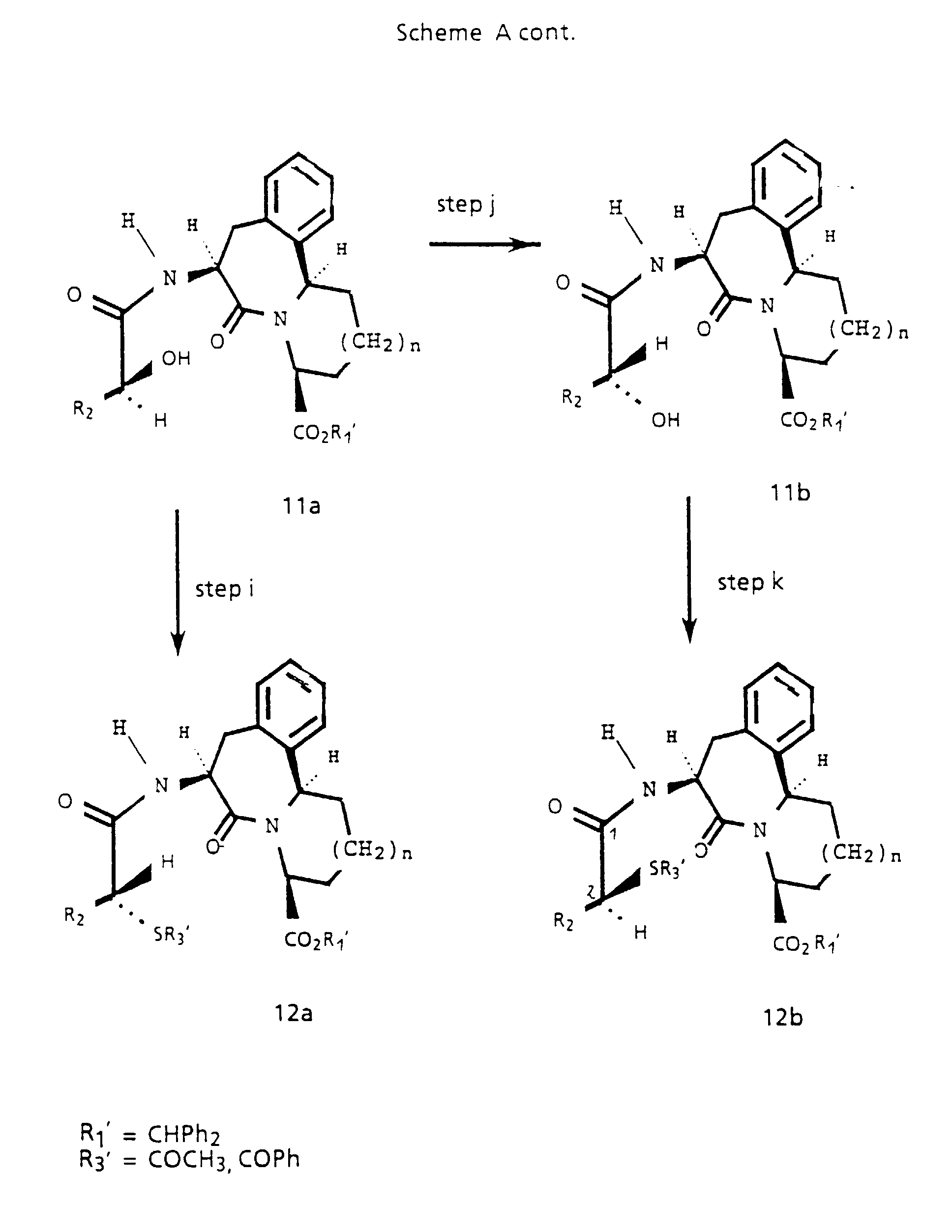

In step a, the appropriate phthalimide blocked (S)-phenylalanine derivative of structure 2 can be prepared by reacting the appropriate (S)-phenylalanine derivative of structure 1 with phthalic anhydride in a suitable aprotic solvent, such as dimethylformamide.

In step b, the appropriate phthalimide blocked (S)-phenylalanine derivative of structure 2 can be converted to the corresponding acid chloride, then reacted with the appropriate amino acid methyl ester of structure 3 in a coupling reaction. For example, the appropriate phthalimide blocked (S)-phenylalanine derivative of structure 2 can be reacted with oxalyl chloride in a suitable aprotic solvent, such as methylene chloride. The resulting acid chloride can then be coupled with the appropriate amino acid methyl ester of structure 3 using N-methylmorpholine in a suitable aprotic solvent, such as dimethylformamide, to give the appropriate 1-oxo-3-phenylpropyl-amino acid methyl ester derivative of structure 4.

In step c, the hydroxymethylene functionality of the appropriate 1-oxo-3-phenylpropyl-amino acid methyl ester derivative of structure 4 can be oxidized to the appropriate aldehyde of structure 5 by oxidation techniques well known and appreciated in the art. For example, the hydroxymethylene functionality of the appropriate 1-oxo-3-phenylpropyl-amino acid methyl ester derivative of structure 4 can be oxidized to the appropriate aldehyde of structure 5 by means of a Swern oxidation using oxalyl chloride and dimethylsulfoxide in a suitable aprotic solvent, such as methylene chloride.

In step d, the appropriate aldehyde of structure 5 can be cyclized to the appropriate enamine of structure 6 by acid catalysis. For example, the appropriate aldehyde of structure 5 can be cyclized to the appropriate enamine of structure 6 by treatment with trifluoroacetic acid in a suitable aprotic solvent, such as methylene chloride.

In step e, the appropriate enamine of structure 6 can be converted to the corresponding tricyclic compound of structure 7 by an acid catalyzed Friedel-Crafts reaction. For example, the appropriate enamine of structure 6 can be converted to the corresponding tricyclic compound of structure 7 by treatment with a mixture of trifluoromethane sulfonic acid and trifluoroacetic anhydride in a suitable aprotic solvent, such as methylene chloride.

In step e, it may be necessary to reesterify the carboxy functionality due to the conditions of the work-up. For example, treatment of the crude product with bromodiphenylmethane in a suitable aprotic solvent, such as dimethylformamide along with a non-nucleophilic base, such as cesium carbonate, may be used to give the corresponding diphenylmethyl ester.

In step f, the phthalimide protecting group of the appropriate tricyclic compound of structure 7 can be removed using techniques and procedures well known in the art. For example, the phthalimide protecting group of the appropriate tricyclic compound cf structure 7 can be removed using hydrazine monohydrate in a suitable protic solvent such as methanol, to give the corresponding amino compound of structure 8.

In step g, the appropriate (S)-acetate compound of structure 10 can be prepared by reacting the appropriate amino compound of structure 8 with the appropriate (S)-acetate of structure 9. For example, the appropriate amino compound of structure 8 can be reacted with the appropriate (S)-acetate compound of structure 9 in the presence of a coupling reagent such as EEDQ (1-ethoxycarbonyl-2-ethoxy-1,2-dihydroquinoline), DCC (1,3-dicyclohexylcarbodi-imide), or diethylcyanophosponate in a suitable aprotic solvent, such as methylene chloride to give the appropriate (S)-acetoxy compound of structure 10.

In step h, the (S)-acetate functionality of the appropriate amide compound of structure 10 can be hydrolized to the corresponding (S)-alcohol of structure 11a with a base, such as lithium hydroxide in a suitable solvent mixture, such as tetrahydrofuran and ethanol.

In step i, the (S)-alcohol functionality of the appropriate amide compound of structure 11a can be converted to the corresponding (R)-thioacetate or (R)-thiobenzoate of structure 12a. For example, the appropriate (S)-alcohol of structure 11a can be treated with thiolacetic acid in a Mitsunobu reaction using triphenylphosphine and DIAD (diisopropylazodicarboxylate) in a suitable aprotic solvent, such as tetrahydrofuran.

In step j, the (S)-alcohol functionality of the appropriate amide compound of structure 11a can be converted to the corresponding (R)-alcohol of structure 11b. For example, the appropriate (S)-alcohol of structure 11a can be treated with acetic acid in a Mitsunobu reaction using triphenylphosphine and DIAD in a suitable aprotic solvent, such as tetrahydrofuran. The resulting (R)-acetate can then be hydrolyzed with a base, such as lithium hydroxide.

In step k, the (R)-alcohol functionality of the appropriate amide compound of structure 11b can be converted to the corresponding (S)-thioacetate or (S)-thiobenzoate of structure 12b. For example, the appropriate (R)-alcohol of structure 11b can be treated with thiolacetic acid in a Mitsunobu reaction using triphenylphosphine and DIAD in a suitable aprotic solvent, such as tetrahydrofuran.

As summarized in Table 1, the R₃ and R₄ groups on the compounds of structures 12a and 12b can be manipulated using techniques and procedures well known and appreciated by one of ordinary skill in the art to give the corresponding compounds of structures 13a-18a and 13b-18b.

For example, the diphenylmethyl ester functionality of the appropriate compound of structure 12a can be removed using trifluoroacetic acid to give the appropriate carboxylic acid compound of structure 13a. Similarly, the diphenylmethyl ester functionality of the appropriate compound of structure 12b can be removed using trifluoroacetic acid to give the carboxylic acid compound of structure 13b.

The (R)-thioacetate or (R)-thiobenzoate functionality of the appropriate compound of structure 13a can be removed with lithium hydroxide in a suitable solvent mixture such as tetrahydrofuran and ethanol to give the appropriate (R)-thio compound of structure 14a. Similarly, the (S)-thioacetate or (S)-thiobenzoate functionality of the appropriate compound of structure 13b can be removed with lithium hydroxide in a suitable solvent mixture such as tetrahydrofuran and ethanol to give the appropriate (S)-thio compound of structure 14b.

Alternatively, the carboxylic acid functionality of the appropriate compound of structure 13a can be re-esterified using techniques and procedures well known and appreciated in the art. For example, a compound of structure 15a can be prepared by treating the carboxylic acid compound of structure 13a with the appropriate alkyl halide in a suitable aprotic solvent, such as dimethylformamide along with a non-nucleophilic base, such as cesium carbonate. Similarly, the carboxylic acid functionality of the appropriate compound of structure 13b can be esterified to the appropriate ester compound of structure 15b as described above for 15a.

The (R)-thioacetate or (R)-thiobenzoate functionalities of the appropriate compounds of structure 15a can be hydrolyzed to the corresponding (R)-thiol compounds of structure 16a with ammonia in a suitable protic solvent, such as methanol. Similarly, the (S)-thioacetate or (S)-thiobenzoate functionalities of the appropriate compounds of structure 15b can be hydrolyzed to the corresponding (S)-thiol compounds of structure 16b.

The thiol functionality of the appropriate compound of structure 14a can be alkylated using techniques and procedures well known and appreciated in the art. For example, a compound of structure 17a can be prepared by treating the thiol compound of structure 14a with the appropriate chloromethyl pivalate in a suitable aprotic solvent, such as dimethylformamide along with a non-nucleophilic base, such as cesium carbonate. Similarly, the thiol functionality of the appropriate compound of structure 14b can be alkylated to the appropriate pivalate compound of structure 17b as described above for 17a.

The thiol functionality of the appropriate compound of structure 16a can be alkylated using techniques and procedures well known and appreciated in the art. For example, a compound of structure 18a can be prepared by treating the thiol compound of structure 16a with the appropriate chloromethyl pivalate as described above for the conversion of 14a to 17a. Similarly, the thiol functionality of the appropriate compound of structure 16b can be alkylated to the appropriate pivalate compound of structure 18b as described above for 18a.

**TABLE 1**

| MANIPULATION OF R₃ AND R₁ | | |
|---|---|---|
| Compound | R₃ | R₁ |
| 13a and 13b | COCH₃ or COPh | H |
| 14a and 14b | H | H |
| 15a and 15b | COCH₃ or COPh | C₁-C₄ alkyl, Ar-Y, -CH₂OCOC(CH₃)₃ |
| 16a and 16b | H | C₁-C₄ alkyl, Ar-Y, diphenylmethyl, -CH₂OCOC(CH₃)₃ |
| 17a and 17b | -CH₂OCOC(CH₃)₃ | H |
| 18a and 18b | -CH₂OCOC(CH₃)₃ | C₁-C₄ alkyl, Ar-Y, diphenylmethyl, -CH₂OCOC(CH₃)₃ |

Starting materials for use in the general synthetic procedures outlined in Scheme A are readily available to one of ordinary skill in the art. For example, certain (R)- and (S)-carboxy acetate or benzoate starting materials of structure 9 can be prepared by stereoselective reduction of the corresponding pyruvate compounds with alpine boranes as described in J. *Org. Chem.* 47, 1606 (1982), J. *Org. Chem.* 49 , 1316 (1984), and *J.Am.Chem.Soc.* 106*,* 1531 (1984), followed by treating the resulting alcohol with acetic anhydride or benzoic anhydride to give the corresponding (R)- or (S)-carboxy acetate or benzoate compounds of structure 9.

Alternatively, certain tricyclic compounds of structure 7 may be prepared as described in European Patent Application of Flynn and Beight, EP-A-0 249 223 (June 11, 1987).

The following examples present typical syntheses as described in Scheme A. These examples are understood to be illustrative only and are not intended to limit the scope of the present invention in any way. As used herein, the following terms have the indicated meanings: "g" refers to grams; "mmol" refers to millimoles; "mL" refers to milliliters; "bp" refers to boiling point; "°C" refers to degrees Celsius; "mm Hg" refers to millimeters of mercury; "µL" refers to microliters; "µg" refers to micrograms; and "µM" refers to micromolar.

### Example 1

### Preparation of [4S-[4α,7α(R*), 12bβ]]-7-[(1-Oxo-2(R)-acetylthio-3-phenylpropyl)amino]-1,2,3,4,6,7,8,12b -octahydro-6-oxopyrido[2,1-a][2]benzazepine-4-carboxylic acid, diphenylmethyl ester

### Step a: N-phthaloyl-(S)-phenylalanine (2)

Mix phthalic anhydride (1.82kgs, 12.3mole), (S)-phenylalanine (1.84kgs, 11.1 moles) and anhydrous dimethylformamide (2.26L). Stir at 115-120°C for 2 hours under a nitrogen atmosphere. Pour into rapidly stirring water (32.6L) and cool overnight at 0°C. Filter, wash with cold water (2X2L) and air dry. Dissolve in a mixture of 9A ethanol (8.05L) and water (8.05L) and heat at reflux temperature. Gravity filter, cool to ambient temperature and refrigerate overnight at about 0°C. Filter the crystallized product, wash with cold 50:50 9A ethanol/water (2X2L) and air dry to yield 2.96kg (90.3%) of the title compound; mp 177-179°C.

### Step b: (S)-N-[2-(1,3-Dihydro-1,3-dioxo-2H-isoindol-2-yl)-1-oxo-3-phenylpropyl]-6-hydroxy-(S)-norleucine, methyl ester

Mix N-phthaloyl-(S)-phenylalanine (2) (50.2g, 0.17mole), methylene chloride (660mL) and dimethylformamide (0.5mL) under a nitrogen atmosphere. Add oxalyl chloride (17.7mL, 0.2mole) over about 5 minutes with stirring. Stir at ambient temperature for 3 hours and evaporate the solvent in vacuo to leave N-phthaloyl-(S)-phenylalanine, acid chloride as a solid (54.3g, 101.9%).

Mix 6-hydroxy-(S)-norleucine, methyl ester, hydrochloride salt (33.5g, 0.1mole) and dimethylformamide (201mL), cool to about 0°C and place under nitrogen atmosphere. Add by dropwise addition, N-methylmorpholine (51mL, 0.46mole) with cooling so that the pot temperature stays at 0-5°C. Stir at 0-5°C for an additional 10 minutes, than add a solution of N-phthaloyl-(S)-phenylalanine, acid chloride (53.5g, 0.17mole) in methylene chloride (270mL) over 30 minutes with cooling so that the temperature stays at 0-5°C. Remove the cooling bath and stir at room temperature for 18 hours.

Evaporate the methylene chloride in vacuo and dilute the remaining residue with ethyl acetate (800mL). Extract the resulting mixture with water (800mL), separate the organic layer and extract with 1N hydrochloric acid (270mL), followed by water (3X500mL). Dry the organic layer (MgSO₄), filter and evaporate in vacuo to yield crude product (76g, 98%). Dissolve the crude product in hot toluene (223.5mL), cool to room temperature, than cool overnight at about 0°C. Filter the crystallized product, wash with cold toluene and air dry to yield 56.6g (76%) of the title compound; mp 128-130°C.

### Step c: 2-(1,3 -Dihydro-1,3-dioxo-2H-isoindol-2-yl)-1-oxo-3-phenylpropyl-6-oxo-(S)-norleucine, methyl ester

Mix oxalyl chloride (80mL, 0.92mole) and methylene chloride (2L) and place under nitrogen atmosphere. Cool below -50°C and add a solution of dimethyl sulfoxide (65.4mL, 0.92mole) in methylene chloride (425mL). Stir for 15 minutes and add a solution of (S)-N-[2-(1,3-dihydro-1,3-dioxo-2H-isoindol-2-yl)-1-oxo-3-phenylpropyl]-6-hydroxy-(S)-norleucine, methyl ester (200g, 0.456mole) in methylene chloride (800mL) over about 45 minutes, keeping the Dot temperature below -50°C for 30 minutes. Add triethylamine (420mL, 3.01mole) over 30 minutes. Stir while warming to 0°C over 1.25 hours. Transfer the reaction mixture to a 12-liter flask. Stir and cool while adding a solution of OXONE (potassium peroxymonosulfate) (566g) in water (6.74L) at such a rate that the Dot temperature stays below 15°C. Stir for 5 minutes, separate the organic layer and extract the aqueous layer with methylene chloride (1L). Combine the organic phases, dry (MgSO₄) and filter to yield the title compound as a solution.

### Step d: [S-(R*,R*)]-N-[2-(1,3-Dihydro-1,3-dioxo-2H-isoindol-2-yl)-1-oxo-3-phenylpropyl]-1,2,3,4-tetrahydro-2 -pyridinecarboxylic acid, methyl ester

Transfer the solution of 2-(1,3-dihydro-1,3-dioxo-2H-isoindol-2-yl)-1-oxo-3-phenylpropyl -6-oxo-(S)-norleucine, methyl ester in methylene chloride (volume about 4.5L) to a 12-liter flask and place under nitrogen atmosphere. Stir and add trifluoroacetic acid (440mL, 5.71mole) in one portion. Stir the resulting mixture at room temperature for one hour, then rapidly cool to about 0°C. Add a solution of sodium hydroxide (240g, 6.0mole) in water (3.4L) in a slow stream to the vigorously stirred mixture at such a rate that the Dot temperature stays at about 0°C. Separate the organic phase and extract the aqueous phase with methylene chloride (1L). Combine the organic phases and dry (MgSO₄). Filter and remove the solvent in vacuo to leave a residue (262g, 137%).

Dissolve the above residue in diethyl ether (1L) and wash with water (5X500mL). Evaporate the organic phase in vacuo to leave a residue of 229g. Dilute the residue with methylene chloride (200mL) and purify by silica gel chromatography (methylene chloride) to yield a viscous residue of 225g.

Dilute the above residue with diethyl ether (250mL) and allow to stand at room temperature for 24 hours. Filter the solid, wash with diethyl ether, and air dry to yield 123.2g; mp 140-142.5°C. Recrystallize (methylene chloride (125mL)/isopropanol (615mL)) by boiling off the solvent until the pot temperature reaches 75°C and allowing the resulting sample to stand at room temperature for 24 hours. Filter, wash with cold isopropanol and air dry to yield 101.5g of the title compound; mp 144-146°C.

Evaporate the filtrate from the 101.5g in vacuo to yield 24g. Recrystallize (isopropanol) to yield an additional 3.5g of the title compound.

Evaporate the filtrate from the 123.2g in vacuo to leave 62g of oil. Purify by silica gel chromatography (25% ethyl acetate/75% hexane), collecting 21500mL fractions. Combine fractions 9-20 and evaporate in vacuo to yield 35g of a viscous oil. Recrystallize three times (isopropanol/5mL/g) to yield an additional 11.9g of the title compound; mp 142.5-144.5°C. Total yield of useful material: 116.9g (61.3%).

### Step e: [4S-[4α,7α(R*), 12bβ]]-7-[(1,3-Dihydro-1,3-dioxo-2H-isoindol-2-yl)]-1,2,3,4,6,7,8,12b-octahydro-6 -oxopyrido[2,1-a][2]benzazepine-4-carboxylic acid, diphenylmethyl ester

Mix trifluoromethane sulfonic acid (500g, 3.33mole) and trifluoroacetic anhydride (74.8mL, 0.53mole) and place under nitrogen atmosphere. Stir and add a solution of [S-(R*,R*)]-N-[2-(1,3-dihydro-1,3-dioxo-2H-isoindol-2-yl)-1-oxo-3-phenylpropyl]-1,2,3,4-tetrahydro-2-pyridinecarboxylic acid, methyl ester (200g, 0.48mole) in methylene chloride (1L) with cooling at such a rate as to keep the Dot temperature below 35°C. Stir at ambient temperature for 2 days. Pour into vigorously stirring ice water (5L) and stir for 30 minutes. Extract with ethyl acetate (3X1L), combine the organic phases and wash with water (3x500mL). Evaporate in vacuo to a residue. Dissolve the residue in ethyl acetate (4L) and extract with 1/4 saturated potassium hydrogen carbonate (1L), then 1/3 saturated potassium hydrogen carbonate (7X1L). Combine the aqueous extracts and dilute with ethyl acetate (2L). Stir the resulting mixture and cool to 5-10°C. Adjust to pH 2 using concentrated hydrochloric acid (about 750mL).

Separate the organic phase and extract the aqueous phase with ethyl acetate (3X1L). Combine the ethyl acetate extracts, wash with water (3X1L), then saturated sodium chloride (0.8L), and dry (MgSO₄). Filter and wash with ethyl acetate (3X200mL). Evaporate in vacuo to leave (188.3g, 101.5%) [4S-[4α, 7α(R*), 12bβ]]-7-[(1,3-dihydro-1,3-dioxo-2H-isoindol-2-yl)]-1,2,3,4,6,7,8,12b-octahydro-6-oxopyrido[2,1-a][2]benzazepine-4-carboxylic acid as a colorless foam.

Dissolve [4S-[4α, 7α(R*), 12bβ]]-7-[(1,3-dihydro-1,3-dioxo-2H-isoindol-2-yl)]-1,2,3,4,6,7,8,12b-octahydro-6-oxopyrido[2,1-a][2]benzazepine-4-carboxylic acid (113.9g, 0.28mole) in methylene chloride (1.2L) and dry over anhydrous MgSO₄ (60g). Filter and wash with methylene chloride (3X200mL). Evaporate in vacuo to a residue. Dissolve the residue in anhydrous dimethylformamide (860mL) and place under nitrogen atmosphere. Add cesium carbonate (98.9g, 0.3mole) in one portion. Stir for 45 minutes at ambient temperature. Add bromodiphenylmethane (164.8g, 0.67mole). Stir the resulting mixture at ambient temperature for 18 hours. Quench the reaction with ethyl acetate (2.464L) and water (630mL). Separate the organic phase and wash with water (7X625mL), 1/4 saturated potassium hydrogen carbonate (625mL), water (625mL), and saturated sodium chloride (625mL). Dry (MgSO₄), filter and evaporate in vacuo to yield 214.4g of an oil. Extract the combined aqueous washings with ethyl acetate (3X500mL), wash with water (4X300mL) and dry (MgSO₄). Filter and evaporate in vacuo to yield an additional 20.2g of an oil.

Dissolve the crude product (234.6g) in methylene chloride (200mL) and plug filter through 213g of silica gel, eluting with methylene chloride (2L). Boil off the solvent and replace with hexane (3L), with the Dot temperature reaching a maximum of 65°C. Cool to ambient temperature, decant off the precipitated oil and crystallize (9A ethanol) to yield 96.6g (60%) of the title compound; mp 153-155°C.

### Step f: [4S-[4α,7α(R*),12bβ]]-7-(Amino)-1,2,3,4,6,7,8,12b-octahydro-6-oxopyrido[2,1 -a][2]benzazepine-4-carboxylic acid, diphenylmethyl ester

Mix [4S-[4α, 7α(R*), 12bβ]]-7-[(1,3-dihydro-1,3-dioxo-2H-isoindol-2-yl)]-1,2,3,4,6,7,8,12b-octahydro-6-oxopyrido[2,1-a][2]benzazepine-4-carboxylic acid, diphenylmethyl ester (170.9g, 0.3mole), hydrazine monohydrate (34.4g, 0.68mole) and methanol (3.4L) under nitrogen atmosphere. Heat at reflux for 5 hours. Cool to ambient temperature and filter to remove phthaloyl hydrazide. Evaporate the filtrate in vacuo to a residue and slurry in chloroform (600mL). Remove insoluble phthaloyl hydrazide by filtration and wash with chloroform (4X210mL). Wash the filtrate with water (4X429mL), dry (MgSO₄), and filter. Evaporate the filtrate to a solid residue of the title compound weighing 142g (107.7%).

### Step g: [4S-[4α, 7α(R*), 12bβ]]-7-[(1-Oxo-2(S)-acetyloxy-3-phenylpropyl)amino]-1,2,3,4,6,7,8,12b-octahydro-6-oxo -pyrido[2,1-a][2]benzazepine-4-carboxylic acid, diphenylmethyl ester

Mix (S)-3-phenyllactic acid (11.17g, 67.2mmol) and sulfuric acid (0.3mL of a 10% solution in acetic acid). Add acetic anhydride (6.34mL, 67.2mmol) over 10 minutes. Warm to 90°C with stirring for 45 minutes. Allow to cool, pour into diethyl ether and wash with water three times. Separate the organic phase, dry (MgSO₄) and concentrate in vacuo to yield 13.16g (94%) (S)-3-phenyl-2-acetyloxypropionic acid as a white oil.

Mix (S)-3-phenyl-2-acetyloxypropionic acid (3.6g, 17mmol) and [4S-[4α, 7α(R*), 12bβ]]-7-(amino)-1,2,3,4,6,7,8,12b-octahydro-6-oxopyrido[2,1-a][2]benzazepine-4-carboxylic acid, diphenylmethyl ester (7.6g, 17mmol) in methylene chloride (50mL). Add EEDQ (4.3g, 17mmol). Stir for 18 hours at ambient temperature under argon atmosphere. Extract with 2N hydrochloric acid, separate the organic phase, wash with water, then with saturated sodium hydrogen carbonate. Dry (MgSO₄) and concentrate in vacuo to yield an off-white foam. Purify by silica gel chromatography (30%, then 40%, then 50% ethyl acetate/hexane) to yield 8.5g (78%) of the title compound.

### Step h: [4S-[4α, 7α(R*), 12bβ]]-7-[(1-Oxo-2(S)-hydroxy-3-phenylpropyl)amino]-1,2,3,4,6,7,8,12b-octahydro-6-oxo -pyrido[2,1-a][2]benzazepine-4-carboxylic acid, diphenylmethyl ester

Dissolve [4S-[4α, 7α(R*), 12bβ]]-7-[(1-oxo-2(S)-acetyloxy-3-phenylpropyl)amino]-1,2,3,4,6,7,8,12b-octahydro-6-oxo-pyrido[2,1-a][2]benzazepine-4-carboxylic acid, diphenylmethyl ester (11.0g, 17.4mmol) in ethanol (75mL) and tetrahydrofuran (40mL) and add lithium hydroxide (22mL of a 1M solution, 22mmol). Stir the reaction mixture for 2 hours. Remove the solvent in vacuo at 35°C and partition the residue between ethyl acetate and 1N hydrochloric acid. Separate the organic phase, dry (MgSO₄), and concentrate in vacuo. Purify by silica gel chromatography (1:1/ tetrahydrofuran:hexane) to yield 9.6g (94%) of the title compound.
Anal. Calcd for C₃₇H₃₆N₂O₅: C, 75.49; H, 6.16; N, 4.76;
Found: C, 75.30; H, 6.44; N, 4.54.

### Step i: [4S-[4α, 7α(R*), 12bβ]]-7-[(1-Oxo-2(R)-acetylthio-3-phenylpropyl)amino] -1,2,3,4,6,7,8,12b-octahydro-6-oxopyrido[2,1-a][2]benzazepine-4-carboxylic acid, diphenylmethyl ester

Mix DIAD ( 3.4g, 16.3mmol), triphenylphosphine (4.28g, 16.3mmol) and anhydrous tetrahydrofuran (200mL). Cool to 0°C and stir for 30 minutes under nitrogen atmosphere. Add a solution of the [4S-[4α, 7α(R*), 12bβ]]-7-[(1-oxo-2(S)-hydroxy-3-phenylpropyl)amino]-1,2,3,4,6,7,8,12b-octahydro-6-oxo-pyrido[2,1-a][2]benzazepine-4-carboxylic acid, diphenylmethyl ester (9.6g, 16.3mmol) and thiolacetic acid (1.75mL, 24.5mmol) in anhydrous tetrahydrofuran (200mL).

Stir at 0°C for 30 minutes, then allow to warm to ambient temperature. Remove the volatiles in vacuo and purify by silica gel chromatography (1L each of 20%, 30%, 40% then 50% tetrahydrofuran/hexane) to yield 5.127g (49%) of the title compound.

### Example 2

### Preparation of [4S-[4α, 7α(R*), 12bβ]]-7-[(1-Oxo-2(R)-acetylthio-3-phenylpropyl)amino]-1,2,3,4,6,7,8,12b -octahydro-6-oxopyrido[2,1-a][2]benzazepine-4-carboxylic acid

Mix [4S-[4α, 7α(R*), 12bβ]]-7-[(1-oxo-2(R)-acetylthio-3-phenylpropyl)amino]-1,2,3,4,6,7,8,12b-octahydro-6-oxo-pyrido[2,1-a][2]benzazepine-4-carboxylic acid, diphenylmethyl ester (3.125g, 4.83mmol), anisole (5g) and trifluoroacetic acid (30mL). Stir for 2 hours at ambient temperature under nitrogen atmosphere. Remove the volatiles in vacuo and purify by silica gel chromatography (1400mL 30% tetrahydrofuran/hexane with 5% acetic acid added, then 1000mL 40% tetrahydrofuran/hexane with 5% acetic acid added) to yield a viscous foam. Dissolve the foam in a minimal amount of methylene chloride and dilute with hexane until cloudy. Allow to stand overnight at -30°C, filter the resulting white solid and dry at 56°C under high vacuum to yield 2.079g (89.53%) of the title compound.

### Example 3

### Preparation of [4S-[4α, 7α(R*), 12bβ]]-7-[(1-Oxo-2(R)-thio-3-phenylpropyl)amino]-1,2,3,4,6,7,8,12b-octahydro-6-oxo -pyrido[2,1-a][2]benzazepine-4-carboxylic acid

Dissolve [4S-[4α, 7α(R*), 12bβ]]-7-[(1-oxo-2(R)-acetylthio-3-phenylpropyl)amino]-1,2,3,4,6,7,8,12b-octahydro-6-oxo-pyrido[2,1-a][2]benzazepine-4-carboxylic acid (2.079g, 4.33mmol) in tetrahydrofuran (10mL). Add to a solution of lithium hydroxide (20mL of a 1N solution, 20mmoi) in methanol (60mL, deoxygenated by bubbling with nitrogen for 10 minutes). Stir the cloudy solution for 40 minutes. Acidify and dilute the water until cloudy. Remove l0mL of volatile solvent under high vacuum then dilute the mixture to 25mL with water. Stir the mixture for 10 minutes, filter under a blanket of nitrogen, and partially suction dry under nitrogen atmosphere. Dry the solid overnight under high vacuum, then for 18 hours at 35°C under high vacuum, 24 hours at 40°C, then 64 hours at 50°C at 10 mm Hg.

In a second run, dissolve [4S-[4α, 7α(R*), 12bβ]]-7-[(1-oxo-2(R)-acetylthio-3-phenylpropyl)amino]-1,2,3,4,6,7,8,12b-octahydro-6-oxo-pyrido[2,1-a][2]benzazepine-4-carboxylic acid (2.819g, 5.87mmol) in tetrahydrofuran (degassed). Add to a degassed solution of lithium hydroxide (27mL of a 1N solution, 27mmol) in methanol (80mL). Stir at ambient temperature under nitrogen atmosphere for 45 minutes. Acidify with hydrochloric acid (15mL of a 2N solution), dilute with water to 250mL and stir for 15 minutes. Collect the solid by filtration under a blanket of nitrogen and wash with water. Dry for 2 hours at ambient temperature under high vacuum, then for 18 hours at 40°C under high vacuum, then for 64 hours at 56°C.

Combine materials from both runs to yield 3.955g (88.5%) of the title compound.

### Example 4

### Preparation of [4S-[4α, 7α(R*), 12bβ]]-7-[(1-Oxo-2(R)-acetylthio-3-phenylpropyl)amino]-1,2,3,4,6,7,8,12b -octahydro-6-Oxo-pyrido][2,1-a][2]benzazepine-4-carboxylic acid, benzyl ester

Dissolve [4S-[4α, 7α(R*), 12bβ]]-7-[(1-oxo-2(R)-acetylthio-3-phenylpropyl)amino]-1,2,3,4,6,7,8,12b-octahydro-6-oxo-pyrido[2,1-a][2]benzazepine-4-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.0mmol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to yield the title compound.

### Example 5

### Preparation of [4S-[4α, 7α(R*), 12bβ]]-7-[(1-Oxo-2(R)-thio-3-phenylpropyl)amino]-1,2,3,4,6,7,8,12b-octahydro-6-oxo -pyrido[2,1-a][2]benzazepine-4-carboxylic acid, benzyl ester

Stir [4S-[4α, 7α(R*), 12bβ]]-7-[(1-oxo-2(R)-acetylthio-3-phenylpropyl)amino]-1,2,3,4,6,7,8,12b-octahydro-6-oxo-pyrido[2,1-a][2]benzazepine-4-carboxylic acid, benzyl ester (2.28g, 4mmol) and saturated methanolic ammonia at ambient temperature until hydrolysis is complete. Evaporate the solvent in vacuo and purify by silica gel chromatography to give the title compound.

### Example 6

### Preparation of [4S-[4α, 7α(R*), 12bβ]]-7-[(1-Oxo-2(S)-acetylthio-3-phenylpropyl)amino]-1,2,3,4,6,7,8,12b

### octahydro -6 -oxo-pyrido[2,1-a][2]benzazepine-4-carboxylic acid, diphenylmethyl ester

### Step j: [4S-[4α, 7α(R*), 12bβ]]-7-[(1-Oxo-2(R)-hydroxy-3-phenylpropryl)amino]-1,2,3,4,6,7,8,12b-octahydro-6-oxo -pyrido[2,1-a][2]benzazepine-4-carboxylic acid, diphenylmethyl ester

Dissolve [4S-[4α, 7α(R*), 12bβ]]-7-[(1-oxo-2(S)-hydroxy-3-phenylpropyl)amino]-1,2,3,4,6,7,8,12b-octahydro-6-oxopyrido[2,1-a][2]benzazepine-4-carboxylic acid, diphenylmethyl ester (59mg, 0.1mmol), triphenylphosphine (39mg, 0.15mmol) and acetic acid (8.7µL, 0.15mmol) in anhydrous tetrahydrofuran (3mL). Treat with DIAD (32mg, 0.15mmol) at 0°C. Stir for 5 minutes at 0°C, then allow to stir at ambient temperature for 45 minutes. Remove the volatiles in vacuo and purify the residue by silica gel chromatography (3:1/hexane:tetrahydrofuran) to yield 67mg (106%) [4S-[4α, 7α(R*), 12bβ]]-7-[(1-oxo-2(R)-acetyloxy-3-phenylpropyl)amino]-1,2,3,4,6,7,8,12b-octahydro-6-oxo-pyrido[2,1-a][2]benzazepine-4-carboxylic acid, diphenylmethyl ester.

Dissolve [4S-[4α, 7α(R*), 12bβ]]-7-[(1-oxo-2(R)-acetyloxy-3-phenylpropyl)amino]-1,2,3,4,6,7,8,12b-octahydro-6-oxo-pyrido[2,1-a][2]benzazepine-4-carboxylic acid, diphenylmethyl ester (366mg, 0.58mmol) in methanol (5mL) and tetrahydrofuran (5mL) and add lithium hydroxide (0.8mL of a 1M solution, 0.8mmol). Stir the reaction mixture for 2 hours and remove the solvent in vacuo. Acidify and partition between methylene chloride and water. Separate the organic phase, dry (MgSO₄) and concentrate in vacuo to yield a white foam.

In another run, dissolve [4S-[4α, 7α(R*), 12bβ]]-7-[(1-oxo-2(R)-acetyloxy-3-phenylpropyl)amino]-1,2,3,4,6,7,8,12b-octahydro-6-oxo-pyrido[2,1-a][2]benzazepine-4-carboxylic acid, diphenylmethyl ester (100mg, 0.16mmol) in methanol (5mL) and tetrahydrofuran (5mL) and add lithium hydroxide (0.2mL of a 1M solution, 0.2mmol). Stir the reaction mixture for 1 hour, dilute with water (50mL), make acidic and extract with diethyl ether (50mL). Separate the organic phase, wash with water (2X50mL), dry (MgSO₄) and concentrate in vacuo.

Combine the material from both runs and purify by silica gel chromatography (30%, then 50% tetrahydrofuran/hexane) to yield 355mg (82%) of the title compound as a white foam.

### Step k: [4S-[4α, 7α(R*), 12bβ]]-7-[(1-Oxo-2(S)-acetylthio-3-phenylpropyl)amino]-1,2,3,4,6,7,8,12b-octahydro-6-oxo -pyrido[2,1-a][2]benzazepine-4-carboxylic acid, diphenylmethyl ester

Mix DIAD (31mg, 0.15mmol), triphenylphosphine (39mg, 0.16mmol) and anhydrous tetrahydrofuran (2mL). Cool to 0°C and stir for 30 minutes under an argon atmosphere. Add the [4S-[4α, 7α(R*), 12bβ]]-7-[(1-oxo-2(R)-hydroxy-3-phenylpropyl)amino]-1,2,3,4,6,7,8,12b-octahydro-6-oxo-pyrido[2,1-a][2]benzazepine-4-carboxylic acid, diphenylmethyl ester (59mg, 0.1mmol) as a solid, then immediately add thiolacetic acid (11µL, 0.15mmol). Allow the reaction to warm to ambient temperature and stir overnight. Remove the solvent in vacuo and purify the residue by silica gel chromatography (40% ethyl acetate/hexane then 50% ethyl acetate/hexane) to yield 51mg of the title compound.

### Example 7

### Preparation of [4S-[4α, 7α(R*), 12bβ]]-7-[(1-Oxo-2(S)-acetylthio-3-phenylpropyl)amino]-1,2,3,4,6,7,8,12b -octahydro-6-oxo-pyrido[2,1-a][2]benzazepine-4-carboxylic acid

Dissolve [4S-[4α, 7α(R*), 12bβ]]-7-[(1-oxo-2(S)-acetylthio-3-phenylpropyl)amino]-1,2,3,4,6,7,8,12b-octahydro-6-oxo-pyrido[2,1-a][2]benzazepine-4-carboxylic acid, diphenylmethyl ester (51mg, 0.079mmol) in anisole (4 drops) and trifluoroacetic acid (1mL) under argon atmosphere at ambient temperature. Allow to stand for 45 minutes, remove the trifluoroacetic acid in vacuo and purify by silica gel chromatography (50mL of 40% ethyl acetate/hexane then 50mL of 40% ethyl acetate/hexane with 5% acetic acid added) to yield 30mg (74%) of the title compound.

### Example 8

### Preparation of [4S-[4α, 7α(R*), 12bβ]]-7-[(1-Oxo-2(S)-thio-3-phenylpropyl)amino]-1,2,3,4,6,7,8,12b-octahydro-6-oxo -pyrido[2,1-a][2]benzazepine-4-carboxylic acid

Dissolve [4S-[4α, 7α(R*), 12bβ]]-7-[(1-oxo-2(S)-acetylthio-3-phenylpropyl)amino]-1,2,3,4,6,7,8,12b-octahydro-6-oxo-pyrido[2,1-a][2]benzazepine-4-carboxylic acid (57mg, 0.12mmol) in deoxygenated methanol (3mL) plus 1N lithium hydroxide (0.25mL, 0.25mmol). Stir for 30 minutes under argon atmosphere at ambient temperature. Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to yield 30mg (58%) of the title compound as a white electrostatic powder.

### Example 9

### Preparation of [4S-[4α,7α(R*), 12bβ]]-7-[(1-Oxo-2(S)-acetylthio-3-phenylpropyl)amino]-1,2,3,4,6,7,8,12b -octahydro-6-oxo-pyrido[2,1-a][2]benzazepine-4-carboxylic acid, pivaloyloxymethyl ester

Dissolve [4S-[4α, 7α(R*), l2bβ]]-7-[(1-oxo-2(S)-acetylthio-3-phenylpropyl)amino]-1,2,3,4,6,7,8,12b-octahydro-6-oxo-pyrido[2,1-a][2]benzazepine-4-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add chloromethyl pivalate (753mg, 5.0mmol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to yield the title compound.

### Example 10

### Preparation of [4S-[4α, 7α(R*), 12bβ]]-7-[(1-Oxo-2(S)-thio-3-phenylpropyl)amino]-1,2,3,4,6,7,8,12b-octahydro-6-oxo -pyrido[2,1-a][2]benzazepine-4-carboxylic acid, pivaloyloxymethyl ester

Stir [4S-[4α, 7α(R*), 12bβ]]-7-[(1-oxo-2(S)-acetylthio-3-phenylpropyl)amino]-1,2,3,4,6,7,8,12b-octahydro-6-oxo-pyrido[2,1-a][2]benzazepine-4-carboxylic acid, pivaloyloxymethyl ester (2.38g, 4mmol) and saturated methanolic ammonia at ambient temperature until hydrolysis is complete. Evaporate the solvent in vacuo and purify by silica gel chromatography to give the title compound.

The following compounds can be prepared by procedures analogous to those described above in Examples 1 - 10:
[4S-[4α, 7α(R*), 12bβ]]-7-[(1-oxo-2(R)-benzoylthio-3-phenylpropyl)amino]-1,2,3,4,6,7,8,12b-octahydro-6-oxopyrido[2,1-a][2]benzazepine-4-carboxylic acid, diphenylmethyl ester;
[4S-[4α, 7α(R*), 12bβ]]-7-[(1-oxo-2(R)-benzoylthio-3-phenylpropyl)amino]-1,2,3,4,6,7,8,12b-octahydro-6-oxopyrido[2,1-a][2]benzazepine-4-carboxylic acid;
[4S-[4α, 7α(R*), 12bβ]]-7-[(1-oxo-2-thio-ethyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2(R)-benzoylthio-3-phenylpropyl)amino]-1,2,3,4,6,7,8,12b-octahydro-6-oxo-pyrido[2,1-a][2]benzazepine-4-carboxylic acid, benzyl ester;
[4S-[4α, 7α(R*), 12bβ]]-7-[(1-oxo-2-thio-ethyl)amino]-1,2,3,4,6,7,8,12b-octahydro-6-oxo-pyrido[2,1-a][2]benzazepine-4-carboxylic acid, benzyl ester;
[4S-[4α, 7α(R*), 12bβ]]-7-[(1-oxo-2(S)-benzoylthio-3-phenylpropyl)amino]-1,2,3,4,6,7,8,12b-octahydro-6-oxo-pyrido[2,1-a][2]benzazepine-4-carboxylic acid, diphenylmethyl ester;
[4S-[4α, 7α(R*), 12bβ]]-7-[(1-oxo-2(S)-benzoylthio-3-phenylpropyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2(S)-benzoylthio-3-phenylpropyl)amino]-1,2,3,4,6,7,8,12b-octahydro-6-oxo-pyrido[2,1-a][2]benzazepine-4-carboxylic acid, benzyl ester;
[4R-[4β, 7α(R*), 12bβ]]-7-[(1-oxo-2-benzoylthioethyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2-thio-2-phenylethyl)amino]-1,2,3,4,6,7,8,12b-octahydro-6-oxo-pyrido[2,1-a][2]benzazepine-4-carboxylic acid;

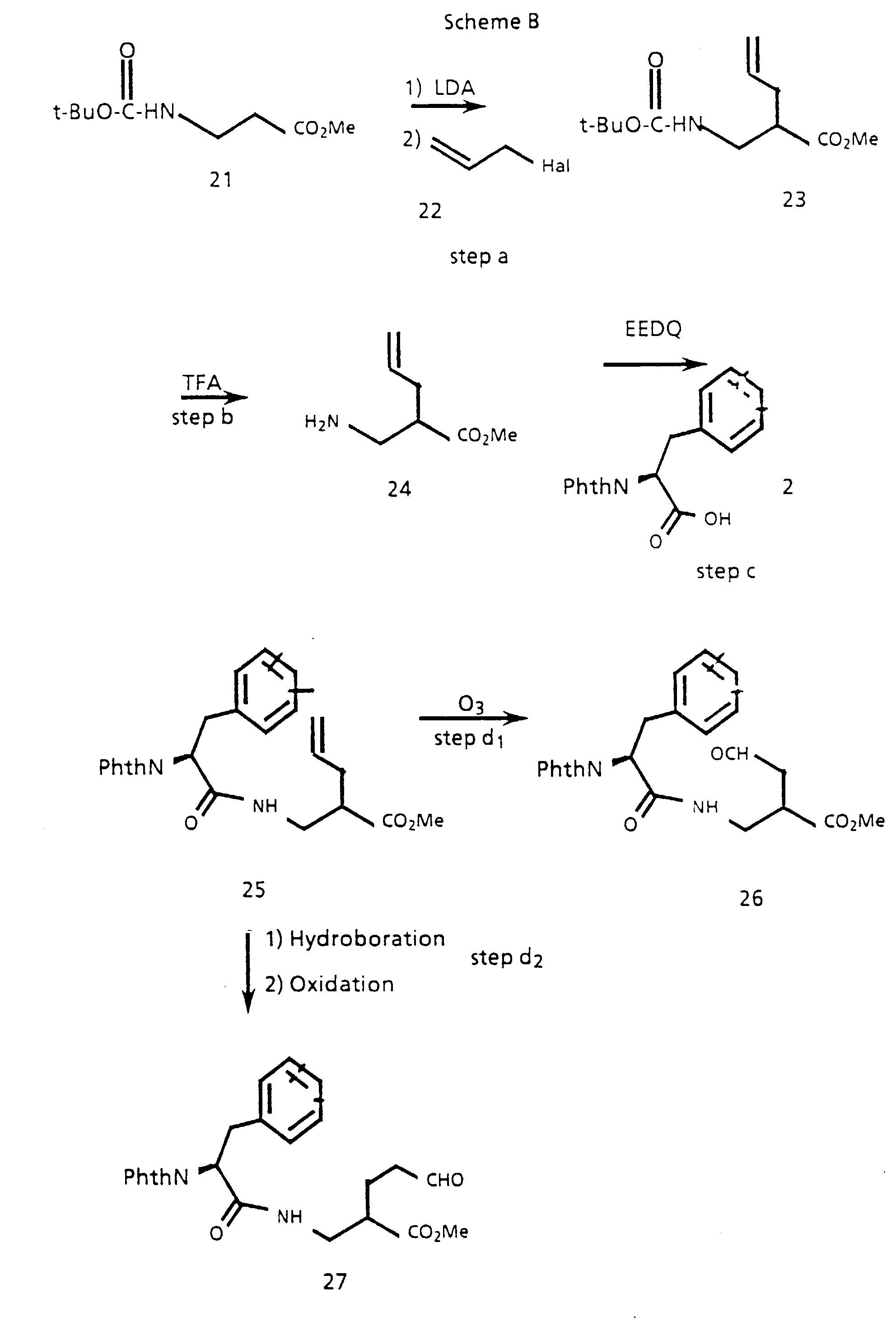

In step a, N-(t-butyloxycarbonyl)-β-alanine methyl ester (21) can be treated with two equivalents of a non-nucleophilic base, such as lithium diisopropylamide, in a suitable aprotic solvent, such as tetrahydrofuran, followed by addition of an allyl halide of structure 22 to give 2-(2-propenyl)-N-(t-butyloxycarbonyl)-β-alanine methyl ester (23).

In step b, the t-butyloxycarbonyl-amino -amino functionality of 2-(2-propenyl) -N-(t-butyloxycarbonyl)-β-alanine methyl ester (23) can be hydrolyzed under acidic conditions, such as with trifluoroacetic acid in a suitable aprotic solvent, such as methylene chloride to give 2-(2-propenyl)-β-alanine methyl ester (24).

In step c, the appropriate amide compound of structure 25 can be prepared by reacting the appropriate phthalimide protected (S)-phenylalanine compound of structure 2 (described previously in Scheme A) with 2-(2-propenyl)-β-alanine methyl ester (24) under coupling reaction conditions, such as with EEDQ, in a suitable aprotic solvent, such as methylene chloride.

In step d₁, the olefin functionality of the appropriate amide compound of structure 25 can be converted to the appropriate aldehyde compound of structure 26 under conditions of oxidative cleavage, such as treatment with ozone in a suitable solvent mixture, such as methylene chlorite and methanol.

Alternatively, in step d₂, the olefin functionality of the appropriate amide compound of structure 25 can be converted to the appropriate aldehyde compound of structure 27 by first subjecting to hydroboration, followed by oxidation. For example, the olefin functionality of the appropriate amide compound of structure 25 can be hydroborated with 9-borabicyclo[3.3.1]nonane (9-BBN) in a suitable aprotic solvent, such as tetrahydrofuran. The hydroborated olefin can than be oxidized under techniques and procedures well known and appreciated in the art, such as treatment with sodium hydroxide and hydrogen peroxide to give the alcohol, followed by Swern oxidation, using oxalyl chloride and dimethyl sulfoxide in a suitable aprotic solvent, such as methylene chloride.

The compounds of Formula 1 wherein R₃ is acetate or benzoate, R₁ is methyl, and n = 0 can be prepared from an appropriate aldehyde of structure 26 in a process as outlined previously in Scheme A, steps d-k.

The groups R₃ and R₁ may be manipulated by techniques and procedures well known and appreciated in the art and described previously in Scheme A and Table 1.

Starting materials for use in the general synthetic procedures outlined in Scheme B are readily available to one of ordinary skill in the art and described previously in Scheme A.

The following examples present typical syntheses as described in Scheme B. These examples are understood to be illustrative only and are not intended to limit the scope of the present invention in any way.

### Example 11

### Preparation of [6α(R*), 11bβ]-6-[(S)-(1-Oxo-2(R)-acetylthio-3-phenylpropyl)amino]-1,2,3,5,6,7,11b-heptahydro-5-oxo-pyrrolo[2,1-a][2]benzazepine-2(R)-carboxylic acid, methyl ester

### Step a: N-(t-Butyloxycarbonyl)-2-(2-propenyl)-β-alanine, methyl ester

Dissolve diisopropylamine (5.6mL, 40mmol) in anhydrous tetrahydrofuran, cool to -78°C and place under nitrogen atmosphere. Treat with n-butyllithium (25mL of a 1.6M solution in hexane, 40mmol). Stir for 30 minutes and add, by dropwise addition, a solution of N-(t-butyloxycarbonyl)-β-alanine methyl ester (4.06g, 20mmol) in tetrahydrofuran (25mL) over 20 minutes. Stir for an additional 30 minutes. Add allyl bromide (1.72mL, 20mmol) and allow the mixture to warm to room temperature with stirring until homogeneous. Cool to -78°C and slowly warm to -20°C over 30 minutes. Pour into 5% hydrochloric acid, extract into ether, wash with saturated sodium chloride, dry, and concentrate to yield 5.01g yellow oil. Purify by silica gel chromatography (25% ethyl acetate/hexane) to yield 4.2g (87%) of a racemic mixture of the title compound as a clear colorless oil.

### Step b: 2-(2-Propenyl)-β-alanine, methyl ester

Dissolve N-(t-butyloxycarbonyl)-2-(2-propenyl)-β-alanine, methyl ester (5.6g, 23.0mmol) in methylene chloride (25mL) and treat with trifluoroacetic acid (15mL). Stir for three hours and remove the volatiles in vacuo. Treat the residue with water, make basic with sodium hydroxide (1.0g, 25mmol) and extract with ethyl acetate. Separate the organic phase and dry (Na₂SO₄). Evaporate to give 4.0g of the title compound as an oil in ethyl acetate.

### Step c: (S) -N-[2-(1,3-Dihydro-1,3-dioxo-2H-isoindol-2-yl)-1-oxo-3-phenylpropyl]-2 -(2-propenyl)-β-alanine, methyl esters

Stir a solution of N-phthaloyl-(S)-phenylalanine (7.5g, 25mmol) and EEDQ (7.0g, 28mmol) in methylene chloride (50mL). Add crude 2-(2-propenyl)-β-alanine, methyl ester (4.0g, 23mmol) and stir under nitrogen atmosphere for 2.5 days. Pour into ethyl acetate, wash with 10% hydrochloric acid (2X200mL), and saturated sodium hydrogen carbonate. Dry (MgSO₄) and concentrate. Treat the residue with ethyl acetate, dilute with hexane and allow to crystallize. Remove the solid to yield 5.33g (55%) of the diastereomeric title compounds as a white powder; mp 133-5°C.
Anal. Calcd for C₂₄H₂₄N₂O₅: C, 68.56; H, 5.75; N, 6.66;
Found: C, 68.32; H, 5.81; N, 6.47.

### Step d₁: (S)-N-[2-(1,3-Dihydro-1,3-dioxo-2H-isoindol-2-yl)]-(S)-phenylalanyl-2-(2-oxoethyl)-β-alanine, methyl esters

Dissolve the diastereomeric (S)-N-[2-(1,3-dihydro-1,3-dioxo-2H-isoindol-2-yl)-1-oxo-3-phenylpropyl]-2-(2-propenyl)-β-alanine, methyl esters (4.2g, 10mmol) in methylene chloride (150mL) and methanol (25mL). Cool to -70°C and treat with ozone until a blue color persists. Add dimethyl sulfide (7mL) and pyridine (2mL) and allow the solution to warm to 25°C, then stir for 18 hours. Dilute the solution with methylene chloride, wash with 10% hydrochloric acid, dry (MgSO₄) and concentrate to yield 4.5g of the title compound as a foam.

### Scheme A, Step d: (S)-N-[2-(1,3-Dihydro-1,3-dioxo-2H-isoindol-2-yl)]-1-oxo-3-phenylpropyl-1,2,3-trihydro-3 -pyrrolecarboxylic acid, methyl esters

Mix the (S)-N-[2-(1,3-dihydro-1,3-dioxo-2H-isoindol-2-yl)]-(S)-1-oxo-3-phenylpropyl-2-(2-oxoethyl)-β-alanine, methyl esters (4.5g), methylene chloride (150mL) and trifluoroacetic acid (0.5mL). Reflux for 8 hours and purify by silica gel chromatography (50% ethyl acetate/hexane) to yield 3.5g (87%) of the title compound.

### Scheme A, Step e: [6α(R*), 11bβ]-6-[(S)-(1,3-Dihydro-1,3-dioxo-2H-isoindol-2-yl)]-1,2,3,5,6,7,11b-heptahydro-5-oxo -pyrrolo[2,1-a][2]benzazepine-2(R)-carboxylic acid, methyl ester and [6α(R*), 11bβ]-6-[(S)-(1,3-Dihydro-1,3-dioxo-2H-isoindol-2-yl)]-1,2,3,5,6,7,11b-heptahydro-5-oxo -pyrrolo[2,1-a][2]benzazepine-2(S)-carboxylic acid, methyl ester

Place trifluoromethanesulfonic acid (10mL) in a flask under nitrogen atmosphere at 25°C. Add (S)-N-[2-(1,3-dihydro-1,3-dioxo-2H-isoindol-2-yl)]-1-oxo-3-phenylpropyl-1,2,3-trihydro-3-pyrrolecarboxylic acid, methyl esters (2.5g, 6mmol) in methylene chloride (10mL). Stir the mixture for 6 hours, pour into water and extract with ethyl acetate. Wash well with water, dry (MgSO₄) and concentrate to yield 2.3g foam. An additional 1.0g foam can be obtained by treating (S)-N-[2-(1,3-dihydro-1,3-dioxo-2H-isoindol-2-yl)]-1-oxo-3-phenylpropyl-1,2,3-trihydro-3-pyrrolecarboxylic acid, methyl ester (1.0g, from a second crop of the above experiment) with trifluoromethane-sulfonic acid (5mL) in methylene chloride (5mL), stirring under nitrogen atmosphere for 6 hours, and working-up as described above.

Combine both products, concentrate and allow to stand for 2 days. Take up the crude product in ethyl acetate to form a solid. Dilute with hexane and filter to yield 1.85g solid. Recrystallize (50mL hot ethyl acetate to 1:1 ethyl acetate/hexane) to yield 1.37g of the 2(R)-carboxylic acid title compound as colorless needles; mp 199-200°C.
Anal. Calcd for C₂₃H₂₀N₂O₅: C, 68.31; H, 4.98; N, 6.93;
Found: C, 68.01; H, 5.06; N, 6.80.

Purify the mother liquors by chromatography to give the 2(S)-carboxylic acid title compound.

### Scheme A, Step f: [6α(R*), 11bβ]-6-[(S)-Amino]-1,2,3,5,6,7,11b-heptahydro-5-oxo-pyrrolo[2,1 -a][2]benzazepine-2(R)-carboxylic acid, methyl ester

Mix [6α(R*), 11bβ]-6-[(S)-(1,3-dihydro-1,3-dioxo-2H-isoindol-2-yl)]-1,2,3,5,6,7,11b-heptahydro-5-oxo-pyrrolo[2,1-a][2]benzazepine-2(R)-carboxylic acid, methyl ester (1.37g, 3.38mmol), hydrazine monohydrate (389mg, 7.7mmol) and methanol (38mL) under nitrogen atmosphere. Heat at reflux for 5 hours. Cool to ambient temperature and filter to remove phthaloyl hydrazide. Evaporate the filtrate in vacuo to a residue and slurry in chloroform (60mL). Remove insoluble phthaloyl hydrazide by filtration and wash with chloroform (4X21mL). Wash the filtrate with water (4X50mL), dry (MgSO₄), and filter. Evaporate the filtrate to give the title compound.

### Scheme A, Step g: [6α(R*), 11bβ]-6-[(S)-(1-Oxo-2(S)-acetyloxy-3-phenylpropyl)amino]-1,2,3,5,6,7,11b-heptahydro -5 -oxo-pyrrolo[2,1-a][2]benzazepine-2(R)-carboxylic acid, methyl ester

Mix (S)-3-phenyl-2-acetyloxypropionic acid (3.6g, 17mmol) and [6α(R*), 11bβ]-6-[(S)-amino]-1,2,3,5,6,7,11b-heptahydro-5-oxo-pyrrolo[2,1-a][2]benzazepize-2(R)-carboxylic acid, methyl ester (4.64g, 17mmol) in methylene chloride (50mL). Add EEDQ (4.3g, 17mmol). Stir for 18 hours at ambient temperature under argon atmosphere. Extract with 2N hydrochloric acid, separate the organic phase, wash with water, then with saturated sodium hydrogen carbonate. Dry (MgSO₄), concentrate in vacuo and purify by silica gel chromatography to give the title compound.

### Scheme A, Step h: [6α(R*), 11bβ]-6-[(S)-(1-Oxo-2(S)-hydroxy-3-phenylpropyl)amino]-1,2,3,5,6,7,11b-heptahydro-5 -oxo-pyrrolo[2,1-a][2]benzazepine-2(R)-carboxylic acid, methyl ester

Dissolve [6α(R*), 11bβ]-6-[(S)-(1-oxo-2(S)-acetyloxy-3-phenylpropyl)amino]-1,2,3,5,6,7,11b-heptahydro-5-oxo-pyrrolo[2,1-a][2]benzazepine-2(R)-carboxylic acid, methyl ester (7.87g, 17.4mmol) in ethanol (75mL) and tetrahydrofuran (40mL) and add lithium hydroxide (22mL of a 1M solution, 22mmol). Stir the reaction mixture for 2 hours. Remove the solvent in vacuo at 35°C and partition the residue between ethyl acetate and 1N hydrochloric acid. Separate the organic phase, dry (MgSO₄), and concentrate in vacuo and treat with diazomethane. Purify by silica gel chromatography to give the title compound.

### Scheme A, Step i: [6α(R*), 11bβ]-6-[(S)-(1-Oxo-2(R)-acetylthio-3-phenylpropyl)amino]-1,2,3,5,6,7,11b-heptahydro -5-oxo-pyrrolo[2,1-a][2]benzazepine-2(R)-carboxylic acid, methyl ester

Mix DIAD ( 3.4g, 16.3mmol), triphenylphosphine (4.28g, 16.3mmol) and anhydrous tetrahydrofuran (200mL). Cool to 0°C and stir for 30 minutes under nitrogen atmosphere. Add a solution of [6α(R*), 11bβ]-6-[(S)-(1-oxo-2-(S)-hydroxy-3-phenylpropyl)amino]-1,2,3,5,6,7,11b-heptahydro-5-oxo-pyrrolo[2,1-a][2]benzazepine-2(R)-carboxylic acid, methyl ester (6.7g, 16.3mmol) and thiolacetic acid (1.75mL, 24.5mmol) in anhydrous tetrahydrofuran (200mL). Stir at 0°C for 30 minutes, then allow to warm to ambient temperature. Remove the volatiles in vacuo and purify by silica gel chromatography to give the title compound.

### Example 12

### Preparation of [6α(R*), 11bβ]-6-[(S)-(1-Oxo-2(R)-thio-3-phenylpropyl)amino]-1,2,3,5,6,7,11b-heptahydro-5-oxo -pyrrolo[2,1-a][2]benzazepine-2(R)-carboxylic acid

Dissolve [6α(R*), 11bβ]-6-[(S)-(1-oxo-2(R)-acetylthio-3-phenylpropyl)amino]-1,2,3,5,6,7,11b-heptahydro-5-oxo-pyrrolo[2,1-a][2]benzazepine-2(R)-carboxylic acid, methyl ester (56mg, 0.12mmol) in deoxygenated methanol (3mL) plus 1N lithium hydroxide (0.50mL, 0.50mmol). Stir for 30 minutes under argon atmosphere at ambient temperature. Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to give the title compound.

### Example 13

### Preparation of [6α(R*), 11bβ]-6-[(S)-(1-Oxo-2(R)-acetylthio-3-phenylpropyl)amino]-1,2,3,5,6,7,11b-heptahydro-5-oxo -pyrrolo[2,1-a][2]benzazepine-2(R)-carboxylic acid

Mix [6α(R*), 11bβ]-6-[(S)-(1-oxo-2(R)-thio-3-phenylpropyl)amino]-1,2,3,5,6,7,11b-heptahydro-5-oxo-pyrrolo[2,1-a][2]benzazepine-2(R)-carboxylic acid (51mg, 0.12mmol) and sulfuric acid (0.3mL of a 10% solution in acetic acid). Add acetic anhydride (11mg, 0.12mmol) over 10 minutes. Warm to 90°C with stirring for 45 minutes. Allow to cool, pour into diethyl ether and wash with water three times. Separate the organic phase, dry (MgSO₄) and concentrate in vacuo. Purify by silica gel chromatography to yield the title compound.

### Example 14

### Preparation of [6α(R*), 11bβ]-6-[(S)-(1-Oxo-2(R)-acetylthio-3-phenylpropyl)amino]-1,2,3,5,6,7,11b-heptahydro-5-oxo -pyrrolo[2,1-a][2]benzazepine-2(R)-carboxylic acid, benzyl ester

Dissolve [6α(R*), 11bβ]-6-[(S)-(1-oxo-2(R)-acetylthio-3-phenylpropyl)amino]-1,2,3,5,6,7,11b-heptahydro-5-oxo-pyrrolo[2,1-a][2]benzazepine-2(R)-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.0mmol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to yield the title compound.

### Example 15

### Preparation of [6α(R*), 11bβ]-6-[(S)-(1-Oxo-2(R)-thio-3-phenylpropyl)amino]-1,2,3,5,6,7,11b-heptahydro-5-oxo -pyrrolo[2,1-a][2]benzazepine-2(R)-carboxylic acid, benzyl ester

Stir [6α(R*), 11bβ]-6-[(S)-(1-oxo-2-(R)-acetylthio-3-phenylpropyl)amino]-1,2,3,5,6,7,11b-heptahydro-5-oxo-pyrrolo[2,1-a][2]benzazepine-2(R)-carboxylic acid, benzyl ester (1.87g, 4mmol) and saturated methanolic ammonia at ambient temperature until hydrolysis is complete. Evaporate the solvent in vacuo and purify by silica gel chromatography to give the title compound.

### Example 16

### Preparation of [6α(R*), 11bβ]-6-[(S)-(1-Oxo-2(R)-acetylthio-3-phenylpropyl)amino]-1,2,3,5,6,7,11b-heptahydro-5-oxo -pyrrolo[2,1-a][2]benzazepine-2(S)-carboxylic acid, methyl ester

### Scheme A, Step f: [6α(R*), 11bβ]-6-[(S)-Amino]-1,2,3,5,6,7,11b-heptahydro-5-oxo-pyrrolo[2,1 -a][2]benzazepine-2(S)-carboxylic acid, methyl ester

Mix [6α(R*), 11bβ]-6-[(S)-(1,3-dihydro-1,3-dioxo-2H-isoindol-2-yl)]-1,2,3,5,6,7,11b-heptahydro-5-oxo-pyrrolo[2,1-a][2]benzazepine-2(S)-carboxylic acid, methyl ester (see Example 11; Scheme A, Step e) (1.37g, 3.38mmol), hydrazine monohydrate (389mg, 7.7mmol) and methanol (38mL) under nitrogen atmosphere. Heat at reflux for 5 hours. Cool to ambient temperature and filter to remove phthaloyl hydrazide. Evaporate the filtrate in vacuo to a residue and slurry in chloroform (60mL). Remove insoluble phthaloyl hydrazide by filtration and wash with chloroform (4X21mL). Wash the filtrate with water (4X50mL), dry (MgSO₄), and filter. Evaporate the filtrate to give the title compound.

### Scheme A, Step g: [6α(R*), 11bβ]-6-[(S)-(1-Oxo-2(S)-acetyloxy-3-phenylpropyl)amino]-1,2,3,5,6,7,11b-heptahydro -5-oxo-pyrrolo[2,1-a][2]benzazepine-2(S)-carboxylic acid, methyl ester

Mix (S)-3-phenyl-2-acetyloxypropionic acid (3.6g, 17mmol) and [6α(R*), 11bβ]-6-[(S)-amino]-1,2,3,5,6,7,11b-heptahydro-5-oxo-pyrrolo[2,1-a][2]benzazepine-2(S)-carboxylic acid, methyl ester (4.64g, 17mmol) in methylene chloride (50mL). Add EEDQ (4.3g, 17mmol). Stir for 18 hours at ambient temperature under argon atmosphere. Extract with 2N hydrochloric acid, separate the organic phase, wash with water, then with saturated sodium hydrogen carbonate. Dry (MgSO₄), concentrate in vacuo and purify by silica gel chromatography to give the title compound.

### Scheme A, Step h: [6α(R*), 11bβ]-6-[(S)-(1-Oxo-2(S)-hydroxy-3-phenylpropyl)amino]-1,2,3,5,6,7,11b-heptahydro-5 -oxo-pyrrolo[2,1-a][2]benzazepine-2(S)-carboxylic acid, methyl ester

Dissolve [6α(R*), 11bβ]-6-[(S)-(1-oxo-2(S)-acetyloxy-3-phenylpropyl)amino]-1,2,3,5,6,7,11b-heptahydro-5-oxo-pyrrolo[2,1-a][2]benzazepine-2(S)-carboxylic acid, methyl ester (7.87g, 17.4mmol) in ethanol (75mL) and tetrahydrofuran (40mL) and add lithium hydroxide (22mL of a 1M solution, 22mmol). Stir the reaction mixture for 2 hours. Remove the solvent in vacuo at 35°C and partition the residue between ethyl acetate and 1N hydrochloric acid. Separate the organic phase, dry (MgSO₄), and concentrate in vacuo and treat with diazomethane. Purify by silica gel chromatography to give the title compound.

### Scheme A, Step i: [6α(R*), 11bβ]-6-[(S)-(1-Oxo-2(R)-acetylthio-3-phenylpropyl)amino]-1,2,3,5,6,7,11b-heptahydro -5-oxo-pyrrolo[2,1-a][2]benzazepine-2(S)-carboxylic acid, methyl ester

Mix DIAD ( 3.4g, 16.3mmol), triphenylphosphine (4.28g, 16.3mmol) and anhydrous tetrahydrofuran (200mL). Cool to 0°C and stir for 30 minutes under nitrogen atmosphere. Add a solution of [6α(R*), 11bβ]-6-[(S)-(1-oxo-2-(S)-hydroxy-3-phenylpropyl)amino]-1,2,3,5,6,7,11b-heptahydro-5-oxo-pyrrolo[2,1-a][2]benzazepine-2(S)-carboxylic acid, methyl ester (6.7g, 16.3mmol) and thiolacetic acid (1.75mL, 24.5mmol) in anhydrous tetrahydrofuran (200mL). Stir at 0°C for 30 minutes, then allow to warm to ambient temperature. Remove the volatiles in vacuo and purify by silica gel chromatography to give the title compound.

### Example 17

### Preparation of [6α(R*), 11bβ]-6-[(S)-(1-Oxo-2(R)-thio-3-phenylpropyl)amino]-1,2,3,5,6,7,11b-heptahydro-5-oxo -pyrrolo[2,1-a][2]benzazepine-2(S)-carboxylic acid

Dissolve [6a(R*), 11bβ]-6-[(S)-(1-oxo-2(R)-acetylthio-3-phenylpropyl)amino]-1,2,3,5,6,7,11b-heptahydro-5-oxo-pyrrolo[2,1-a][2]benzazepine-2(R)-carboxylic acid, methyl ester (56mg, 0.12mmol) in deoxygenated methanol (3mL) plus 1N lithium hydroxide (0.50mL, 0.50mmol). Stir for 30 minutes under argon atmosphere at ambient temperature. Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to give the title compound.

### Example 18

### Preparation of [6α(R*), 11bβ]-6-[(S)-(1-Oxo-2(R)-acetylthio-3-phenylpropyl)amino]-1,2,3,5,6,7,11b-heptahydro-5-oxo -pyrrolo[2,1-a][2]benzazepine-2(S)-carboxylic acid

Mix [6α(R*), 11bβ]-6-[(S)-(1-oxo-2(R)-thio-3-phenylpropyl)amino]-1,2,3,5,6,7,11b-heptahydro-5-oxo-pyrrolo[2,1-a][2]benzazepine-2(S)-carboxyl acid (51mg, 0.12mmol) and sulfuric acid (0.3mL of a 10% solution in acetic acid). Add acetic anhydride (11mg, 0.12mmol) over 10 minutes. Warm to 90°C with stirring for 45 minutes. Allow to cool, pour into diethyl ether and wash with water three times. Separate the organic phase, dry (MgSO₄) and concentrate in vacuo. Purify by silica gel chromatography to yield the title compound.

### Example 19

### Preparation of [6a(R*), 11bβ]-6-[(S)-(1-Oxo-2(R)-acetylthio-3phenylpropyl)amino]-1,2,3,5,6,7,11b-heptahydro-5-oxo -pyrrolo[2,1-a][2]benzazepine-2(S)-carboxylic acid, benzyl ester

Dissolve [6α(R*), 11bβ]-6-[(S)-(1-oxo-2(R)-acetylthio-3-phenylpropyl)amino]-1,2,3,5,6,7,11b-heptahydro-5-oxo-pyrrolo[2,1-a][2]benzazepine-2(S)-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.0mmol). Stir the resulting mixture at ambient temperature for 18 hours. Quench the reaction with ethyl acetate (50mL) and water (50mL). Separate the organic phase and wash with water (7x50mL), 1/4 saturated potassium hydrogen carbonate (50mL), water (50mL), and saturated sodium chloride (50mL). Dry (MgSO₄), filter and evaporate in vacuo to yield the title compound.

### Example 20

### Preparation of [6α(R*), 11bβ]-6-[(S)-(1-Oxo-2(R)-thio-3-phenylpropyl)amino]-1,2,3,5,6,7,11b-heptahydro-5-oxo -pyrrolo[2,1-a][2]benzazepine-2(S)-carboxylic acid, benzyl ester

Stir [6α(R*), 11bβ]-6-[(S)-(1-oxo-2-(R)-acetylthio-3-phenylpropyl)amino]-1,2,3,5,6,7,11b-heptahydro-5-oxo-pyrrolo[2,1-a][2]benzazepine-2(S)-carboxylic acid, benzyl ester (1.87g, 4mmol) and saturated methanolic ammonia at ambient temperature until hydrolysis is complete. Evaporate the solvent in vacuo and purify by silica gel chromatography to give the title compound.

### Example 21

### Preparation of [6α(R*), 11bβ]-6-[(S)-(1-Oxo-2(S)-acetylthio-3-phenylpropyl)amino]-1,2,3,5,6,7,11b-heptahydro-5-oxo -pyrrolo[2,1-a][2]benzazepine-2(R)-carboxylic acid, methyl ester

### Scheme A, Step j: [6α(R*), 11bβ]-6-[(S)-(1-Oxo-2(R)-hydroxy-3-phenylpropy)amino]-1,2,3,5,6,7,11b-heptahydro-5 -oxo-pyrrolo[2,1-a][2]benzazepine-2(R)-carboxylic acid, methyl ester

Dissolve [6α(R*), 11bβ]-6-[(S)-(1-oxo-2(S)-hydroxy-3-phenylpropyl)amino]-1,2,3,5,6,7,11b-heptahydro-5-oxo-pyrrolo[2,1-a][2]benzazepine-2(R)-carboxylic acid, methyl ester (see Example 11; Scheme A, Step h) (41mg, 0.1mmol), triphenylphosphine (39mg, 0.15mmol) and acetic acid (8.7µL, 0.15mmol) in anhydrous tetrahydrofuran (3mL). Treat with DIAD (32mg, 0.15mmol) at 0°C. Stir for 5 minutes at 0°C, then allow to stir at ambient temperature for 45 minutes. Remove the volatiles in vacuo and purify the residue by silica gel chromatography to give [6α(R*), 11bβ]-6-[(S)-(1-oxo-2(R)-acetyloxy-3-phenylpropyl)amino]-1,2,3,5,6,7,11b-heptahydro-5-oxo-pyrrolo[2,1-a][2]benzazepine-2(R)-carboxylic acid, methyl ester.

Dissolve [6α(R*), 11bβ]-6-[(S)(1-oxo-2(R)-acetyloxy-3-phenylpropyl)amino] -1,2,3,5,6,7,11b-heptahydro-5-oxo-pyrrolo[2,1-a][2]benzazepine-2(R)-carboxyl acid, methyl ester (262mg, 0.58mmol) in methanol (5mL) and tetrahydrofuran (5mL) and add lithium hydroxide (0.8mL of a 1M solution, 0.8mmoi). Stir the reaction mixture for 2 hours and remove the solvent in vacuo. Acidify and partition between methylene chloride and water. Separate the organic phase, dry (MgSO₄), concentrate in vacuo and purify by silica gel chromatography to give the title compound.

### Scheme A, Step k: [6α(R*), 11bβ]-6-[(S)-(1-Oxo-2(S)-acetylthio-3-phenylpropyl)amino]-1,2,3,5,6,7,11b-heptahydro -5-oxo-pyrrolo[2,1-a][2]benzazepine-2(R)-carboxylic acid, methyl ester

Mix DIAD (31mg, 0.15mmol), triphenylphosphine (39mg, 0.16mmol) and anhydrous tetrahydrofuran (2mL). Cool to 0°C and stir for 30 minutes under an argon atmosphere. Add [(6α(R*), 11bβ]-6-[(S)-(1-oxo-2(R)-hydroxy-3-phenylpropyl)amino]-1,2,3,5,6,7,11b-heptahydro-5-oxo-pyrrolo[2,1-a][2]benzazepine-2(R)-carboxylic acid, methyl ester (41mg, 0.1mmol) as a solid, then immediately add thiolacetic acid (11µL, 0.15mmol). Allow the reaction to warm to ambient temperature and stir overnight. Remove the solvent in vacuo and purify the residue by silica gel chromatography to give the title compound.

### Example 22

### Preparation of [6α(R*), 11bβ]-6-[(S)-(1-Oxo-2(S)-thio-3-phenylpropyl)amino]-1,2,3,5,6,7,11b-heptahydro-5-oxo -pyrrolo[2,1-a][2]benzazepine-2(R)-carboxylic acid

Dissolve [6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-2(R)-carboxylic acid, methyl ester (56mg, 0.12mmol) in deoxygenated methanol (3mL) plus 1N lithium hydroxide (0.50mL, 0.50mmol). Stir for 30 minutes under argon atmosphere at ambient temperature. 5 Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to give the title compound.

### Example 23

### Preparation of [6α(R*), 11bβ]-6-[(S)-1-Oxo-2(S)-acetylthio-3-phenylpropyl)amino]-1,2,3,5,6,7,11b-heptahydro-5-oxo -pyrrolo[2,1-a][2]benzazepine-2(R)-carboxylic acid

Mix [6α(R*), 11bβ]-6-[(S)-(1-oxo-2(S)-thio-3-phenylpropyl)amino]-1,2,3,5,6,7,11b-heptahydro-5-oxo-pyrrolo[2,1-a][2]benzazepine-2(R)-carboxylic acid (51mg, 0.12mmol) and sulfuric acid (0.3mL of a 10% solution in acetic acid). Add acetic anhydride (11mg, 0.12mmol) over 10 minutes. Warm to 90°C with stirring for 45 minutes. Allow to cool, pour into diethyl ether and wash with water three times. Separate the organic phase, dry (MgSO₄) and concentrate in vacuo. Purify by silica gel chromatography to give the title compound.

### Example 24

### Preparation of [6α(R*), 11bβ]-6-[(S)-(1-Oxo-2(S)-acetylthio-3-phenylpropyl)amino]-1,2,3,5,6,7,11b-heptahydro-5-oxo -pyrrolo[2,1-a][2]benzazepine-2(R)-carboxylic acid, benzyl ester

Dissolve [6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-2(R)-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.0mmol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to give the title compound.

### Example 25

### Preparation of [6α(R*), 11bβ]-6-[(S)-(1-Oxo-2(S)-thio-3-phenylpropyl)amino]-1,2,3,5,6,7,11b-heptahydro-5-oxo -pyrrolo[2,1-a][2]benzazepine-2(R)-carboxylic acid, benzyl ester

Stir [6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-2(R)-carboxylic acid, benzyl ester(1.87g, 4mmol) and saturated methanolic ammonia at ambient temperature until hydrolysis is complete. Evaporate the solvent in vacuo and purify by silica gel chromatography to give the title compound.

### Example 26

### Preparation of [6α(R*), 11bβ]-6-[(S)-(1-Oxo-2(S)-acetylthio-3-phenylpropyl)amino]-1,2,3,5,6,7,11b-heptahydro-5-oxo -pyrrolo[2,1-a][2]benzazepine-2(S)-carboxylic acid, methyl ester

### Scheme A, Step j: [6α(R*), 11bβ]-6-[(S)-(1-Oxo-2(R)-hydroxy-3-phenylpropyl)amino-1,2,3,5,6,7,11b-heptahydro-5 -oxo-pyrrolo[2,1-a][2]benzazepine-2(S)-carboxylic acid, methyl ester

Dissolve [6α(R*), 11bβ]-6-[(S)-(1-Oxo-2(S)-hydroxy-3-phenylpropyl)amino]-1,2,3,5,6,7,11b-heptahydro-5-oxo-pyrrolo[2,1-a][2]benzazepine-2(S)-carboxylic acid, methyl ester (see Example 16; Scheme A, Step h) (41mg, 0.1mmol), triphenylphosphine (39mg, 0.15mmol) and acetic acid (8.7µL, 0.15mmol) in anhydrous tetrahydrofuran (3mL). Treat with DIAD (32mg, 0.15mmol) at 0°C. Stir for 5 minutes at 0°C, then allow to stir at ambient temperature for 45 minutes. Remove the volatiles in vacuo and purify the residue by silica gel chromatography to give [6α(R*), 11bβ]-6-[(S)-(1-oxo-2(R)-acetyloxy-3-phenylpropyl)amino]-1,2,3,5,6,7,11b-heptahydro-5-oxo-pyrrolo[2,1-a][2]benzazepine-2(S)-carboxylic acid, methyl ester.

Dissolve [6α(R*), 11bβ]-6-[(S)(1-oxo-2(R)-acetyloxy-3-phenylpropyl)amino]-1,2,3,5,6,7,11b-heptahydro-5-oxo-pyrrolo[2,1-a][2]benzazepine-2(S)-carboxylic acid, methyl ester (262mg, 0.58mmol) in methanol (5mL) and tetrahydrofuran (5mL) and add lithium hydroxide (0.8mL of a 1M solution, 0.8mmol). Stir the reaction mixture for 2 hours and remove the solvent in vacuo. Acidify and partition between methylene chloride and water. Separate the organic phase, dry (MgSO₄), concentrate in vacuo and purify by silica gel chromatography to give the title compound.

### Scheme A, Step k: [6α(R*), 11bβ]-6-[(S)-(1-Oxo-2(S)-acetylthio-3-phenylpropyl)amino]-1,2,3,5 6,7,11b-heptahydro-5-oxo-pyrrolo[2,1-a][2]benzazepine-2(S)-carboxylic acid, methyl ester

Mix DIAD (31mg, 0.15mmol), triphenylphosphine (39mg, 0.16mmol) and anhydrous tetrahydrofuran (2mL). Cool to 0°C and stir for 30 minutes under an argon atmosphere. Add [6α(R*), 11bβ]-6-[(S)-(1-oxo-2(R)-hydroxy-3-phenylpropyl)amino]-1,2,3,5,6,7,11b-heptahydro-5-oxo-pyrrolo [2,1-a][2]benzazepine-2(S)-carboxylic acid, methyl ester (41mg, 0.1mmol) as a solid, then immediately add thiolacetic acid (11µL, 0.15mmol). Allow the reaction to warm to ambient temperature and stir overnight. Remove the solvent in vacuo and purify the residue by silica gel chromatography to give the title compound.

### Example 27

### Preparation of [6α(R*), 11bβ]-6-[(S)-(1-Oxo-2(S)-thio-3-phenylpropyl)amino]-1,2,3,5,6,7,11b-heptahydro-5-oxo -pyrrolo[2,1-a][2]benzazepine-2(S)-carboxylic acid

Dissolve [6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-2(S)-carboxylic acid, methyl ester (56mg, 0.12mmol) in deoxygenated methanol (3mL) plus 1N lithium hydroxide (0.50mL, 0.50mmol). Stir for 30 minutes under argon atmosphere at ambient temperature. Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to give the title compound.

### Example 28

### Preparation of [6α(R*), 11bβ]-6-[(S)-(1-Oxo-2(S)-acetylthio-3-phenylpropyl)amino]-1,2,3,5,6,7,11b-heptahydro-5-oxo -pyrrolo[2,1-a][2]benzazepine-2(S)-carboxylic acid

Mix [6α(R*), 11bβ]-6-[(S)-(1-oxo-2(S)-thio-3-phenylpropyl)amino]-1,2,3,5,6,7,11b-heptahydro-5-oxo-pyrrolo[2,1-a][2]benzazepine-2(S)-carboxylic acid (51mg, 0.12mmol) and sulfuric acid (0.3mL of a 10% solution in acetic acid). Add acetic anhydride (11mg, 0.12mmol) over 10 minutes. Warm to 90°C with stirring for 45 minutes. Allow to cool, pour into diethyl ether and wash with water three times. Separate the organic phase, dry (MgSO₄) and concentrate in vacuo. Purify by silica gel chromatography to give the title compound.

### Example 29

### Preparation of [6α(R*), 11bβ]-6 -[(S)-(1-Oxo-2(S)-acetylthio-3-phenylpropyl)amino]-1,2,3,5,6,7,11b-heptahydro-5-oxo -pyrrolo[2,1-a][2]benzazepine-2(S)-carboxylic acid, benzyl ester

Dissolve [6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-2(S)-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.0mmol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to give the title compound.

### Example 30

### Preparation of [6α(R*), 11bβ]-6-[(S)-(1-Oxo-2(S)-thio-3-phenylpropyl)amino]-1,2,3,5,6,7,11b-heptahydro-5-oxo -pyrrolo[2,1-a][2]benzazepine-2(S)-carboxylic acid, benzyl ester

Stir [6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-2(S)-carboxylic acid, benzyl ester(1.87g, 4mmol) and saturated ethanolic ammonia at ambient temperature until hydrolysis is complete. Evaporate the solvent in vacuo and purify by silica gel chromatography to give the title compound.

### Example 31

### Preparation of [7α(R*), 12bβ]-7-[(S)-(1-Oxo-2(R)-acetylthio-3-phenylpropyl)amino]-1,2,3,4,6,7,8,12b-octahydro-6-oxo -pyrido[2,1-a][2]benzazepine-3(R)-carboxylic acid, methyl ester

### Step d₂: (S)-N-[2-(1,3-Dihydro-1,3-dioxo-2H-isoindol-2-yl)-1-oxo-3-phenylpropyl]-2-(3-oxopropyl)-β-alanine, methyl esters

Mix (S)-N-[2-(1,3-dihydro-1,3-dioxo-2H-isoindol-2-yl)-1-oxo-3-phenylpropyl]-2-(2-propenyl)-β-alanine, methyl ester (4.2g, 10mmol) in anhydrous tetrahydrofuran (5mL) under nitrogen atmosphere. Add, by dropwise addition, a solution of 9-BBN (20mL of a 0.5M solution in tetrahydrofuran, 10mmol). Stir until hydroboration is complete. Add 30% hydrogen peroxide (0.2mL) and 10N sodium hydroxide (2.0mL), then isolate the alcohol.
Mix oxalyl chloride (2mL, 20mmole) and methylene chloride (40mL) and place under nitrogen atmosphere. Cool below - 50°C and add a solution of dimethyl sulfoxide (1.6g, 20mmol) in methylene chloride (1.5mL). Stir for 15 minutes and add a solution of the above alcohol (10mmol) in methylene chloride, keeping the pot temperature below -50°C for 30 minutes. Add triethylamine (8mL, 60mmol) over 30 minutes. Stir while warming to 0°C, quench with water, extract with ethyl acetate and dry (MgSO₄). Purify by silica gel chromatography to give the diastereomeric title compounds.

### Scheme A, Step d: (S)-N-[2-(1,3-Dihydro-1,3-dioxo-2H-isoindol-2-yl)]-1-oxo-3-phenylpropyl-1,2,3,4-tetrahydro-3 -pyridinecarboxylic acid, methyl esters

Mix (S)-N-[2-(1,3-dihydro-1,3-dioxo-2H-isoindol-2-yl)]-(S)-1-oxo-3-phenylpropyl]-2-(3-oxopropyl)-β-alanine, methyl esters (4.5g), methylene chloride (150mL) and trifluoroacetic acid (0.5mL). Reflux for 8 hours and purify by silica gel chromatography (50% ethyl acetate/hexane) to yield 3.5g (87%) diastereomeric title compounds.

### Scheme A, Step e: [7α(R*), 12bβ]-7-[(S)-(1,3-Dihydro-1,3-dioxo-2H-isoindol-2-yl)]-1,2,3,4,6,7,8,12b-octahydro-6-oxo -pyrido[2,1-a][2]benzazepine-3(R)-carboxylic acid, methyl ester and [7α(R*), 12bβ]-7-[(S)-(1,3-Dihydro-1,3-dioxo-2H-isoindol-2-yl)]-1,2,3,4,6,7,8,12b-octahydro-6-oxo -pyrido[2,1-a][2]benzazepine-3(S)-carboxylic acid, methyl ester

Place trifluoromethanesulfonic acid (10mL) in a flask under nitrogen atmosphere at 25°C. Add (S)-N-[2-(1,3-dihydro-1,3-dioxo-2H-isoindol -2-yl)]-1-oxo-3-phenylpropyl-1,2,3,4-tetrahydro-3-pyridinecarboxylic acid, methyl ester (2.5g, 6mmol) in methylene chloride (10mL). Stir the mixture for 6 hours until the reaction is complete. Pour into water and extract with ethyl acetate. Wash well with water, dry (MgSO₄) and concentrate to yield the diastereomeric title compounds. Separate by HPLC.

### Scheme A, Step f: [7α(R*), 12bβ]-7-[(S)-Amino]-1,2,3,4,6,7,8,12b-octahydro-6-oxo-pyrido[2,1 -a][2]benzazepine-3(R)-carboxylic acid, methyl ester

Mix [7α(R*), 12bβ]-7-[(S)-(1,3-dihydro-1,3-dioxo-2H-isoindol-2-yl)]-1,2,3,4,6,7,8,12b-octahydro-6-oxo-pyrido[2,1-a][2]benzazepine-3(R)-carboxylic acid, methyl ester (1.37g, 3.38mmol), hydrazine monohydrate (389mg, 7,7mmol) and methanol (38mL) under nitrogen atmosphere. Heat at reflux for 5 hours. Cool to ambient temperature and filter to remove phthaloyl hydrazide. Evaporate the filtrate in vacuo to a residue and slurry in chloroform (60mL). Remove insoluble phthaloyl hydrazide by filtration and wash with chloroform (4X20mL). Wash the filtrate with water (4X50mL), dry (MgSO₄), and filter. Evaporate the filtrate to yield the title compound.

### Scheme A, Step g: [7α(R*), 12bβ]-7-[(S)-1-Oxo-2(S)-acetyloxy-3-phenylpropyl)amino]-1,2,3,4,6 7,8 12b-octahydro-6-oxo-pyrido[2,1-a][2]benzazepine-3(R)-carboxylic acid, methyl ester

Mix (S)-3-pbenyl-2-acetyloxypropionic acid (3.6g, 17mmol) and [7α(R*), 12bβ]-7-[(S)-amino]-1,2,3,4,6,7,8,12b-octahydro-6-oxo-pyrido[2,1-a][2]benzazepine-3(R)-carboxylic acid, methyl ester (4.64g, 17mmol) in methylene chloride (50mL). Add EEDQ (4.3g, 17mmol). Stir for 18 hours at ambient temperature under argon atmosphere. Extract with 2N hydrochloric acid, separate the organic phase, wash with water, then with saturated sodium hydrogen carbonate. Dry (MgSO₄), concentrate in vacuo and purify by silica gel chromatography to give the title compound.

### Scheme A, Step h: [7α(R*), 12bβ]-7-[(S)-(1-Oxo-2(S)-hydroxy-3-phenylpropyl)amino]-1,2,3,4,6,7,8,12b-octahydro-6 -oxo-pyrido[2,1-a][2]benzazepine-3(R)-carboxylic acid, methyl ester

Dissolve [7α(R*), 12bβ]-7-[(S)-(1-oxo-2(S)-acetyloxy-3-phenylpropyl)amino] -1,2,3,4,6,7,8,12b-octahydro-6-oxo-pyrido[2,1-a][2]benzazepine-3(R)-carboxylic acid, methyl ester (7.87g, 17.4mmol) in ethanol (75mL) and tetrahydrofuran (40mL) and add lithium hydroxide (22mL of a 1M solution, 22mmol). Stir the reaction mixture for 2 hours. Remove the solvent in vacuo at 35°C and partition the residue between ethyl acetate and 1N hydrochloric acid. Separate the organic phase, dry (MgSO₄), and concentrate in vacuo. Purify by silica gel chromatography to give the title compound.

### Scheme A, Step i: [7α (R*), 12bβ]-7-[(S)-(1-Oxo-2(R)-acetylthio-3-phenylpropyl)amino]-1,2,3,4,6,7,8,12b -octahydro-6-oxo-pyrido[2,1-a]l[2]benzazepine-3(R)-carboxylic acid, methyl ester

Mix DIAD ( 3.4g, 16.3mmol), triphenylphosphine (4.28g, 16.3mmol) and anhydrous tetrahydrofuran (200mL). Cool to 0°C and stir for 30 minutes under nitrogen atmosphere. Add a solution of [7α(R*), 12bβ]-7-[(S)-(1-oxo-2(S)-hydroxy-3-phenylpropyl)amino]-1,2,3,4,6,7,8,12b-octahydro-6-oxo-pyrido[2,1-a][2]benzazepine-3(R)-carboxylic acid, methyl ester (6.7g, 16.3mmol) and thiolacetic acid (1.75mL, 24.5mmol) in anhydrous tetrahydrofuran (200mL). Stir at 0°C for 30 minutes, then allow to warm to ambient temperature. Remove the volatiles in vacuo and purify by silica gel chromatography to give the title compound.

### Example 32

### Preparation of [7α(R*), 12bβ]-7-[(S)-(1-Oxo-2(R)-thio-3-phenylpropyl)amino]-1,2,3,4,6,7,8,12b-octahydro-6-oxo -pyrido[2,1-a][2]benzazepine-3(R)-carboxylic acid

Dissolve [7α(R*), 12bβ]-7-[(S)-(1-oxo-2(R)-acetylthio-3-phenylpropyl)amino]-1,2,3,4,6,7,8,12b-octahydro-6-oxo-pyrido[2,1-a][2]benzazepine-3(R)-carboxylic acid, methyl ester (56mg, 0.12mmol) in deoxygenated methanol (3mL) plus 1N lithium hydroxide (0.50mL, 0.50mmol). Stir for 30 minutes under argon atmosphere at ambient temperature. Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to give the title compound.

### Example 33

### Preparation of [7α(R*), 12bβ]-7-[(S)-(1-Oxo-2(R)-acetylthio-3-phenylpropyl)amino]-1,2,3,4,6,7,8,12b-octahydro-6-oxo -pyrido[2,1-a][2]benzazepine-3(R)-carboxylic acid

Mix [7α(R*), 12bβ]-7-[(S)-(1-oxo-2(R)-thio-3-phenylpropyl)amino] -1,2,3,4,6,7,8,12b-octahydro-6-oxo-pyrido[2,1-a][2]benzazepine-3(R)-carboxylic acid (51mg, 0.12mmol) and sulfuric acid (0.3mL of a 10% solution in acetic acid). Add acetic anhydride (11mg, 0.12mmol) over 10 minutes. Warm to 90°C with stirring for 45 minutes. Allow to cool, pour into diethyl ether and wash with water three times. Separate the organic phase, dry (MgSO₄) and concentrate in vacuo. Purify by silica gel chromatography to give the title compound.

### Example 34

### Preparation of [7α(R*), 12bβ]-7-[(S)-(1-Oxo-2(R)-acetylthio-3-phenylpropyl)amino]-1,2,3,4,6,7,8,12b-octahydro-6-oxo -pyrido[2,1-a][2]benzazepine-3(R)-carboxylic acid, benzyl ester

Dissolve [7α(R*), 12bβ]-7-[(S)-(1-oxo-2(R)-acetylthio-3-phenylpropyl)amino]-1,2,3,4,6,7,8,12b-octahydro-6-oxo-pyrido[2,1-a][2]benzazepine-3(R)-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.0mmol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to yield the title compound.

### Example 35

### Preparation of [7α(R*), 12bβ]-7-[(S)-(1-Oxo-2(R)-thio-3-phenylpropyl)amino]-1,2,3,4,6,7,8,12b-octahydro-6-oxo -pyrido[2,1-a][2]benzazepine-3(R)-carboxylic acid, benzyl ester

Stir [7α(R*), 12bβ]-7-[(S)-(1-oxo-2(R)-acetylthio-3-phenylpropyl)amino]-1,2,3,4,6,7,8,12b-octahydro-6-oxo-pyrido[2,1-a][2]benzazepine-3(R)-carboxylic acid, benzyl ester(1.87g, 4mmol) and saturated methanolic ammonia at ambient temperature until hydrolysis is complete. Evaporate the solvent in vacuo and purify by silica gel chromatography to give the title compound.

### Example 36

### Preparation of [7α(R*), 12bβ]-7-[(S) -(1-Oxo-2(R)-acetylthio-3-phenylpropyl)amino]-1,2,3,4,6,7,8,12b-octahydro-6-oxo -pyrido[2,1-a][2]benzazepine-3(S)-carboxylic acid, methyl ester

### Scheme A, Step f: [7α(R*), 12bβ]-7-[(S)-Amino]-1,2,3,4,6,7,8,12b-octahydro-6-oxo-pyrido[2,1 -a][2]benzazepine-3(S)-carboxylic acid, methyl ester

Mix [7α(R*), 12bβ]-7-[(S)-(1,3-dihydro-1,3-dioxo-2H-isoindol-2-yl)]-1,2,3,4,6,7,8,12b-octahydro-6-oxo-pyrido[2,1-a][2]benzazepine-3(S)-carboxylic acid, methyl ester (see Example 31;Scheme A, Step e) (1.37g, 3.38mmol), hydrazine monohydrate (389mg, 7.7mmol) and methanol (38mL) under nitrogen atmosphere. Heat at reflux for 5 hours. Cool to ambient temperature and filter to remove phthaloyl hydrazide. Evaporate the filtrate in vacuo to a residue and slurry in chloroform (60mL). Remove insoluble phthaloyl hydrazide by filtration and wash with chloroform (4X20mL). Wash the filtrate with water (4X50mL), dry (MgSO₄), and filter. Evaporate the filtrate to yield the title compound.

### Scheme A, Step c: [7α(R*), 12bβ]-7-[(S)-(1-Oxo-2(S)-acetyloxy-3(S)-phenylpropyl)amino]-1,2,3,4,6,7,8,12b -octahydro-6-oxo-pyrido[2,1-a][2]benzazepine-3(S)-carboxylic acid, methyl ester

Mix (S)-3-phenyl-2-acetyloxypropionic acid (3.6g, 17mmol) and [7α(R*), 12bβ]-7-[(S)-amino]-1,2,3,4,6,7,8,12b-octahydro-6-oxo-pyrido[2,1-a][2]benzazepine-3(S)-carboxylic acid, methyl ester (4.64g, 17mmol) in methylene chloride (50mL). Add EEDQ (4.3g, 17mmol). Stir for 18 hours at ambient temperature under argon atmosphere. Extract with 2N hydrochloric acid, separate the organic phase, wash with water, then with saturated sodium hydrogen carbonate. Dry (MgSO₄), concentrate in vacuo and purify by silica gel chromatography to give the title compound.

### Scheme A, Step h: [7α(R*), 12bβ]-7-[(S)-(1-Oxo-2(S)-hydroxy-3-phenylpropyl)amino]-1,2,3,4,6,7,8,12b-octahydro-6 -oxo-pyrido[2,1-a][2]benzazepine-3(S)-carboxylic acid, methyl ester

Dissolve [7α(R*), 12bβ]-7-[(S)-(1-oxo-2(S)-acetyloxy-3-phenylpropyl)amino]-1,2,3,4,6,7,8,12b-octahydro-6-oxo-pyrido[2,1-a][2]benzazepine-3(S)-carboxylic acid, methyl ester (7.87g, 17.4mmol) in ethanol (75mL) and tetrahydrofuran (40mL) and add lithium hydroxide (22mL of a 1M solution, 22mmol). Stir the reaction mixture for 2 hours. Remove the solvent in vacuo at 35°C and partition the residue between ethyl acetate and 1N hydrochloric acid. Separate the organic phase, dry (MgSO₄), and concentrate in vacuo. Purify by silica gel chromatography to give the title compound.

### Scheme A, Step i: [7α(R*), 12bβ]-7-[(S)-(1-Oxo-2(R)-acetylthio-3-phenylpropyl)amino]-1,2,3,4,6,7,8,12b -octahydro-6-oxo-pyrido[2,1-a][2]benzazepine-3(S)-carboxylic acid, methyl ester

Mix DIAD ( 3.4g, 16.3mmol), triphenylphosphine (4.28g, 16.3mmol) and anhydrous tetrahydrofuran (200mL). Cool to 0°C and stir for 30 minutes under nitrogen atmosphere. Add a solution of [7α(R*), 12bβ]-7-[(S)-(1-oxo-2(S)-hydroxy-3-phenylpropyl)amino]-1,2,3,4,6,7,8,12b-octahydro-6-oxo-pyrido[2,1-a][2]benzazepine-3(S)-carboxylic acid, methyl ester (6.7g, 16.3mmol) and thiolacetic acid (1.75mL, 24.5mmol) in anhydrous tetrahydrofuran (200mL). Stir at 0°C for 30 minutes, then allow to warm to ambient temperature. Remove the volatiles in vacuo and purify by silica gel chromatography to give the title compound.

### Example 37

### Preparation of [7α(R*), 12bβ]-7-[(S)-(1-Oxo-2(R)-thio-3-phenylpropyl)amino]-1,2,3,4,6,7,8,12b-octahydro-6-oxo -pyrido[2,1-a][2]benzazepine-3(S)-carboxylic acid

Dissolve [7α(R*), 12bβ]-7-[(S)-(1-oxo-2(R)-acetylthio-3-phenylpropyl)amino]-1,2,3,4,6,7,8,12b-octahydro-6-oxo-pyrido[2,1-a][2]benzazepine-3(S)-carboxylic acid, methyl ester (56mg, 0.12mmol) in deoxygenated methanol (3mL) plus 1N lithium hydroxide (0.50mL, 0.50mmol). Stir for 30 minutes under argon atmosphere at ambient temperature. Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to give the title compound.

### Example 38

### Preparation of [7α(R*), 12bβ]-7-[(S)-(1-Oxo-2(R)-acetylthio-3-phenylpropyl)amino]-1,2,3,4,6,7,8,12b-octahydro-6-oxo -pyrido[2,1-a][2]benzazepine-3(S)-carboxylic acid

Mix [7α(R*), 12bβ]-7-[(S)-(1-oxo-2(R)-thio-3-phenylpropyl)amino]-1,2,3,4,6,7,8,12b-octahydro-6-oxo-pyrido[2,1-a][2]benzazepine-3(S)-carboxylic acid (51mg, 0.12mmol) and sulfuric acid (0.3mL of a 10% solution in acetic acid). Add acetic anhydride (11mg, 0.12mmol) over 10 minutes. Warm to 90°C with stirring for 45 minutes. Allow to cool, pour into diethyl ether and wash with water three times. Separate the organic phase, dry (MgSO₄) and concentrate in vacuo. Purify by silica gel chromatography to give the title compound.

### Example 39

### Preparation of [7α(R*), 12bβ]-7-[(S)-(1-Oxo-2(R)-acetylthio-3-phenylpropyl)amino]-1,2,3,4,6,7,8,12b-octahydro-6-oxo -pyrido[2,1-a][2]benzazepine-3(S)-carboxylic acid, benzyl ester

Dissolve [7α(R*), 12bβ]-7-[(S)-(1-oxo-2(R)-acetylthio-3-phenylpropyl)amino]-1,2,3,4,6,7,8,12b-octahydro-6-oxo-pyrido[2,1-a][2]benzazepine-3(S)-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.Ommol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to yield the title compound.

### Example 40

### Preparation of [7α(R*), 12bβ]-7-[(S)-(1-Oxo-2(R)-thio-3-phenylpropyl)amino]-1,2,3,4,6,7,8,12b-octahydro-6-oxo -pyrido[2,1-a][2]benzazepine-3(S)-carboxylic acid, benzyl ester

Stir [7α(R*), 12bβ]-7-[(S)-(1-oxo-2(R)-acetylthio-3-phenylpropyl)amino]-1,2,3,4,6,7,8,12b-octahydro-6-oxo-pyrido[2,1-a][2]benzazepine-3(S)-carboxylic acid, benzyl ester(1.87g, 4mmol) and saturated methanolic ammonia at ambient temperature until hydrolysis is complete. Evaporate the solvent in vacuo and purify by silica gel chromatography to give the title compound.

### Example 41

### Preparation of [7α(R*), 12bβ]-7-[(S)-(1-Oxo-2(S)-acetylthio-3-phenylpropyl)amino]-1,2,3,4,6,7,8,12b-octahydro-6-oxo -pyrido[2,1-a][2]benzazepine-3(R)-carboxylic acid, methyl ester

### Scheme A, Step j: [7α(R*), 12bβ]-7-[(S)-(1-Oxo-2(R)-hydroxy-3-phenylpropyl)amino]-1,2,3,4,6,7,8,12b-octahydro-6 -oxo-pyrido[2,1-a][2]benzazepine-3(R)-carboxylic acid, methyl ester

Dissolve [7α(R*), 12bβ]-7-[(S)-(1-oxo-2(S)-hydroxy-3-phenylpropyl)amino]-1,2,3,4,6,7,8,12b-octahydro-6-oxo-pyrido[2,1-a][2]benzazepine-3(R)-carboxylic acid, methyl ester (see Example 31; Scheme A, Step h) (41mg, 0.1mmol), triphenylphosphine (39mg, 0.15mmol) and acetic acid (8.7µL, 0.15mmol) in anhydrous tetrahydrofuran (3mL). Treat with DIAD (32mg, 0.15mmol) at 0°C. Stir for 5 minutes at 0°C, then allow to stir at ambient temperature for 45 minutes. Remove the volatiles in vacuo and purify the residue by silica gel chromatography to give [7α(R*), 12bβ]-7-[(S)-(1-oxo-2(R)-acetyloxy-3-phenylpropyl)amino]-1,2,3,4,6,7,8,12b-octahydro-6-oxo-pyrido[2,1-a][2]benzazepine-3(R)-carboxylic acid, methyl ester.

Dissolve [7α(R*), 12bβ]-7-[(S)-(1-oxo-2(R)-acetyloxy-3-phenylpropyl)amino]-1,2,3,4,6,7,6,12b-octahydro-6-oxo-pyrido[2,1-a][2]benzazepine-3(R)-carboxylic acid, methyl ester (262mg, 0.58mmol) in methanol (5mL) and tetrahydrofuran (5mL) and add lithium hydroxide (0.8mL of a 1M solution, 0.8mmol). Stir the reaction mixture for 2 hours and remove the solvent in vacuo. Acidify and partition between methylene chloride and water. Separate the organic phase, dry (MgSO₄), concentrate in vacuo and purify by silica gel chromatography to give the title compound.

### Scheme A, Step k: [7α(R*), 12bβ]-7-[(S)-(1-Oxo-2(S)-acetylthio-3-phenylpropyl)amino]-1,2,3,4,6,7,8,12b -octahydro-6-oxo-pyrido[2,1-a][2]benzazepine-3(R)-carboxylic acid, methyl ester

Mix DIAD (31mg, 0,15mmol), triphenylphosphine (39mg, 0.16mmol) and anhydrous tetrahydrofuran (2mL). Cool to 0°C and stir for 30 minutes under an argon atmosphere. Add [7α(R*), 12bβ]-7-[(S)-(1-oxo-2(R)-hydroxy-3-phenylpropyl)amino]-1,2,3,4,6,7,8,12b-octahydro-6-oxo-pyrido[2,1-a][2]benzazepine-3(R)-carboxylic acid, methyl ester (41mg, 0.1.mmol) as a solid, then immediately add thiolacetic acid (11µL, 0.15mmol). Allow the reaction to warm to ambient temperature and stir overnight. Remove the solvent in vacuo and purify the residue by silica gel chromatography to give the title compound.

### Example 42

### Preparation of [7α(R*), 12bβ]-7-[(S)-(1-Oxo-2(S)-thio-3-phenylpropyl)amino]-1,2,3,4,6,7,8,12b-octahydro-6-oxo -pyrido[2,1-a][2]benzazepine-3(R)-carboxylic acid

Dissolve [7α(R*), 12bβ]-7-[(S)-(1-oxo-2(S)-acetylthio-3-phenylpropyl)amino]-1,2,3,4,6,7,8,12b-octahydro-6-oxo-pyrido[2,1-a][2]benzazepine-3(R)-carboxylic acid, methyl ester (56mg, 0.12mmol) in deoxygenated methanol (3mL) plus 1N lithium hydroxide (0.50mL, 0.50mmol). Stir for 30 minutes under argon atmosphere at ambient temperature. Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to give the title compound.

### Example 43

### Preparation of [7α(R*), 12bβ]-7-[(S)-(1-Oxo-2(S)-acetylthio-3-phenylpropyl)amino]-1,2,3,4,6,7,8,12b-octahydro-6-oxo -pyrido[2,1-a][2]benzazepine-3(R)-carboxylic acid

Mix [7α(R*), 12bβ]-7-[(S)-(1-oxo-2(S)-thio-3-phenylpropyl)amino]-1,2,3,4,6,7,8,12b-octahydro-6-oxo-pyrido[2,1-a][2]benzazepine-3(R)-carboxylic acid (51mg, 0.12mmol) and sulfuric acid (0.3mL of a 10% solution in acetic acid). Add acetic anhydride (11mg, 0.12mmol) over 10 minutes. Warm to 90°C with stirring for 45 minutes. Allow to cool, pour into diethyl ether and wash with water three times. Separate the organic phase, dry (MgSO₄) and concentrate in vacuo. Purify by silica gel chromatography to give the title compound.

### Example 44

### Preparation of [7α(R*), 12bβ]-7-[(S)-(1-Oxo-2(S)-acetylthio-3-phenylpropyl)amino]-1,2,3,4,6,7,8,12b-octahydro-6-oxo -pyrido[2,1-a][2]benzazepine-3(R)-carboxylic acid, benzyl ester

Dissolve [7α(R*), 12bβ]-7-[(S)-(1-oxo-2(S)-acetylthio-3-phenylpropyl)amino]-1,2,3,4,6,7,8,12b-octahydro-6-oxo-pyrido[2,1-a][2]benzazepine-3(R)-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.0mmol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to give the title compound.

### Example 45

### Preparation of [7α(R*), 12bβ]-7-(S)-(1-Oxo-2(S)-thio-3-phenylpropyl)amino]-1,2,3,4,6,7,8,12b-octahydro-6-oxo -pyrido[2,1-a]benzazepine-3(R)-carboxylic acid, benzyl ester

Stir [7α(R*), 12bβ]-7-[(S)-(1-oxo-2(S)-acetylthio-3-phenylpropyl)amino]-1,2,3,4,6,7,8,12b-octahydro-6-oxo-pyrido[2,1-a][2]benzazepine-3(R)-carboxylic acid, benzyl ester(1.87g, 4mmol) and saturated methanolic ammonia at ambient temperature until hydrolysis is complete. Evaporate the solvent in vacuo and purify by silica gel chromatography to give the title compound.

### Example 46

### Preparation of [7α(R*), 12bβ]-7-[(S)-(1-Oxo-2(S)-acetylthio-3-phenylpropyl)amino]-1,2,3,4,6,7,8,12b-octahydro-6-oxo -pyrido[2,1-a][2]benzazepine-3(S)-carboxylic acid, methyl ester

### Scheme A, Step j: [7α(R*), 12bβ]-7-[(S)-(1-Oxo-2(R)-hydroxy-3-phenylpropyl)amino]-1,2,3,4,6,7,8,12b-octahydro-6 -oxo-pyrido[2,1-a][2]benzazepine-3(S)-carboxylic acid, methyl ester

Dissolve [7α(R*), 12bβ]-7-[(S)-(1-oxo-2(S)-hydroxy-3-phenylpropyl)amino]-1,2,3,4,6,7,8,12b-octahydro-6-oxo-pyrido[2,1-a][2]benzazepine-3(S)-carboxylic acid, methyl ester (see Example 36; Scheme A, Step h) (41mg, 0.1mmol), triphenylphosphine (39mg, 0.15mmol) and acetic acid (8.7µL, 0.15mmol) in anhydrous tetrahydrofuran (3mL). Treat with DIAD (32mg, 0.15mmol) at 0°C. Stir for 5 minutes at 0°C, then allow to stir at ambient temperature for 45 minutes. Remove the volatiles in vacuo and purify the residue by silica gel chromatography to give [7α(R*), 12bβ]-7-[(S)-(1-oxo-2(R)-acetyloxy-3-phenylpropyl)amino]-1,2,3,4,6,7,8,12b-octahydro-6-oxo-pyrido[2,1-a][2]benzazepine-3(S)-carboxylic acid, methyl ester.

Dissolve [7α(R*), 12bβ]-7-[(S)-(1-oxo-2(R)-acetyloxy-3-phenylpropyl)amino]-1,2,3,4,6,7,8,12b-octahydro-6-oxo-pyrido[2,1-a][2]benzazepine-3(S)-carboxylic acid, methyl ester (262mg, 0.58mmol) in methanol (5mL) and tetrahydrofuran (5mL) and add lithium hydroxide (0.8mL of a 1M solution, 0.8mmol). Stir the reaction mixture for 2 hours and remove the solvent in vacuo. Acidify and partition between methylene chloride and water. Separate the organic phase, dry (MgSO₄), concentrate in vacuo and purify by silica gel chromatography to give the title compound.

### Scheme A, Step k: [7α(R*), 12bβ]-7-[(S)-(1-Oxo-2(S)-acetylthio-3-phenylpropyl)amino]-1,2,3,4,6,7,8,12b -octahydro-6-oxo-pyrido[2,1-a][2]benzazepine-3(S)-carboxylic acid, methyl ester

Mix DIAD (31mg, 0.15mmol), triphenylphosphine (39mg, 0.16mmol) and anhydrous tetrahydrofuran (2mL). Cool to 0°C and stir for 30 minutes under an argon atmosphere. Add [7α(R*), 12bβ]-7-[(S)-(1-oxo-2(R)-hydroxy-3-phenylpropyl)amino]-1,2,3,4,6,7,8,12b-octahydro-6-oxo-pyrido[2,1-a][2]benzazepine-3(S)-carboxylic acid, methyl ester (41mg, 0.1mmol) as a solid, then immediately add thiolacetic acid (11µL, 0.15mmol) . Allow the reaction to warm to ambient temperature and stir overnight. Remove the solvent in vacuo and purify the residue by silica gel chromatography to give the title compound.

### Example 47

### Preparation of [7α(R*), 12bβ]-7-[(S)-(1-Oxo-2(S)-thio-3-phenylpropyl)amino]-1,2,3,4,6,7,8,12b-octahydro-6-oxo -pyrido[2,1-a][2]benzazepine-3(S)-carboxylic acid

Dissolve [7α(R*), 12bβ]-7-[(S)-(1-oxo-2(S)-acetylthio-3-phenylpropyl)amino]-1,2,3,4,6,7,8,12b-octahydro-6-oxo-pyrido[2,1-a][2]benzazepine-3(S)-carboxylic acid, methyl ester (56mg, 0.12mmol) in deoxygenated methanol (3mL) plus 1N lithium hydroxide (0.50mL, 0.50mmol). Stir for 30 minutes under argon atmosphere at ambient temperature. Reduce in volume to 1.5mL in vacuo, then add, by dropwise addition, to a rapidly stirring solution of 2N hydrochloric acid (2mL). Collect the resulting precipitate, wash with water and dry in a vacuum dessicator for 1 hour. Dry at 35°C overnight to give the title compound.

### Example 48

### Preparation of [7α(R*), 12bβ]-7-[(S)-(1-Oxo-2(S)-acetylthio-3-phenylpropyl)amino]-1,2,3,4,6,7,8,12b-octahydro-6-oxo -pyrido[2,1-a][2]benzazepine-3(S)-carboxylic acid

Mix [7α(R*), 12bβ]-7-[(S)-(1-oxo-2(S)-thio-3-phenylpropyl)amino]-1,2,3,4,6,7,8,12b-octahydro-6-oxo-pyrido[2,1-a][2]benzazepine-3(S)-carboxylic acid (51mg, 0.12mmol) and sulfuric acid (0.3mL of a 10% solution in acetic acid). Add acetic anhydride (11mg, 0.12mmol) over 10 minutes. Warm to 90°C with stirring for 45 minutes. Allow to cool, pour into diethyl ether and wash with water three times. Separate the organic phase, dry (MgSO₄) and concentrate in vacuo. Purify by silica gel chromatography to give the title compound.

### Example 49

### Preparation of [7α(R*), 12bβ]-7-[(S)-(1-Oxo-2(S)-acetylthio-3-phenylpropyl)amino]-1,2,3,4,6,7,8,12b-octahydro-6-oxo -pyrido[2,1-a][2]benzazepine-3(S)-carboxylic acid, benzyl ester

Dissolve [7α(R*), 12bβ]-7-[(S)-(1-oxo-2(S)-acetylthio-3-phenylpropyl)amino]-1,2,3,4,6,7,8,12b-octahydro-6-oxo-pyrido[2,1-a][2]benzazepine-3(S)-carboxylic acid (2.08g, 4.33mmol) in methylene chloride (25mL) and dry over anhydrous MgSO₄. Filter and wash with methylene chloride (3X200mL). Evaporate in vacuo to a residue. Dissolve the residue in anhydrous dimethylformamide (25mL) and place under nitrogen atmosphere. Add cesium carbonate (1.65g, 5.0mmole) in one portion. Stir for 45 minutes at ambient temperature. Add benzyl bromide (550mg, 5.Ommol). Stir the resulting mixture at ambient temperature for 18 hours. Quench the reaction with ethyl acetate (50mL) and water (50mL). Separate the organic phase and wash with water (7X50mL), 1/4 saturated potassium hydrogen carbonate (50mL), water (50mL), and saturated sodium chloride (50mL). Dry (MgSO₄), filter and evaporate in vacuo to give the title compound.

### Example 50

### Preparation of [7α(R*), 12bβ]-7-[(S)-1-Oxo-2(S)-thio-3-phenylpropyl)amino]-1,2,3,4,6,7,8,12b-octahydro-6-oxo -pyrido[2,1-a][2]benzazepine-3(S)-carboxylic acid, benzyl ester

Stir [7α(R*), 12bβ]-7-[(S)-(1-oxo-2(S)-acetylthio-3-phenylpropyl)amino]-1,2,3,4,6,7,8,12b-octahydro-6-oxo-pyrido[2,1-a][2]benzazepine-3(S)-carboxylic acid, benzyl ester(1.87g, 4mmol) and saturated methanolic ammonia at ambient temperature until hydrolysis is complete. Evaporate the solvent in vacuo and purify by silica gel chromatography to give the title compound.

The following compounds can be prepared by procedures analogous to those described above in Example 11 -50:
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-2(R)-carboxylic acid, methyl ester;
[6α(R*), 11bβ]-6-[(S)-(1-oxo-2-thio-ethyl)amino]-1,2,3,5,6,7,11b-heptahydro-5-oxo-pyrrolo[2,1-a][2]benzazepine-2(R)-carboxylic acid;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-2(R)-carboxylic acid;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-2(R)-carboxylic acid, benzyl ester;
[6α(R*), 11bβ]-6-[(S)-(1-oxo-2-thio-ethyl)amino]-1,2,3,5,6,7,11b-heptahydro-5-oxo-pyrrolo[2,1-a][2]benzazepine-2(R)-carboxylic acid, benzyl ester;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-2(R)-carboxylic acid, methyl ester;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-2(R)-carboxylic acid;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-2(R)-carboxylic acid, benzyl ester;
[7α(R*), 12bβ]-7-[(S)-(1-oxo-2(R)-benzoylthio-3-phenylpropyl)amino]-1,2,3,4,6,7,8,12b-octahydro-6-oxo-pyrido[2,1-a][2]benzazepine-3(R)-carboxylic acid, methyl ester;
[7α(R*), 12bβ]-7-[(S)-(1-oxo-2-thio-ethyl)amino]-1,2,3,4,6,7,8,12b-octahydro-6-oxo-pyrido[2,1-a][2]benzazepine-3(R)-carboxylic acid;
[7α(R*), 12bβ]-7-[(S)-(1-oxo-2(R)-benzoylthio-3-phenylpropyl)amino]-1,2,3,4,6,7,8,12b-octahydro-6-oxo-pyrido[2,1-a][2]benzazepine-3(R)-carboxylic acid;
[7α(R*), 12bβ]-7-[(S)-(1-oxo-2(R)-benzoylthio-3-phenylpropyl)amino]-1,2,3,4,6,7,8,12b-octahydro-6-oxo-pyrido[2,1-a][2]benzazepine-3(R)-carboxylic acid, benzyl ester;
[7α(R*), 12bβ]-7-[(S)-(1-oxo-2-thio-ethyl)amino]-1,2,3,4,6,7,8,12b-octahydro-6-oxo-pyrido[2,1-a][2]benzazepine-3(R)-carboxylic acid, benzyl ester;
[7α(R*), 12bβ]-7-[(S)-(1-oxo-2(S)-benzoylthio-3-phenylpropyl)amino]-1,2,3,4,6,7,8,12b-octahydro-6-oxo-pyrido[2,1-a][2]benzazepine-3(R)-carboxylic acid, methyl ester;
[7α(R*), 12bβ]-7-[(S)-(1-oxo-2(S)-benzoylthio-3-phenylpropyl)amino]-1,2,3,4,6,7,8,12b-octahydro-6-oxo-pyrido[2,1-a][2]benzazepine-3(R)-carboxylic acid;
[7α(R*), 12bβ]-7-[(S)-(1-oxo-2(S)-benzoylthio-3-phenylpropyl)amino]-1,2,3,4,6,7,8,12b-octahydro-6-oxo-pyrido[2,1-a][2]benzazepine-3(R)-carboxylic acid, benzyl ester;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-2(S)-carboxylic acid, methyl ester;
[6α(R*), 11bβ]-6-[(S)-(1-oxo-2-thio-ethyl)amino]-1,2,3,5,6,7,11b-heptahydro-5-oxo-pyrrolo[2,1-a][2]benzazepine-2(S)-carboxylic acid;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-2(S)-carboxylic acid;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-2(S)-carboxylic acid, benzyl ester;
[6α(R*), 11bβ]-6-[(S)-(1-oxo-2-thio-ethyl)amino]-1,2,3,5,6,7,11b-heptahydro-5-oxo-pyrrolo[2,1-a][2]benzazepine-2(S)-carboxylic acid, benzyl ester;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-2(S)-carboxylic acid, methyl ester;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-2(S)-carboxylic acid;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-2(S)-carboxylic acid, benzyl ester;
[6α(R*), 12bβ]-7-[(S)-(1-oxo-2(R)-benzoylthio-3-phenylpropyl)amino]-1,2,3,4,6,7,8,12b-octahydro-6-oxo-pyrido[2,1-a][2]benzazepine-3(S)-carboxylic acid, methyl ester;
[7α(R*), 12bβ]-7-[(S)-(1-oxo-2-thio-ethyl)amino]-1,2,3,4,6,7,8,12b-octahydro-6-oxo-pyrido[2,1-a][2]benzazepine-3(S)-carboxylic acid;
[7α(R*), 12bβ]-7-[(S)-(1-oxo-2(R)-benzoylthio-3-phenylpropyl)amino]-1,2,3,4,6,7,8,12b-octahydro-6-oxo-pyrido[2,1-a][2]benzazepine-3(S)-carboxylic acid;
[7α(R*), 12bβ]-7-[(S)-(1-oxo-2(R)-benzoylthio-3-phenylpropyl)amino]-1,2,3,4,6,7,8,12b-octahydro-6-oxo-pyrido[2,1-a][2]benzazepine-3(S)-carboxylic acid, benzyl ester;
[7α(R*), 12bβ]-7-[(S)-(1-oxo-2-thio-ethyl)amino]-1,2,3,4,6,7,8,12b-octahydro-6-oxo-pyrido[2,1-a][2]benzazepine-3(S)-carboxylic acid, benzyl ester;
[7α(R*), 12bβ]-7-[(S)-(1-oxo-2(S)-benzoylthio-3-phenylpropyl)amino]-1,2,3,4,6,7,8,12b-octahydro-6-oxo-pyrido[2,1-a][2]benzazepine-3(S)-carboxylic acid, methyl ester;
[7α(R*), 12bβ]-7-[(S)-(1-oxo-2(S)-benzoylthio-3-phenylpropyl)amino]-1,2,3,4,6,7,8,12b-octahydro-6-oxo-pyrido[2,1-a][2]benzazepine-3(S)-carboxylic acid;
[7α(R*), 12bβ]-7-[(S)-(1-oxo-2(S)-benzoylthio-3-phenylpropyl)amino]-1,2,3,4,6,7,8,12b-octahydro-6-oxo-pyrido[2,1-a][2]benzazepine-3(S)-carboxylic acid, benzyl ester.

The compounds of Formula I wherein X is O, S or NH can be prepared by techniques and procedures well known and appreciated by one of ordinary skill in the art. A general synthetic procedure for preparing these compounds is set forth in Scheme C. In Scheme C, all substituents unless otherwise indicated are as previously defined.

Scheme C provides a general synthetic procedure for 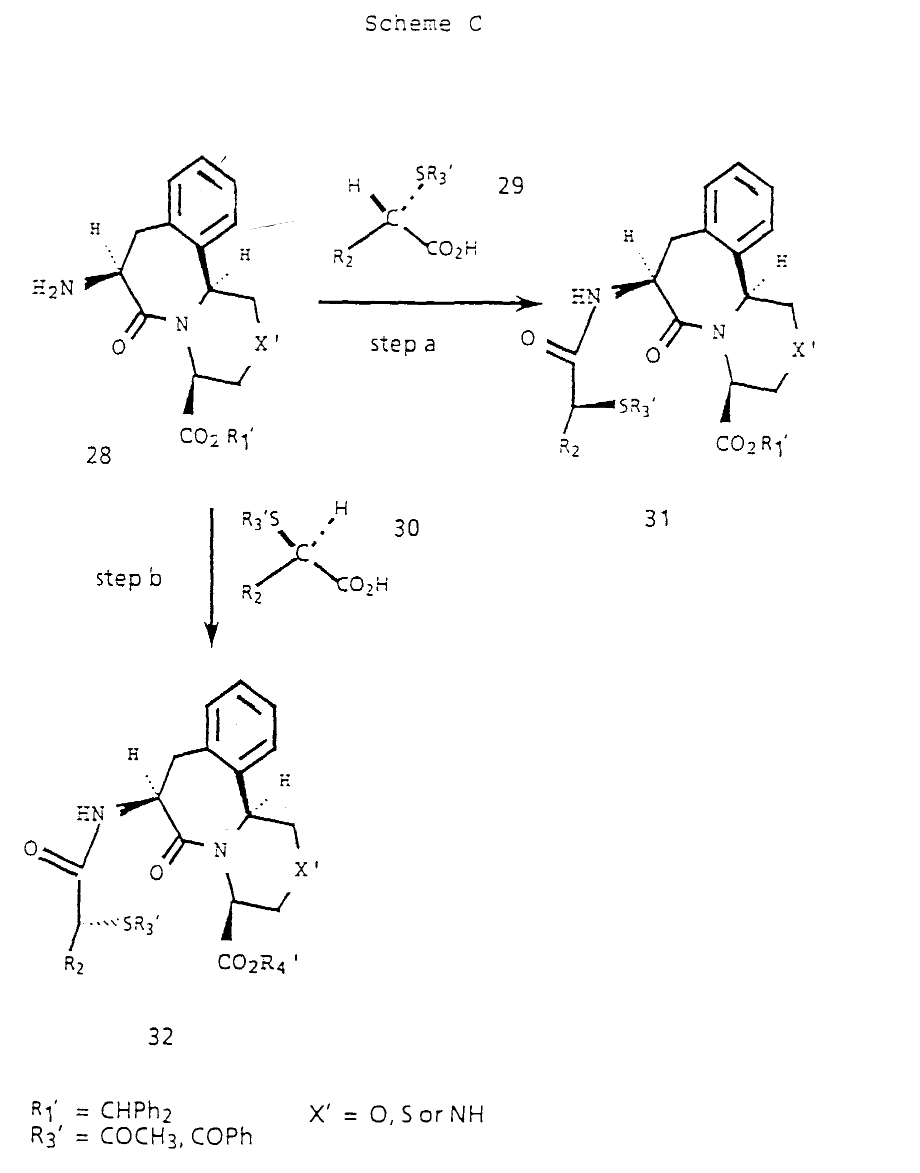 preparing compounds of Formula I wherein X is O, S or NH.

In step a, the appropriate amino compound of structure 28 wherein X is O, S or NH is reacted with the appropriate (S)-thicacetate of structure 29 to give the corresponding (S)-thioacetate compound of structure 31 wherein X is O, S or NH as described previously in Scheme A, step g.

Alternatively, in step b, the appropriate amino compound of structure 28 wherein X is O, S or NH is reacted with the appropriate (R)-thioacetate of structure 30 to give the corresponding (R)-thioacetate compound of structure 32 wherein X is O, S or NH as described previously in Scheme A, step g.

Although Scheme C provides for the preparation of compounds of Formula I wherein X is O, S or NH and the 4-carboxy functionality is of the (S)-configuration, the compounds of Formula I wherein X is O, S or NH and the 4-carboxy functionality is of the (R)-configuration may be prepared by substituting the appropriate (4R)-carboxy compound of structure 28 whose preparation is described in Scheme F, infra.

The groups R₃ and R₄ may be manipulated by techniques and procedures well known and appreciated in the art and described previously in Scheme A and shown in Table 1.

Starting materials for use in Scheme C are readily available to one of ordinary skill in the art. For example, amino compounds of structure 28 wherein X is S are described in European Patent 0 249 223 (December 16, 1987).

The compounds of Formula I wherein X is -NR₆ or -NCOR₇ can be prepared by techniques and procedures well known and appreciated by one of ordinary skill in the art. A general synthetic procedure for preparing these compounds is set forth in Scheme D. In Scheme D, all substituents unless otherwise indicated are as previously defined.

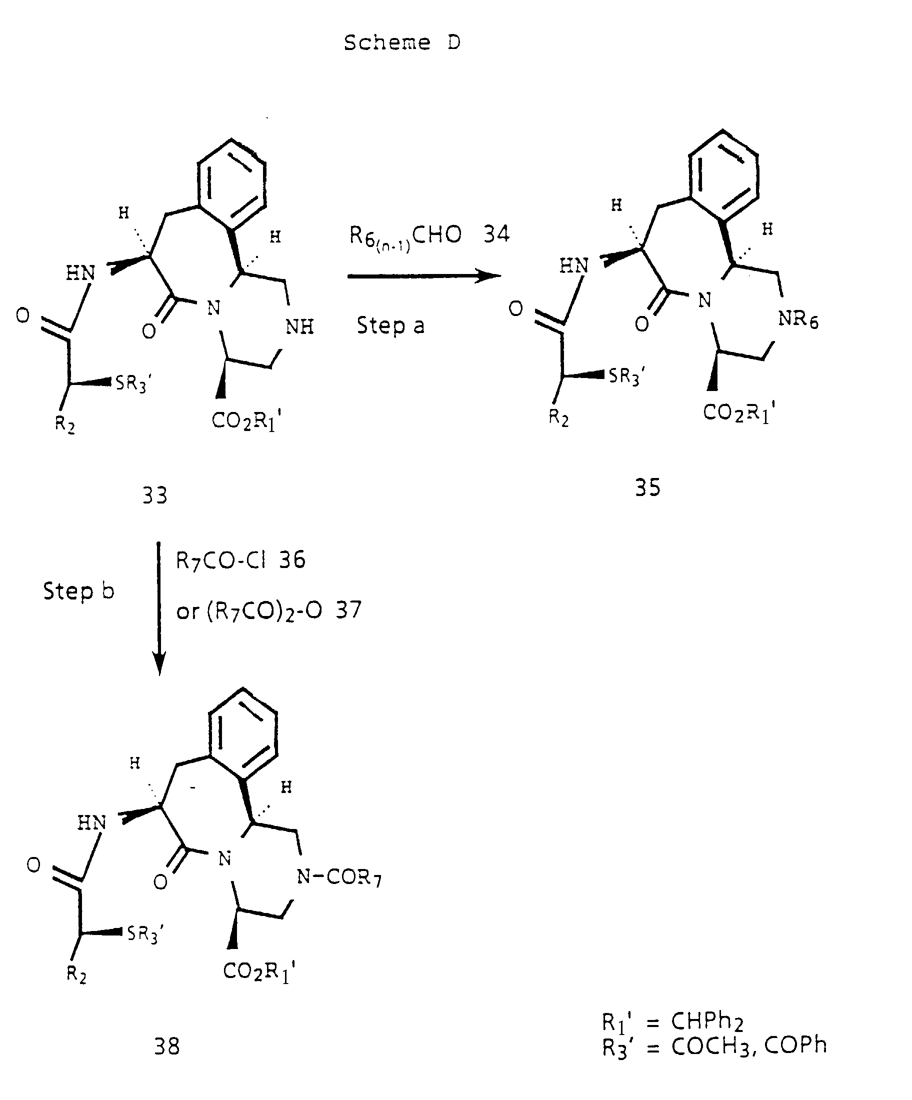

Scheme D provides a general synthetic procedure for preparing the compounds of Formula I wherein X is -NR₆ or -NCOR₇.

In step a, the amino functionality of the appropriate (S)-thioacetate compound of structure 33 is subjected to reductive alkylation with the appropriate aldehyde of structure 34 using sodium cyanoborohydride, as is well known in the art, to give the corresponding N-alkyl-(S)-thioacetate compound of structure 35.

In step b, the amino functionality of the appropriate (S)-thioacetate compound of structure 33 is acylated using the appropriate acyl chloride of structure 36 or the appropriate anhydride of structure 37, as is well known in the art, to give the corresponding N-acyl-(S)-thioacetate compound of structure 38.

The corresponding N-alkyl-(R)-thioacetate compounds and N-acyl-(R)-thioacetate compounds may be prepared in a similar fashion by substituting the appropriate (R)-thioacetate compound for the appropriate (S)-thioacetate compound of structure 33 in Scheme E and the compounds of Formula I wherein X is -NR₆ or -NCOR₇ and the 4-carboxy functionality is of the (R)-configuration may be prepared as described previously in Scheme D.

The groups R₃ and R₁ may be manipulated by techniques and procedures well known and appreciated in the art and described previously in Scheme A and shown in Table 1.

Amino compounds of structure 28 wherein X is O may be prepared as described in Scheme E. In Scheme E, all substituents unless otherwise indicated are as previously defined.

Scheme E provides a general synthetic procedure for preparing amino compounds of structure 28 wherein X is O.

In step a, the appropriate phthalimide blocked (S)-phenylalanine derivative of structure 2 is converted to the corresponding acid chloride, then reacted with the appropriate L-serine methyl ester of structure 39 to give the corresponding 1-oxo-3-phenylpropyl-L-serine methyl ester of structure 40 as described previously in Scheme A, step b.

In step b, the hydroxy functionality of the appropriate 1-oxo-3-phenylpropyl-L-serine methyl ester of structure 40 is allylated with the allyl imidate of structure 41 to give the corresponding 1-oxo-3-phenylpropyl-L-serine-O-allyl methyl ester of structure 42.

For example, appropriate 1-oxo-3-phenylpropyl-L-serine methyl ester of structure 40 is contacted with 2 molar equivalents of the allyl imidate of structure 41 and a molar equivalent of a suitable acid such as trifluoromethanesulfonic acid. The reactants are typically contacted in a suitable organic solvent mixture such as methylene chloride/cyclohexane. The reactants are typically stirred together at room temperature under an inert atmosphere for a period of time ranging from 2-24 hours. The 1-oxo-3-phenylpropyl-L-serine-O-allyl methyl ester of structure 42 is recovered from the reaction zone by extractive methods as is known in the art. It may be purified by silica gel chromatography or crystallization.

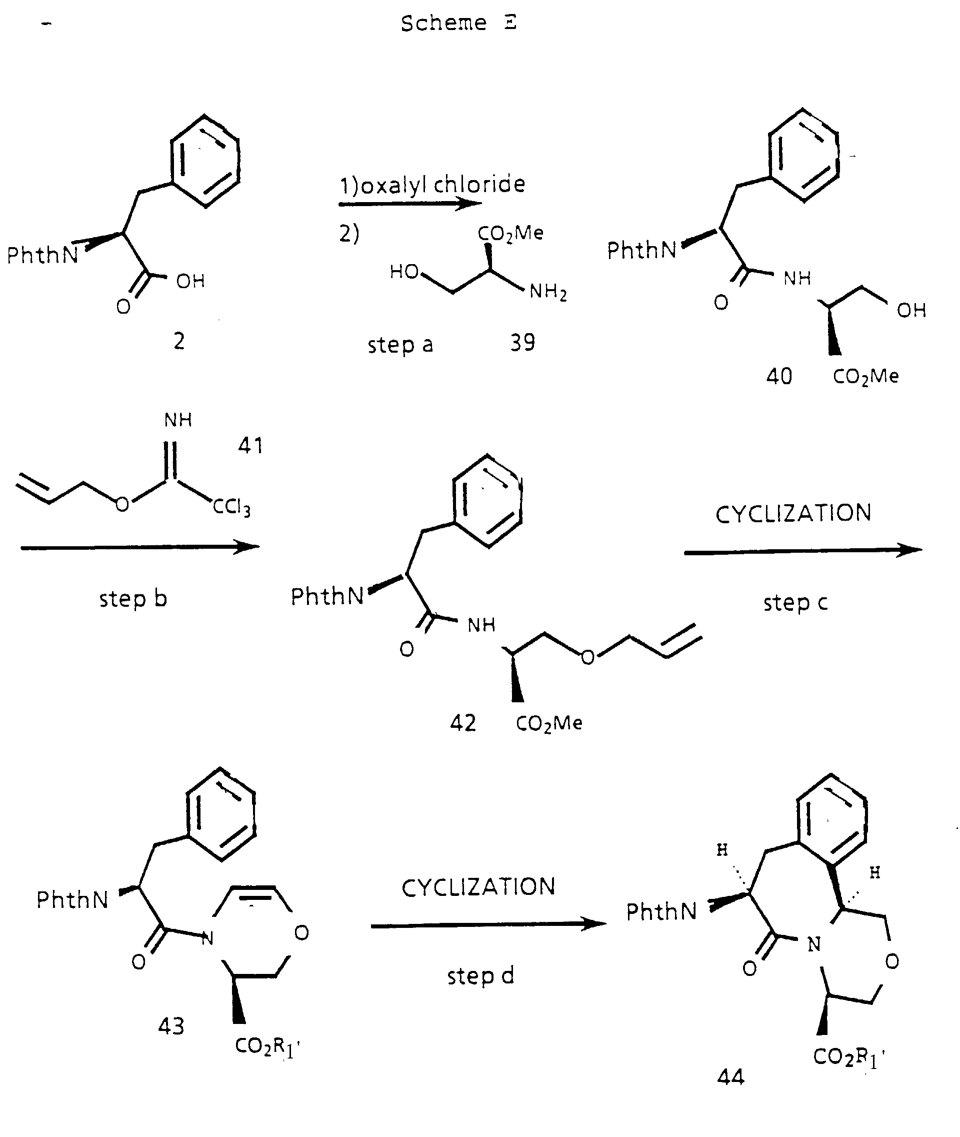

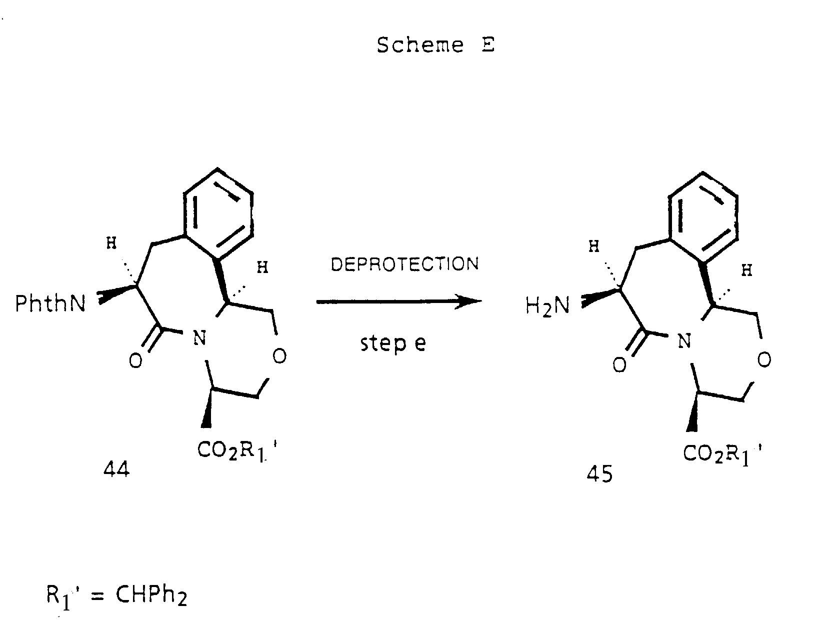

In step c, the appropriate 1-oxo-3-phenylpropyl-L-serine-O-allyl methyl ester of structure 42 is cyclized to give the corresponding (4S)-enamine of structure 43.

For example, the appropriate 1-oxo-3-phenylpropyl-L-serine-O-allyl methyl ester of structure 42 is first contacted with a molar excess of a mixture of ozone/oxygen. The reactants are typically contacted in a suitable organic solvent mixture such as methylene chloride/methanol. The reactants are typically stirred together for a period of time ranging from 5 minutes to 30 minutes or until a blue color persists and at a temperature range of from -78°C to-40°C. The reaction is quenched with an excess of methylsulfide and the intermediate aldehyde compound recovered from the reaction zone by extractive methods as is known in the art.

The intermediate aldehyde compound is then contacted with trifluoroacetic acid to give the corresponding (4S)-enamine of structure 43 as described previously in Scheme A, step d.

In step d, the appropriate (4S)-enamine of structure 43 is cyclized to give the corresponding (4S)-tricyclic compound of structure 44 as described previously in Scheme A, step e. Varying amounts of the (4R)-enamine are also obtained and may be separated using HPLC as described previously in Scheme C, step d.

In step e, the phthalimide protecting group of the appropriate (45)-tricyclic compound of structure 44 is removed to give the corresponding (4S)-amino compound of structure 45 wherein X is O as described in Scheme A, step f.

Alternatively, the appropriate (4R)-amino compound of structure 45 may be prepared by substituting D-serine methyl ester for L-serine methyl ester of structure 39 in step a to give the corresponding 1-oxo-3-phenylpropyl-D-serine methyl ester as described previously in Scheme A, step b. The appropriate 1-oxo-3-phenylpropyl-D-serine methyl ester is then subjected to steps b-f as described previously to give the corresponding (4R)-amino compound of structure 28 wherein X is O.

Amino compounds of structure 28 wherein X is NH may be prepared as described in Scheme F. In Scheme F, all substituents unless otherwise indicated are as previously defined.

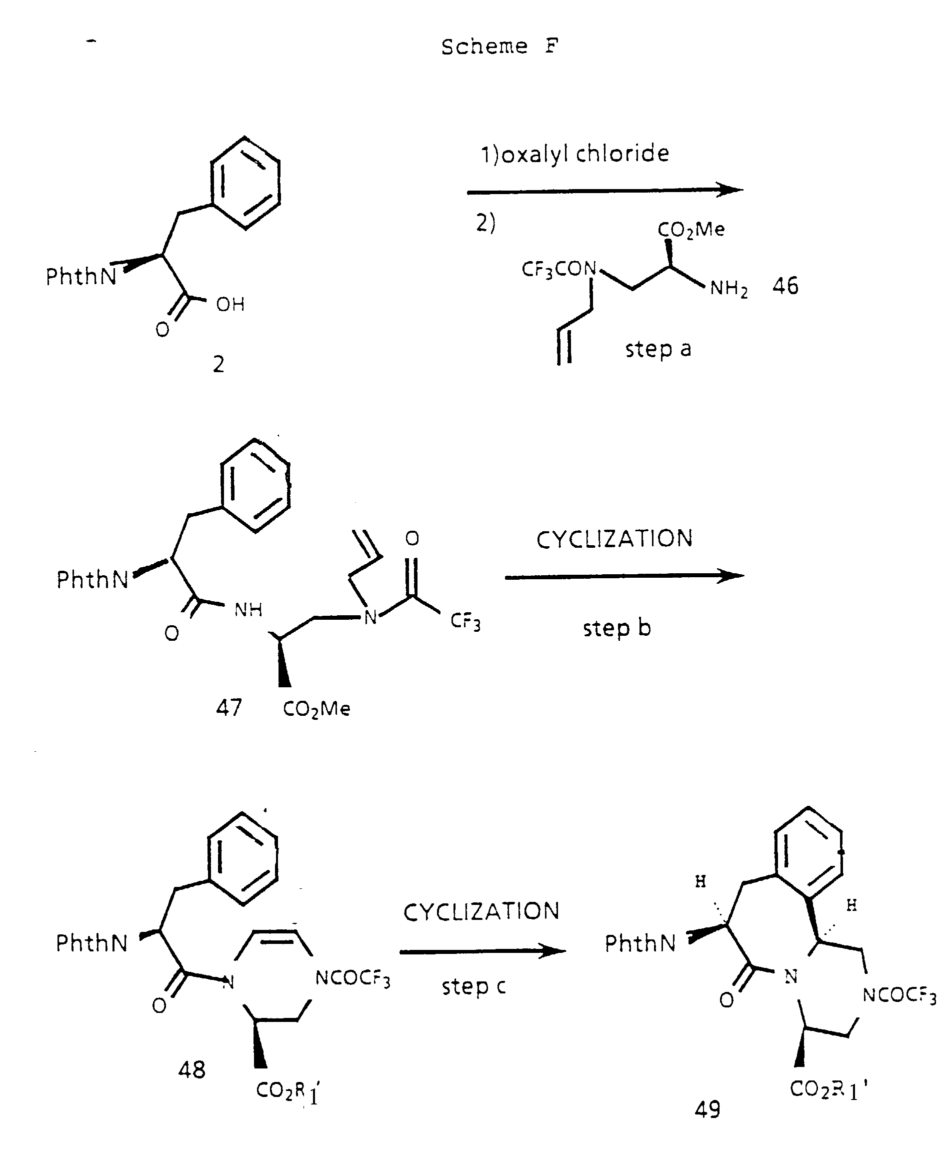

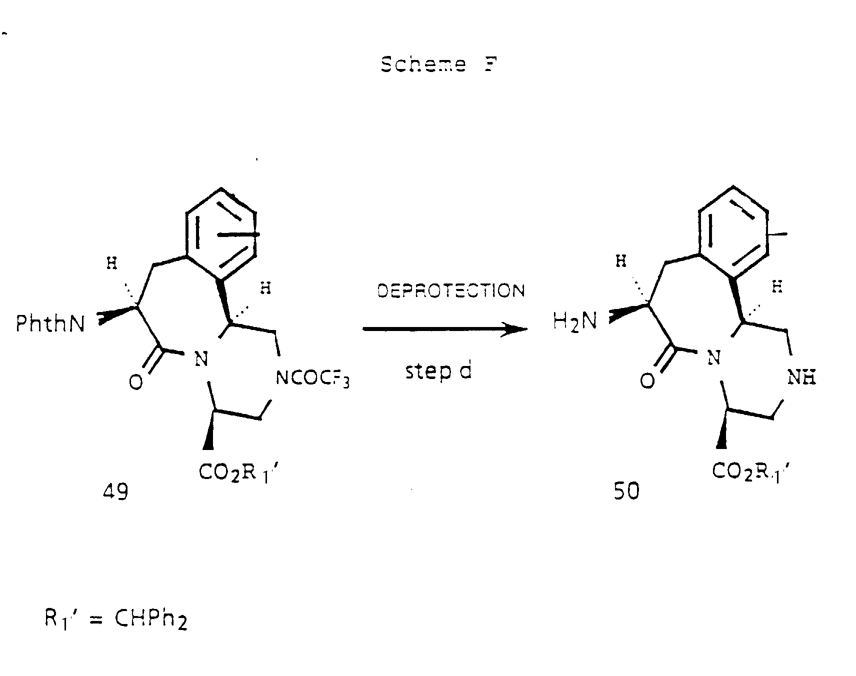

Scheme F provides an alternative general synthetic procedure for preparing amino compounds of structure 28 wherein X is NH.

In step a, the appropriate phthalimide blocked (S)-phenylalanine derivative of structure 2 is converted to the corresponding acid chloride, then reacted with the appropriate 3-trifluoroacetylamino-3-allyl-L-2-aminopropionic acid, methyl ester of structure 46 to give the corresponding 1-oxo-3-phenylpropyl-N-trifluoroacetyl-N-allyl-L-amino acid, methyl ester of structure 47 as described previously in Scheme A, step b.

In step b, the appropriate 1-oxo-3-phenylpropyl-N-trifluoroacetyl-N-allyl-L-amino acid methyl ester of structure 47 is cyclized to give the corresponding enamine of structure 48 as described previously in Scheme F, step c.

In step c, the appropriate (4S)-enamine of structure 48 is cyclized to give the corresponding (4S)-tricyclic compound of structure 49 as described previously in Scheme A, step e.

In step d, the phthalimide protecting group of the appropriate (4S)-tricyclic compound of structure 49 is removed to give the corresponding (4S)-amino compound of structure 50 wherein X is NH as described in Scheme A, step f.

Alternatively, the appropriate (4R)-amino compound of structure 50 may be prepared by substituting 3-trifluoroacetylamino-3-allyl-D-2-aminopropionic acid methyl ester for 3-trrifluoroacetylamino-3-allyl-L-2-aminopropionic acid methyl ester of structure 46 in step a to give the corresponding 1-oxo-3-phenylpropyl-N-trifluoroacetyl-N-allyl-D-amino acid methyl ester as described previously in Scheme A, step b. The appropriate 1-oxo-3-phenylpropyl-N-trifluoroacetyl-N-allyl-D-amino acid methyl ester is then subjected to steps b-f as described previously to give the corresponding-(R)-amino compound of structure 28 wherein X is NH.

The compounds of Formula I wherein n=0 can be prepared by utilizing procedures and techniques well known and appreciated by one of ordinary skill in the art. A general synthetic scheme for preparing these compounds is set forth in Scheme G wherein all substituents, unless otherwise indicated, are previously defined.

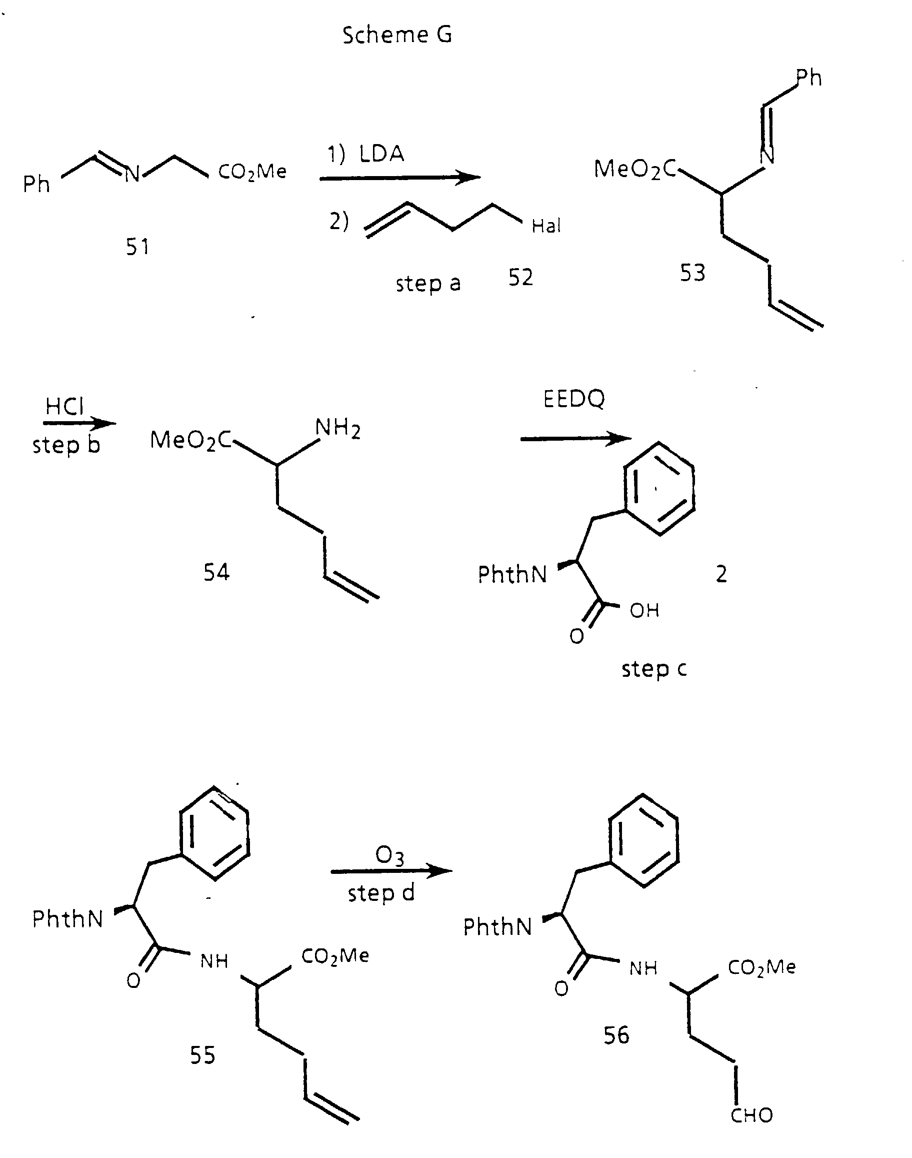

Scheme G provides a general synthetic procedure for preparing compounds of Formula I wherein n=0.

In step a, the N-(phenylmethylene)glycine methyl ester of structure 51 can be treated with one equivalent of a non-nucleophilic base, such as lithium diisopropylamide, in a suitable aprotic solvent, such as tetrahydrofuran, followed by addition of a 4-halobutene of structure 52 to give 2-(3-butenyl)-N-(phenylmethylene)glycine methyl ester of structure 53.

In step b, the N-(phenylmethylene) functionality of 2-(3-butenyl)-N-(phenylmethylene)glycine methyl ester of structure 53 can be hydrolyzed under acidic conditions, such as with hydrochloric acid in a suitable aprotic solvent, such as ethyl ether to give 2-(3-butenyl)-glycine methyl ester of structure 54.

In step c, the appropriate amide compound of structure 55 can be prepared by reacting the appropriate phthalimide protected (S)-phenylalanine compound of structure 2 (described previously in Scheme A) with 2-(3-butenyl)-glycine methyl ester of structure 54 under coupling reaction conditions as described previously in Scheme B, step c and Scheme A, step b.

In step d, the olefin functionality of the appropriate amide compound of structure 55 can be converted to the appropriate aldehyde compound of structure 56 as described previously in Scheme B, step d₁.

The compounds of Formula I wherein n=0, R₃ is acetate or benzoate and R₁ is methyl can be prepared from an appropriate aldehyde of structure 56 in a process as outlined previously in Scheme A, steps d-f and Scheme C, steps a or b.

The individual 3(S) and 3(R) esters of the compounds of Formula I wherein n=0, R₃ is acetate or benzoate and R₁ is methyl can be prepared from an appropriate aldehyde of structure 56 in a process as outlined previously in Scheme A, step d, separating the 3(S) and 3(R) esters of the enamine compounds formed from the cyclization reaction described in Scheme A, step d and completing the process as outlined in Scheme A, steps e-f and Scheme C, steps a or b.

The groups R₃ and R₁ may be manipulated by techniques and procedures well known and appreciated in the art and described previously in Scheme A and Table 1.

Starting materials for use in Scheme C through Scheme G are readily available to one of ordinary skill in the art. For example, (R)- and (S)-3-phenyl-2-acetylthiopropionic acid are described in *J.Org.Chem.,* 51 3664 1986, N^{α}-(benzyloxycarbonyl)-β-(amino)-L-alanine is described in *J. Am. Chem. Soc.*, 107(24) 7105 1985, N-(phenylmethylene)glycine methyl ester is described in *J. Org. Chem.* 41*,* 3491 1976 and allyl trichloroacetimidate is described in *J. Chem. Soc. Perkin Trans.* 1(11) 2247 1985.

The following examples present typical syntheses as described in Scheme C through G. These examples are understood to be illustrative only and are not intended to limit the scope of the present invention in any way.

### Example 51

### Preparation of [4S-[4α, 7α(R*), 12bβ]]-7-[(1-Oxo-2(S)-acetylthio-3-phenylpropyl)amino]-1,2,3,4,6,7,8,12b -hexahydro-6-oxo-1H-[1,4]-oxazino[3,4-a][2]benzazepine-4 -carboxylic acid, diphenylmethyl ester

### Scheme E, step a: N-[2-(1,3-dihydro-1,3-dioxo-2H-isoindol-2-yl)-1-oxo-3-phenylpropyl]-L-serine, methyl ester

Slurry N-phthaloyl-(S)-phenylalanine (90g, 0.3mol) in methylene chloride (450mL) and add, by dropwise addition, oxalyl chloride (54mL, 0.62mol). Place under a dry atmosphere (CaSO₄ tube) and treat with dimethylformamide (10µL). Stir for 5 hours, filter and concentrate in vacuo to give N-phthaloyl-(S)-phenylalanine, acid chloride as off white amorphous solid.

Dissolve serine methyl ester hydrochloride (56g, 0.36mol) in tetrahydrofuran (300mL) then cool to 0°C and add 4-methylmorpholine (88mL, 0.8mol). Add, by dropwise addition, a solution of the N-phthaloyl-(S)-phenylalanine, acid chloride in tetrahydrofuran (200mL). Allow to warm to room temperature and stir for 3 hours. Filter and concentrate the filtrate in vacuo. Dissolve the residue in ethyl acetate and separate the organic phase. Wash with water then saturated sodium chloride and dry (MgSO₄). Evaporate the solvent in vacuo to give an oil. Purify by silica gel chromatography (gradient 50% ethyl acetate/hexane to ethyl acetate) to give the title compound (80.8g, 67%) mp 129-132°C.

### Scheme E, step b: N-[2-(1,3-dihydro-1,3-dioxo-2H-isoindol-2-yl)-1-oxo-3-phenylpropyl]-O-2-propenyl-L-serine, methyl ester

Dissolve N-[2-(1,3-dihydro-1,3-dioxo-2H-isoindol-2-yl)-1-oxo-3-phenylpropyl]-L-serine, methyl ester (25g, 63mmol) in methylene chloride/cyclohexane (1:1, 600mL). Add allyl trichloroacetimidate (26g, 128mmol) and trifluoromethanesulfonic acid (5mL, 56.6mmol). Stir at room temperature under a nitrogen atmosphere for 5 hours and dilute with methylene chloride. Wash with saturated aqueous sodium hydrogen carbonate, water, dry (MgSO₄) and evaporate the solvent in vacuo. Purify by silica gel chromatography (gradient 20% ethyl acetate/hexane to 35% ethyl acetate/hexane) to give the title compound; mp 95-97°C.

### Scheme E, step c: [S-(R*, R*)]-N-[2-(1,3-dihydro-1,3-dioxo-2H-isoindol-2-yl)-1-oxo-3-phenylpropyl]-3,4dihydro-2H-1,4 -oxazine-3-carboxylic acid, methyl ester

Dissolve N-[2-(1,3-dihydro-1,3-dioxo-2H-isoindol-2-yl)-1-oxo-3-phenylpropyl]-O-2-propenyl-L-serine, methyl ester (13g, 29.8mmol) in methylene chloride/methanol (10:1, 220mL). Cool to -78°C and sparge with a mixture of ozone/oxygen for approximately 10 minutes until a blue color persists. Sparge with nitrogen for 10 minutes at -78°C to remove excess ozone. Treat with methylsulfide (60mL, 0.82mol) and allow to warm to room temperature. Stir at room temperature for 2.5 hours, evaporate the solvent in vacuo and dissolve the residue in ethyl acetate (200mL). Wash with water, saturated sodium chloride, dry (MgSO₄) and evaporate the solvent in vacuo to give the intermediate N-[2-(1,3-dihydro-1,3-dioxo-2H-isoindol-2-yl)-1-oxo-3-phenylpropyl]-O-2-oxoethyl-L-serine, methyl ester as a foam (13.6g).

Dissolve N-[2-(1,3-dihydro-1,3-dioxo-2H-isoindol-2-yl)-1-oxo-3-phenylpropyl]-O-2-oxoethyl-L-serine, methyl ester (13.6g) in methylene chloride/trifluoroacetic acid (10:1/330mL). Stir at room temperature for 2.5 hours and evaporate the solvent in vacuo. Purify by silica gel chromatography (35% ethyl acetate/hexane) and recrystallize (ethyl acetate/hexane) to give the title compound (8.52g, 68%); mp 70-72°C.

### Scheme E, step d: [4S-[4α, 7α(R*), 12bβ]]-7-[(1,3-dihydro-1,3-dioxo-2H-isoindol-2-yl)]-3,4,6,7,8,12b-hexahydro-6-oxo -1H-[1,4]-oxazino[3,4-a][2]benzazepine-4-carboxylic acid, diphenylmethyl ester

Dissolve [S-(R*, R*)]-N-[2-(1,3-dihydro-1,3-dioxo-2H-isoindol-2-yl)-1-oxo-3-phenylpropyl]-3,4-dihydro-2H-1,4-oxazine-3-carboxylic acid, methyl ester (2.5g, 5.9mmol) in methylene chloride (5mL) and add, by dropwise addition, to a previously prepared solution of trifluoromethanesulfonic acid (4.0mL, 45mmol) and trifluoroacetic anhydride (1.0mL, 7.lmmol). Place under a nitrogen atmosphere and stir at room temperature for 123 hours. Pour into a separatory funnel containing ice (200g) and ethyl acetate (200mL). Separate the organic phase, wash with water (3X200mL) and saturated aqueous sodium chloride (100mL). Extract the organic phase with 10% wt. potassium hydrogen carbonate (4X40mL) and water (40mL). Layer the combined basic aqueous phases with ethyl acetate (100mL) and cool in an ice bath. Add, by dropwise addition, 6N hydrochloric acid to adjust the pH to 1 while maintaining the temperature at 5-10°C. Separate the organic phase and extract the aqueous phase with ethyl acetate (3X200mL), wash with saturated sodium chloride and dry (MgSO₄). Evaporate the solvent in vacuo and dry the residue under high vacuum at 56°C for 24 hours to give the intermediate [4S-[4α, 7α(R*), 12bβ]]-7-[(1,3-dihydro-1,3-dioxo-2H-isoindol-2-yl)]-3,4,6,7,8,12b-hexahydro-6-oxo-1H-[1,4]-oxazino[3,4-a][2]benzazepine-4-carboxylic acid (1.75g, 73%).

Dissolve [4S-[4α, 7α(R*), 12bβ]]-7-[(1,3-dihydro-1,3-dioxo-2H-isoindol-2-yl)]-3,4,6,7,8,12b-hexahydro-6-oxo-1H-[1,4]-oxazino[3,4-a][2]benzazepine-4-carboxylic acid (500mg, 1.23mmol) in methylene chloride (12mL) and treat with diphenyldiazomethane (360mg, 1.86mmol). Stir for 5.5 hours and evaporate the solvent in vacuo. Purify by silica gel chromatography (gradient 20% ethyl acetate/hexane to 35% ethyl acetate/hexane) to give the title compound (563mg, 80%); mp 178-181°C (isopropanol).

### Scheme E, step e: [4S-[4α, 7α(R*), 12bβ]]-7-(amino)-3,4,6,7,8,12b-hexahydro-6-oxo-1H-[1,4]-oxazino[3,4 -a][2]benzazepine-4-carboxylic acid, diphenylmethyl ester

Dissolve [4S-[4α, 7α(R*), 12bβ]]-7-[(1,3-dihydro-1,3-dioxo-2H-isoindol-2-yl)]-3,4,6,7,8,12b-hexahydro-6-oxo-1H-[1,4]-oxazino[3,4-a][2]benzazepine-4-carboxylic acid, diphenylmethyl ester (296mg, 0.517mmol) in methanol (5mL) and treat with hydrazine monohydrate (1.1mL of a 1M solution in methanol, 1.1mmol). Stir at room temperature for 44 hours, evaporate the solvent in vacuo and slurry the residue in methylene chloride (10mL). Filter and evaporate the solvent in vacuo to give the title compound (218mg, 95%).

### Scheme C, step a: [4S-[4α, 7α(R*), 12bβ]]-7-[(1-Oxo-2(S)-acetylthio-3-phenylpropyl)amino]-1,2,3,4,6,7,8,12b -hexahydro-6-oxo-1H-[1,4]-oxazino[3,4-a][2]benzazepine-4 -carboxylic acid, diphenylmethyl ester

Dissolve [4S-[4α, 7α(R*), 12bβ]]-7-(amino)-3,4,6,7,8,12b-hexahydro-6-oxo-1H-[1,4]-oxazino[3,4-a][2]benzazepine-4-carboxylic acid, diphenylmethyl ester (450mg, 1.018 mmol) and (S)-3-phenyl-2-acetylthiopropionic acid (250mg, 1.12mmol) in methylene chloride (10mL). Add EEDQ (280mg, 1.13mmol) and stir at room temperature for 16 hours. Evaporate the solvent in vacuo and purify by silica gel chromatography (gradient 20% ethyl acetate/hexane to 35% ethyl acetate/hexane) to give the title compound (505mg, 77%).

¹H NMR (CDCl₃) δ 7.44-6.89 (m, 18H), 6.66-6.63 (m, 2H), 6.31 (s, 1H), 5.64-5.53 (m, 1H), 5.10 (d, 1H), 4.94 (d, 1H), 4.71 (t,2H), 4.37 (t, 1H), 3.86 (dd, 1H), 3.77 (dd, 1H), 3.51(B part of ABX, 1H), 3.35 (dd, 1H), 3.07 (dd, 1H), 2.51 (A part of ABX, 1H), 2.40 (s, 3H).

### Example 52 MDL-102,179

### Preparation of [4S-[4α, 7α(R*), 12bβ]]-7-[(1-Oxo-2(S)-acetylthio-3-phenylpropyl)amino]-1,2,3,4,6,7,8,12b -hexahydro-6-oxo-1H-[1,4]-oxazino[3,4-a][2]benzazepine-4 -carboxylic acid

Dissolve [4S-[4α, 7α(R*), 12bβ]]-7-[(1-oxo-2(S)-acetylthio-3-phenylpropyl)amino]-1,2,3,4,6,7,8,12b-hexabydzo-6-oxo-1H-[1,4]-oxazino[3,4-a][2]benzazepine-4-carboxylic acid, diphenylmethyl ester (505mg, 0.78mmol) in methylene chloride (10mL) and treat with anisole (150µL, 1.38mmol) and trifluoroacetic acid (0.8mL, 10.4mmol). Stir for 3.25 hours at room temperature under a nitrogen atmosphere. Evaporate the solvent in vacuo and purify by silica gel chromatography (gradient 35% ethyl acetate/hexane to 0.5% acetic acid in ethyl acetate) to give the title compound (349mg, 93%).

¹H NMR (CDCl₃) δ 7.62-7.00 (m, 10H), 5.65-5.56 (m, 1H), 5.11 (d, 1H), 4.77 (d, 1H), 4.72 (d, 1H), 4.56 (d, 1H), 4.35 (t, 1H), 3.85 (dd, 1H), 3.74 (dd, 1H), 3.64 (B part of ABX, 1H), 3.34 (dd, 1H), 3.05 (dd, 1H), 2.73 (A part of ABX, 1H), 2.36 (s, 3H).

### Example 53

### Preparation of [4S-[4α, 7α(R*), 12bβ]]-7-[(1-Oxo-2(S)-acethylthio-3-phenylpropyl)amino]-1,2,3,4,6,7,8,12b

### hexahydro-6-oxo-1H-[1,4]-oxazino[3,4-a][2]benzazepine-4-carboxylic acid, pivaloyloxymethyl ester

Dissolve [4S-[4α, 7α(R*), 12bβ]]-7-[(1-oxo-2(S)-acetylthio-3-phenylpropyl)amino]-1,2,3,4,6,7,8,12b-hexahydro-6-oxo-1H-[1,4]-oxazino[3,4-a][2]benzazepine-4-carboxylic acid (134mg, 0.28mmol) in methylene chloride (3mL) and dry over anhydrous MgSO₄ (60mg). Filter and wash with methylene chloride (3X200mL). Evaporate in vacuo to a residue. Dissolve the residue in anhydrous dimethylformamide (2mL) and place under nitrogen atmosphere. Add cesium carbonate (99mg, 0.3mmol) in one portion. Stir for 45 minutes at ambient temperature. Add chloromethyl pivalate (101mg, 0.67mmol). Stir the resulting mixture at ambient temperature for 18 hours. Quench the reaction with ethyl acetate (3mL) and water (0.6mL). Separate the organic phase and wash with water (7X6mL), 1/4 saturated potassium hydrogen carbonate (6mL), water (6mL), and saturated sodium chloride (6mL). Dry (MgSO₄), filter and evaporate in vacuo to yield the title compound.

### Example 54 MDL-101,519

### Preparation of [4S-[4α, 7α(R*), 12bβ]]-7-[(1-Oxo-2(S)-thio-3-phenylpropyl)amino]-1,2,3,4,6,7,8,12b-hexahydro-6-oxo-1H -[1,4]-oxazino[3,4-a][2]benzazepine-4-carboxylic acid

Dissolve [4S-[4α, 7α(R*), 12bβ]]-7-[(1-oxo-2(S)-acetylthio-3-phenylpropyl)amino]-1,2,3,4,6,7,8,12b-hexahydro-6-oxo-1H-[1,4]-oxazino[3,4-a][2]benzazepine-4-carboxylic acid (45mg, 0.093mmol) in tetrahydrofuran/methanol (1:1, 4mL) and sparge with nitrogen for 10 minutes at 0°C. Add, by dropwise addition over 4 hours, a solution of lithium hydroxide (0.249mL of a 1M solution of lithium hydroxide degassed using the freeze-thaw technique). Stir at 0°C for 7 hours, treat with 1N hydrochloric acid (0.5mL) at 0°C and evaporate the solvent in vacuo. Purify by silica gel chromatography (gradient ethyl acetate to 0.2% acetic acid in ethyl acetate) to give the title compound (32.4mg, 79%).

¹H NMR (CDCl₃) δ 7.67-6.84 (m, 10H), 5.66-5.56 (m, 1H), 5.11 (d, 1H), 4.74 (d, 1H), 4.55 (d, 1H), 3.84 (dd, 1H), 3.73 (dd, 1H), 3.68-3.53 (m, 2H), 3.27 (dd, 1H), 3.13 (dd, 1H), 2.76 (A part of ABX, 1H), 2.06 (d, 1H).

### Example 55

### Preparation of [4S-[4α, 7α(R*), 12bβ]]-7-[(1-Oxo-2(S)-thio-3-phenylpropyl)amino]-1,2,3,4,6,7,8,12b-hexahydro-6-oxo-1H -[1,4]-oxazino[3,4-a][2]benzazepine-4-carboxylic acid, pivaloyloxymethyl ether ester

Dissolve [4S-[4α, 7α(R*), 12bβ]]-7-[(1-oxo-2(S)-acetylthio-3-phenylpropyl)amino]-1,2,3,4,6,7,8,12b-hexahydro-6-oxo-1H-[1,4]-oxazino[3,4-a][2]benzazepine-4-carboxylic acid, pivaloyl methyl ether ester (55mg, 0.093mmol) in tetrahydrofuran/methanol (1:1, 4mL) and sparge with nitrogen for 10 minutes at 0°C. Add, by dropwise addition over 4 hours, a solution of lithium hydroxide (0.249mL of a 1M solution of lithium hydroxide degassed using the freeze-thaw technique). Stir at 0°C for 7 hours, treat with 1N hydrochloric acid (0.5mL) at 0°C and evaporate the solvent in vacuo. Purify by silica gel chromatography (gradient ethyl acetate to 0.2% acetic acid in ethyl acetate) to give the title compound.

### Example 56

### Preparation of [4S-[4α, 7α(R*), 12bβ]]-7-[(1-Oxo-2(S)-acethylthio-3-phenylpropyl)amino]-1,2,3,4,6,7,8,12b -hexahydro-6-oxo-1H-[1,4]-thiazino[3,4-a][2]benzazepine-4 -carboxylic acid, diphenylmethyl ester

Dissolve [4S-[4α, 7α(R*), 12bβ]]-7-(amino)-3,4,6,7,8,12b-hexahydro-6-oxo-1H-[1,4]-thiazino[3,4-a][2]benzazepine-4-carboxylic acid, diphenylmethyl ester (466mg, 1.018 mmol) and (S)-3-phenyl-2-acetylthiopropionic acid (250mg, 1.12mmol) in methylene chloride (10mL). Add EEDQ (280mg, 1.13mmol) and stir at room temperature for 16 hours. Evaporate the solvent in vacuo and purify by silica gel chromatography (gradient 20% ethyl acetate/hexane to 35% ethyl acetate/hexane) to give the title compound.

### Example 57

### Preparation of [4S-[4α, 7α(R*), 12bβ]]-7-[(1-Oxo-2(S)-acetylthio-3-phenylpropyl)amino]-1,2,3,4,6,7,8,12b -hexahydro-6-oxo-1H-[1,4]-thiazino[3,4-a][2]benzazepine-4 -carboxylic acid

Dissolve [4S-[4α, 7α(R*), 12bβ]]-7-[(1-oxo-2(S)-acetylthio-3-phenylpropyl)amino]-1,2,3,4,6,7,8,12b-hexahydro-6-oxo-1H-[1,4]-thiazino[3,4-a][2]benzazepine-4-carboxylic acid, diphenylmethyl ester (515mg, 0.78mmol) in methylene chloride (10mL) and treat with anisole (150µL, 1.38mmol) and trifluoroacetic acid (0.8mL, 10.4mmol). Stir for 3.25 hours at room temperature under a nitrogen atmosphere. Evaporate the solvent in vacuo and purify by silica gel chromatography (gradient 35% ethyl acetate/hexane to 0.5% acetic acid in ethyl acetate) to give the title compound.

### Example 58

### Preparation of [4S-[4α, 7α(R*), 12bβ]]-7-[(1-Oxo-2(S)-thio-3-phenylpropyl)amino]-1,2,3,4,6,7,8,12b-hexahydro-6-oxo-1H -[1,4]-thiazino[3,4-a][2]benzazepine-4-carboxylic acid

Dissolve [4S-[4α, 7α(R*), 12bβ]]-7-[(1-oxo-2(S)-acetylthio-3-phenylpropyl)amino]-1,2,3,4,6,7,8,12b-hexahydro-6-oxo-1H-[1,4]-thiazino[3,4-a][2]benzazepine-4-carboxylic acid (459mg, 0.093mmol) in tetrahydrofuran/methanol (1:1, 4mL) and sparge with nitrogen for 10 minutes at 0°C. Add, by dropwise addition over 4 hours, a solution of lithium hydroxide (0.249mL of a 1M solution of lithium hydroxide degassed using the freeze-thaw technique). Stir at 0°C for 7 hours, treat with 1N hydrochloric acid (0.5mL) at 0°C and evaporate the solvent in vacuo. Purify by silica gel chromatography (gradient ethyl acetate to 0.2% acetic acid in ethyl acetate) to give the title compound.

### Example 59

### Preparation of [4S-[4α, 7α(R*), 12bβ]]-7-[(1-Oxo-2(S)-acetylthio-3-phenylpropyl)amino]-1,2,3,4,6,7,8,12b -hexahydro-6-oxo-1H-[1,4]-azazino[3,4-a][2]benzazepine-4 -carboxylic acid, diphenylmethyl ester

### Scheme F, step a: N-[2-(1,3-dihydro-1,3-dioxo-2H-isoindol-2-yl)-1-oxo-3-phenylpropyl]-(S)-3-[(trifluoroacetyl-2 -propenyl)amino]-2-amino-propionic acid, methyl ester

Dissolve N^{α}-(benzyloxycarbonyl)-β-(amino)-L-alanine (47.6g, 0.2mol) in methanol (500mL) and treat with concentrated sulfuric acid (0.5mL). Heat to 60°C for 16 hours, cool and reduce the solvent by 50% in vacuo. Dilute with ethyl ether (500mL), wash with saturated sodium hydrogen carbonate, then brine. Dry (MgSO₄) and evaporate the solvent in vacuo to give N^{α}-(benzyloxycarbonyl)-β-(amino)-L-alanine, methyl ester.

Dissolve N^{α}-(benzyloxycarbonyl)-β-(amino)-L-alanine, methyl ester (15.9g, 63mmol) in methylene chloride/cyclohexane (1:1, 600mL). Add allyl trichloroacetimidate (26g, 128mmol) and trifluoromethanesulfonic acid (5mL, 56.6mmol). Stir at room temperature under a nitrogen atmosphere for 5 hours and dilute with methylene chloride. Wash with saturated aqueous sodium hydrogen carbonate, water, dry (MgSO₄) and evaporate the solvent in vacuo. Purify by silica gel chromatography to give N^{α}-(benzyloxycarbonyl)-β-(allylamino)-L-alanine, methyl ester.

Dissolve N^{α}-(benzyloxycarbonyl)-β-(allylamino)-L-alanine, methyl ester (663mg, 2.27mmol) in anhydrous tetrahydrofuran (15mL). Treat with pyridine (183µL, 2.27mmol) followed by trifluoroacetic anhydride (321µL, 2.27mmol) and stir at room temperature overnight. Partition between ethyl ether and water. Separate the organic phase, dry (MgSO₄) and evaporate the solvent in vacuo. Purify by silica gel chromatography to give N^{α}-(benzyloxycarbonyl)-β-(trifluoroacetyl-allylamino)-L-alanine, methyl ester.

Place boron tribromide (215mg, 0.86mmol) in a flask and cool to 0°C. Cautiously add trifluoroacetic acid (5mL) with stirring. Evaporate the solvent to give boron tris(trifluoroacetate).

Dissolve boron tris(trifluoroacetate) (0.3g, 0.86mmol) in trifluoroacetic acid (10mL) and add N^{α}-(benzyloxycarbonyl)-β-(trifluoroacetyl-allylamino)-L-alanine, methyl ester (105mg, 0.27mmol). Stir under an argon atmosphere for 1 hour then evaporate the solvent in vacuo at room temperature. Add methanol and evaporate repeatedly to give β-(trifluoroacetyl-allylamino)-L-alanine, methyl ester, hydrochloride.

Dissolve β-(trifluoroacetyl-allylamino)-L-alanine, methyl ester, hydrochloride (104.8g, 0.36mol) in tetrahydrofuran (300mL) then cool to 0°C and add 4-methylmorpholine (88mL, 0.8mol). Add, by dropwise addition, a solution of the N-phthaloyl-(S)-phenylalanine, acid chloride (108.7g, 0.36mol) in tetrahydrofuran (200mL). Allow to warm to room temperature and stir for 3 hours. Filter and concentrate the filtrate in vacuo. Dissolve the residue in ethyl acetate and separate the organic phase. Wash with water then saturated sodium chloride and dry (MgSO₄). Evaporate the solvent in vacuo to give an oil. Purify by silica gel chromatography to give the title compound.

### Scheme F, step b: [S-(R*, R*)]-N-[2-(1,3-dihydro-1,3-dioxo-2H-isoindol-2-yl)-1-oxo-3-phenylpropyl]-3,4-dihydro-2H-4 -trifluoroacetyl-1,4-azazine-3-carboxylic acid, methyl ester

Dissolve N-[2-(1,3-dihydro-1,3-dioxo-2H-isoindol-2-yl)-1-oxo-3-phenylpropyl]-(S)-3-[(trifluoroacetyl-2-propenyl)amino]-2-amino-propionic acid, methyl ester (15.8g, 29.8mmol) in methylene chloride/methanol (10:1, 220mL). Cool to -78°C and sparge with a mixture of ozone/oxygen for approximately 10 minutes until a blue color persists. Sparge with nitrogen for 10 minutes at -78°C to remove excess ozone. Treat with methylsulfide (60mL, 0.82mol) and allow to warm to room temperature. Stir at room temperature for 2.5 hours, evaporate the solvent in vacuo and dissolve the residue in ethyl acetate (200mL). Wash with water, saturated sodium chloride, dry (MgSO₄) and evaporate the solvent in vacuo to give the intermediate N-[2-(1,3-dihydro-1,3-dioxo-2H-isoindol-2-yl)-1-oxo-3-phenylpropyl]-N-2-oxoethyl, methyl ester.

Dissolve N-[2-(1,3-dihydro-1,3-dioxo-2H-isoindol-2-yl)-1-oxo-3-phenylpropyl]-(S)-3-[(trifluoroacetyl-2-oxoethyl)amino]-2-amino-propionic acid, methyl ester (15.9g, 29.8mmol) in methylene chloride/trifluoroacetic acid (10:1/330mL). Stir at room temperature for 2.5 hours and evaporate the solvent in vacuo. Purify by silica gel chromatography to give the title compound.

### Scheme F, step c: [4S-[4α, 7α(R*), 12bβ]]-7-[(1,3-dihydro-1,3-dioxo-2H-isoindol-2-yl)]-3,4,6,7,8,12b-hexahydro-6-oxo-1H-4-trifluoroacetyl-[1,4]-azazino[3,4-a][2]benzazepine-4-carboxylic acid, diphenylmethyl ester

Dissolve [S-(R*, R*)]-N-[2-(1,3-dihydro-1,3-dioxo-2H-isoindol-2-yl)-1-oxo-3-phenylpropyl]-3,4-dihydro-2H-4-trifluoroacetyl-1,4-azazine-3-carboxylic acid, methyl ester (3.04g, 5.9mmol) in methylene chloride (5mL) and add, by dropwise addition, to a previously prepared solution of trifluoromethanesulfonic acid (4.0mL, 45mmol) and trifluoroacetic anhydride (1.0mL, 7.1mmol). Place under a nitrogen atmosphere and stir at room temperature for 123 hours. Pour into a separatory funnel containing ice (200g) and ethyl acetate (200mL). Separate the organic phase, wash with water (3X200mL) and saturated aqueous sodium chloride (100mL). Extract the organic phase with 10% wt. potassium hydrogen carbonate (4X40mL) and water (40mL). Layer the combined basic aqueous phases with ethyl acetate (100mL) and cool in an ice bath. Add, by dropwise addition, 6N hydrochloric acid to adjust the pH to 1 while maintaining the temperature at 5-10°C. Separate the organic phase and extract the aqueous phase with ethyl acetate (3X200mL), wash with saturated sodium chloride and dry (MgSO₄). Evaporate the solvent in vacuo and dry the residue under high vacuum at 56°C for 24 hours to give the intermediate [4S-[4α, 7α(R*), 12bβ]]-7-[(1,3-dihydro-1,3-dioxo-2H-isoindol-2-yl)]-3,4,6,7,8,12b-hexahydro-6-oxo-1H-4-trifluoroacetyl-[1,4]-azazino[3,4-a][2]benzazepine-4-carboxylic acid.

Dissolve [4S-[4α, 7α(R*), 12bβ]]-7-[(1,3-dihydro-1,3-dioxo-2H-isoindol-2-yl)]-3,4,6,7,8,12b-hexahydro-6-oxo-1H-4-trifluoroacetyl-[1,4]-azazino[3,4-a][2]benzazepine-4-carboxylic acid (616mg, 1.23mmol) in methylene chloride (12mL) and treat with diphenyldiazomethane (360mg, 1.86mmol). Stir for 5.5 hours and evaporate the solvent in vacuo. Purify by silica gel chromatography to give the title compound.

### Scheme F, step e: [4S-[4α, 7α(R*), 12bβ]]-7-(amino)-3,4,6,7,8,12b-hexahydro-6-oxo-1H-[1,4]-azazino[3,4 -a][2]benzazepine-4-carboxylic acid, diphenylmethyl ester

Dissolve [4S-[4α, 7α(R*), 12bβ]]-7-[(1,3-dihydro-1,3-dioxo-2H-isoindol-2-yl)]-3,4,6,7,8,12b-hexahydro-6-oxo-1H-4-trifluoroacetyl-[1,4]-azazino[3,4-a][2]benzazepine-4-carboxylic acid, diphenylmethyl ester (345mg, 0.517mmol) in methanol (5mL) and treat with hydrazine monohydrate (1.1mL of a 1M solution in methanol, 1.1mmol). Stir at room temperature for 44 hours, evaporate the solvent in vacuo and slurry the residue in methylene chloride (10mL). Filter and evaporate the solvent in vacuo to give the title compound.

### Scheme C, step a: [4S-[4α, 7α(R*), 12bβ]]-7-[(1-Oxo-2(S)-acetylthio-3-phenylpropyl)amino]-1,2,3,4,6,7,8,12b -hexahydro-6-oxo-1H-[1,4]-azazino[3,4-a][2]benzazepine-4 -carboxylic acid, diphenylmethyl ester

Dissolve [4S-[4α, 7α(R*), 12bβ]]-7-(amino)-3,4,6,7,8,12b-hexahydro-6-oxo-1H-[1,4]-azazino[3,4-a][2]benzazepine-4-carboxylic acid, diphenylmethyl ester (422mg, 1.018 mmol) and (S)-3-phenyl-2-acetylthiopropionic acid (250mg, 1.12mmol) in methylene chloride (10mL). Add EEDQ (280mg, 1.13mmol) and stir at room temperature for 16 hours. Evaporate the solvent in vacuo and purify by silica gel chromatography to give the title compound.

### Example 60

### Preparation of [4S-[4α, 7α(R*), 12bβ]]-7-[(1-Oxo-2(S)-acetylthio-3-phenylpropyl)amino]-1,2,3,4,6,7,8,12b -hexahydro-6-oxo-1H-[1,4]-azazino[3,4-a][2]benzazepine-4 -carboxylic acid

Dissolve [4S-[4α, 7α(R*), 12bβ]]-7-[(1-oxo-2(S)-acetylthio-3-phenylpropyl)amino]-1,2,3,4,6,7,8,12b-hexahydro-6-oxo-1H-[1,4]-azazino[3,4-a][2]benzazepine-4-carboxylic acid, diphenylmethyl ester (484mg, 0.78mmol) in methylene chloride (10mL) and treat with anisole (150µL, 1.38mmol) and trifluoroacetic acid (0.8mL, 10.4mmol). Stir for 3.25 hours at room temperature under a nitrogen atmosphere. Evaporate the solvent in vacuo and purify by silica gel chromatography to give the title compound.

### Example 61

### Preparation of [4S-[4α, 7α(R*), 12bβ]]-7-[(1-Oxo-2(S)-thio-3-phenylpropyl)amino]-1,2,3,4,6,7,8,12b-hexahydro-6-oxo-1H -[1,4]-azazino[3,4-a][2]benzazepine-4-carboxylic acid

Dissolve [4S-[4α, 7α(R*), 12bβ]]-7-[(1-oxo-2(S)-acetylthio-3-phenylpropyl)amino]-1,2,3,4,6,7,8,12b-hexahydro-6-oxo-1H-[1,4]-azazino[3,4-a][2]benzazepine-4-carboxylic acid (42mg, 0.093mmol) in tetrahydrofuran/methanol (1:1, 4mL) and sparge with nitrogen for 10 minutes at 0°C. Add, by dropwise addition over 4 hours, a solution of lithium hydroxide (0.249mL of a 1M solution of lithium hydroxide degassed using the freeze-thaw technique). Stir at 0°C for 7 hours, treat with 1N hydrochloric acid (0.5mL) at 0°C and evaporate the solvent in vacuo. Purify by silica gel chromatography to give the title compound.

### Example 62

### Preparation of [4S-[4α, 7α(R*), 12bβ]]-7-[(1-Oxo-2(S)-acetylthio-3-phenylpropyl)amino]-1,2,3,4,6,7,8,12b -hexahydro-6-oxo-1H-[1,4]-N⁴-trifluoroacetyl-azazino[3,4 -a][2]benzazepine-4-carboxylic acid, diphenylmethyl ester

Dissolve [4S-[4α, 7α(R*), 12bβ]]-7-[(1-Oxo-2(S)-thio-3-phenylpropyl)amino]-1,2,3,4,6,7,8,12b-hexahydro-6-oxo-1H-[1,4]-azazino[3,4-a][2]benzazepine-4-carboxylic acid (979mg, 2.27mmol) in anhydrous tetrahydrofuran (15mL). Treat with pyridine (183µL, 2.27mmol) followed by trifluoroacetic anhydride (321µL, 2.27mmol) and stir at room temperature overnight. Partition between ethyl ether and water. Separate the organic phase, dry (MgSO₄) and evaporate the solvent in vacuo. Purify by silica gel chromatography to give the title compound.

### Example 63

### [6α(R*), 11bβ]-6-[(S)-(1-Oxo-2(R)-benzoylthio-3-phenylpropyl)amino]-1,2,3,5,6,7,11b-heptahydro-5-oxo-pyrrolo[2,1-a][2]benzazepine-3(S)-carboxylic acid, methyl ester

### Scheme G, step a: N-(Phenylmethylene)-2-(3-butenyl)glycine methyl ester

Dissolve diisopropylamine (15.4mL, 110mmol) in tetrahydrofuran (250mL), place under a nitrogen atmosphere and cool to -78°C. Add n-butyllithium (39mL of a 2.7M solution in hexane, 105mmol). Stir for 30 minutes and add, by dropwise addition, a solution of N-(phenylmethylene)glycine methyl ester (17.7g, 100mmol) in tetrahydrofuran (25mL). Stir for 15 minutes and add 4-bromobutene (13.5g, 100mmol) and allow to warm slowly to room temperature. Add hexamethylphosphoramide (20mL, 100mmol) and stir under a nitrogen atmosphere for 3 hours. Pour into water, extract into ethyl ether and wash with brine several times. Dry (MgSO₄) and evaporate the solvent in vacuo to give the title compound as an amber oil (25g).

### Scheme G, step b: 2-(3-Butenyl)glycine methyl ester

Dissolve N-(phenylmethylene)-2-(3-butenyl)glycine methyl ester (25g) in ethyl ether (400mL) and stir with 1N hydrochloric acid (150mL) and water (150mL). Place under an argon atmosphere and stir for 2 hours. Separate the aqueous phase and adjust to pH 9, extract into chloroform, dry and evaporate the solvent in vacuo to give the title compound as a light oil (4.5g).

### Scheme G, step c: (S)-N-[2-(1,3-Dihydro-1,3-dioxo-2H-isoindol-2-yl)-1-oxo-3-phenylpropyl]-2-(3-butenyl)-glycine, methyl esters

Dissolve N-phthaloyl-(S)-phenylalanine (2) (6.0g, 20mmol) and EEDQ (6.0g, 24mmol) in methylene chloride (30mL). Add 2-(3-butenyl)glycine methyl ester (3.0g, 21mmol) and stir for 18 hours. Pour into methylene chloride, wash with 10% hydrochloric acid then saturated sodium hydrogen carbonate. Dry and evaporate the solvent in vacuo to give 8.3g yellow oil. Purify by silica gel chromatography (25% ethyl acetate/hexane) to give a diastereomeric mixture of the title compounds as foam (5.2g).

### Scheme G, step d: (S)-N-[2-(1,3-Dihydro-1,3-dioxo-2H-isoindol-2-yl)]phenylalanyl]-2-(3-oxopropyl)glycine, methyl esters

Dissolve the diastereomeric mixture of (S)-N-[2-(1,3-dihydro-1,3-dioxo-2H-isoindol-2-yl)-1-oxo-3-phenylpropyl]-2-(3-butenyl)-glycine, methyl esters (4.2g, 10mmol) in methylene chloride (100mL) and absolute methanol (10mL). Cool to -78°C and treat with ozone until blue. Degas with nitrogen and add methyl sulfide (10mL) and pyridine (0.5mL). Allow to warm slowly to room temperature and stir for 18 hours. Wash with 10% hydrochloric acid then brine. Dry and evaporate the solvent in vacuo to give a diastereomeric mixture of the title compounds as an oil (4.5g).

### Scheme A, step d: (S)-N-[2-(1,3-Dihydro-1,3-dioxo-2H-isoindol -2-yl)]-1-oxo-3-phenylpropyl-1,2,3-trihydro-2(S)-pyrrolecarboxylic acid, methyl ester and (S)-N-[2-(1,3-Dihydro-1,3-dioxo-2H-isoindol-2-yl)]-1-oxo-3-phenylpropyl -1,2,3-trihydro-2(R)-pyrrolecarboxylic acid, methyl ester

Dissolve the diastereomeric mixture of (S)-N-[2-(1,3-dihydro-1,3-dioxo-2H-isoindol-2-yl)]-phenylalanyl]-2-(3-oxopropyl)glycine, methyl esters (4.5g) in 1,1,1-trichloroethane (150mL) and treat with trifluoroacetic acid (0.5mL). Heat at reflux for 18 hours, evaporate the solvent and purify by silica gel chromatography (80% ethyl acetate/hexane) to give the 2(S)-title compound (700mg) and the 2(R)-title compound (600mg).

### Scheme A, Step e: [6α(R*), 11bβ]-6-[(S)-(1,3-Dihydro-1,3-dioxo-2H-isoindol-2-yl)]-1,2,3,5,6,7,11b-heptahydro-5-oxo -pyrrolo[2,1-a][2]benzazepine-3(S)-carboxylic acid, methyl ester

Dissolve (S)-N-[2-(1,3-dihydro-1,3-dioxo-2H-isoindol-2-yl)]-1-oxo-3-phenylpropyl-1,2,3-trihydro-2(S)-pyrrolecarboxylic acid, methyl ester (338mg, 0.836mmol) in anhydrous methylene chloride (10mL) and add to trifluoromethanesulfonic acid (5mL). Stir for 3.5 hours, cool in an ice bath and carefully add water (25mL). Extract with ethyl acetate (75mL) and wash with saturated sodium hydrogen carbonate (25mL). Dry (Na₂SO₄) and evaporate the solvent in vacuo. Purify by silica gel chromatography (1:1 ethyl acetate/hexane to 2:1 ethyl acetate/hexane) to give the title compound as a white foam (314mg, 93%).

### Scheme A, Step f: [6α(R*), 11bβ]-6-[(S)-Amino]-1,2,3,5,6,7,11b-heptahydro-5-oxo-pyrrolo[2,1 -a][2]benzazepine-3(S)-carboxylic acid, methyl ester

Dissolve [6α(R*), 11bβ]-6-[(S)-(1,3-dihydro-1,3-dioxo-2H-isoindol-2-yl)]-1,2,3,5,6,7,11b-heptahydro-5-oxo-pyrrolo[2,1-a][2]benzazepine-3(S)-carboxylic acid, methyl ester (244mg, 0.603mmol) in methanol (3mL), treat with hydrazine monohydrate (0.70mL of a 1M solution in methanol) and stir at room temperature for 24 hours. Add additional hydrazine monohydrate (0.3mL of a 1M solution in methanol) and stir for 48 hours. Filter through filter aid, evaporate the solvent in vacuo and add methylene chloride. Filter slowly through filter aid (MgSO₄) and evaporate the solvent in vacuo to give the title compound as a yellow oil (181mg).

### Scheme C, Step b: [6α(R*), 11bβ]-6-[(S)-(1-Oxo-2(R)-benzoylthio-3-phenylpropyl)amino]-1,2,3,5,6,7,11b -heptahydro-5-oxo-pyrrolo[2,1-a][2]benzazepine-3(S) -carboxylic acid, methyl ester

Dissolve (R)-3-phenyl-2-benzoylthiopropionic acid (242mg, 0.845mmol) in methylene chloride (6mL), cool in an ice-methanol bath and treat with oxalyl chloride (0.94mL, 11mmol). Stir for 1.5 hours and evaporate the solvent in vacuo at 0-5°C. Dilute the residue with methylene chloride (3mL) and add a solution of [6α(R*), 11bβ]-6-[(S)-Amino]-1,2,3,5,6,7,11b-heptahydro-5-oxo-pyrrolo[2,1-a][2]benzazepine-3(S)-carboxylic acid, methyl ester (155mg, 0.565mmol) in methylene chloride (6mL). Add pyridine (68µL, 0.85mmol) and stir for 2 hours. Dilute with ethyl acetate (60mL) and wash with 1N hydrochloric acid (30mL) and saturated sodium hydrogen carbonate (2X30mL). Dry (MgSO₄), evaporate the solvent in vacuo and purify by silica gel chromatography (3:2 hexane/ethyl acetate) to give the title compound as a white solid (159g, 53.1%).

### Example 64

### Preparation of [6α[R*), 11bβ]-6-[(S)-(1-Oxo-2(R)-thio-3-phenylpropyl)amino]-1,2,3,5,6,7,11b-heptahydro-5-oxo -pyrrolo[2,1-a][2]benzazepine-3(S)-carboxylic acid

Dissolve [6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-3(S)-carboxylic acid, methyl ester (52mg, 0.098mmol) in methanol (1.5mL) and degas at 0°C. Add aqueous lithium hydroxide (0.6mL of a 1N degassed solution, 0.6mmol) at 0°C. Add tetrahydrofuran to obtain a solution (4mL) and stir for 17 hours at room temperature. Cool in an ice bath and add 1N hydrochloric acid (1mL). Partition between methylene chloride (30mL) and water (15mL) and separate the organic phase. Dry (Na₂SO₄), evaporate the solvent in vacuo and purify by silica gel chromatography (2:1 hexane/ethyl acetate) to give the title compound as a white solid (32.2g, 77%).

The following compounds can be prepared by procedures analagous to those described above in Example 51 -64:
[4S-[4α, 7α(R*), 12bβ]]-7-[(1-Oxo-2(R)-thio-3-phenylpropyl)amino]-1,2,3,4,6,7,8,12b-hexahydro-6-oxo-1H-[1,4]-oxazino[3,4-a][2]benzazepine-4-carboxylic acid, pivaloyloxymethyl ester;
[4S-[4α, 7α(R*), 12bβ]]-7-[(1-Oxo-2(R)-acetylthio-3-phenylpropyl)amino]-1,2,3,4,6,7,8,l2b-hexahydro-6-oxo-1H-[1,4]-oxazino[3,4-a][2]benzazepine-4-carboxylic acid, pivaloyloxymethyl ester;
[4S-[4α, 7α(R*), 12bβ]]-7-[(1-Oxo-2(R)-thio-3-phenylpropyl)amino]-1,2,3,4,6,7,8,12b-hexahydro-6-oxo-1H-[1,4]-thiazino[3,4-a][2]benzazepine-4-carboxylic acid
[4S-[4α, 7α(R*), 12bβ]]-7-[(1-Oxo-2(R)-thio-3-phenylpropyl)amino]-1,2,3,4,6,7,8,12b-hexahydro-6-oxo-1H-[1,4]-thiazino[3,4-a][2]benzazepine-4-carboxylic acid, pivaloyloxymethyl ester;
[4S-[4α, 7α(R*), 12bβ]]-7-[(1-Oxo-2(R)-acetylthio-3-phenylpropyl)amino]-1,2,3,4,6,7,8,12b-hexahydro-6-oxo-1H-[1,4]-thiazino[3,4-a][2]benzazepine-4-carboxylic acid, pivaloyloxymethyl ester;
[4S-[4α, 7α(R*), 12bβ]]-7-[(1-Oxo-2(R)-thio-3-phenylpropyl)amino]-1,2,3,4,6,7,8,12b-hexahydro-6-oxo-1H-[1,4]-azazino[3,4-a][2]benzazepine-4-carboxylic acid, pivaloyloxymethyl ester;
[4S-[4α, 7α(R*), 12bβ]]-7-[(1-Oxo-2(R)-thio-3-phenylpropyl)amino]-1,2,3,4,6,7,8,12b-hexahydro-6-oxo-1H-[1,4]-N⁴-benzyl-azazino[3,4-a][2]benzazepine-4-carboxylic acid, pivaloyloxymethyl ester;
[4S-[4α, 7α(R*), 12bβ]]-7-[(1-Oxo-2(R)-acetylthio-3-phenylpropyl)amino]-1,2,3,4,6,7,8,12b-hexahydro-6-oxo-1H-[1,4]-N⁴-benzoyl-azazino[3,4-a][2]benzazepine-4-carboxylic acid, pivaloyloxymethyl ester;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-3(R)-carboxylic acid, methyl ester;
[6α(R*), 11bβ]-6-[(S)-(1-oxo-2-thio-ethyl)amino]-1,2,3,5,6,7,11b-heptahydro-5-oxo-pyrrolo[2,1-a][2]benzazepine-3(R)-carboxylic acid;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-3(R)-carboxylic acid;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-3(R)-carboxylic acid, benzyl ester;
[6α(R*), 11bβ]-6-[(S)-(1-oxo-2-thio-ethyl)amino]-1,2,3,5,6,7,11b-heptahydro-5-oxo-pyrrolo [2,1-a][2]benzazepine-3(R)-carboxylic acid, benzyl ester;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-3(R)-carboxylic acid, methyl ester;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-3(R)-carboxylic acid;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-3(R)-carboxylic acid, benzyl ester;
[6α(R*), 11bβ]-6-[(S)-(1-oxo-2-thio-ethyl)amino]-1,2,3,5,6,7,11b-heptahydro-5-oxo-pyrrolo[2,1-a][2]benzazepine-3(S)-carboxylic acid;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-3(S)-carboxylic acid;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-3(S)-carboxylic acid, benzyl ester;
[6α(R*), 11bβ]-6-[(S)-(1-oxo-2-thio-ethyl)amino]-1,2,3,5,6,7,11b-heptahydro-5-oxo-pyrrolo[2,1-a][2]benzazepine-3(S)-carboxylic acid, benzyl ester;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-3(S)-carboxylic acid, methyl ester;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-3(S)-carboxylic acid;
[6α(R*), 11bβ]-6-[(S)-(1-oxo-2(S)-benzoylthio-3-phenylpropyl)amino]-1,2,3,5,6,7,11b-heptahydro-5-oxo-pyrrolo[2,1-a][2]benzazepine-3(S)-carboxylic acid, benzyl ester;
[6α(R*), 11bβ]-6-[(S)-(1-oxo-2(R)-benzoylthio-3-phenylpropyl)amino]-1,2,3,5,6,7,11b-heptahydro-5-oxo-pyrrolo[2,1-a][2]benzazepine-3(S)-carboxylic acid, methyl ester;
[6α(R*), 11bβ]-6-[(S)-(1-oxo-2(S)-thio-3-phenylpropyl)amino]-1,2,3,5,6,7,11b-heptahydro-5-oxo-pyrrolo[2,1-a][2]benzazepine-3(S)-carboxylic acid, pivaloyloxymethyl ester;
[6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-3(S)-carboxylic acid, pivaloyloxymethyl ester.

As used herein, the term "patient" refers to warm-blooded animals or mammals, including mice, rats and humans. A patient is in need of treatment to inhibit enkephalinase when the patient is suffering from acute or chronic pain and is in need of an endorphin- or enkephalin-mediated analgesic effect. In addition, a patient is in need of treatment to inhibit enkephalinase when the patient is suffering from a disease state characterized by abnormalities in fluid, electrolyte, blood pressure, intraocular pressure, renin, or aldosterone homeostasis, such as, but not limited to, hypertension, renal diseases, hyperaldosteronemia, cardiac hypertrophy, glaucoma and congestive heart failure. In these instances the patient is in need of an ANP-mediated diuretic, natriuretic, hypotensive, hypoaldosteronemic effect. Inhibition of enkephalinase would provide an endorphin- or enkephalin-mediated analgesic effect by inhibiting the metabolic degradation of endorphins and enkephalins. Inhibition of enkephalinase would provide an ANP-mediated diuretic, natriuretic, hypotensive, hypoaldosteronemic effect by inhibiting the metabolic degradation of ANP.

In addition, a patient is in need of treatment to inhibit enkephalinase when the patient is in need of an antidepressant effect or a reduction in severity of withdrawal symptoms associated with termination of opiate or morphine administration.

The identification of those patients who are in need of treatment to inhibit enkephalinase is well within the ability and knowledge of one skilled in the art. A clinician skilled in the art can readily identify, by the use of clinical tests, physical examination and medical/family history, those patients who are in need of an endorphin- or enkephalin-mediated analgesic effect or who are in need of an ANP-mediated diuretic, natriuretic, hypotensive or hypoaldosteronemic effect.

An effective enkephalinase inhibitory amount of a compound of Formula (I) is an amount which is effective in inhibiting enkephalinase and in thus inhibiting the metabolic degradation of the naturally-occurring circulating regulatory peptides such as the endorphins, including enkephalins, and ANP. Successful treatment is also understood to include prophylaxis in treating a patient in those instances such as, for example, in a pre-operative procedure, where a patient will be suffering from acute or chronic pain in the near future.

An effective enkephalinase inhibitory amount of a compound of Formula (I) is an amount which is effective in inhibiting enkephalinase in a patient in need thereof which results, for example, in endorphin- or enkephalin-mediated analgesic effects or in ANP-mediated diuretic, natriuretic, hypotensive, hypoaldosteronemic effect.

An effective enkephalinase inhibitory dose can be readily determined by the use of conventional techniques and by observing results obtained under analogous circumstances. In determining the effective dose, a number of factors are considered including, but not limited to: the species of patient; its size, age, and general health; the specific disease involved; the degree of or involvement or the severity of the disease; the response of the individual patient; the particular compound administered; the mode of administration; the bioavailability characteristics of the preparation administered; the dose regimen selected; and the use of concomitant medication.

An effective enkephalinase inhibitory amount of a compound of Formula (I) will generally vary from about 0.01 milligram per kilogram of body weight per day (mg/kg/day) to about 20 mg/kg/day. A daily dose of from about 0.1 mg/kg to about 10 mg/kg is preferred.

A patient is in need of treatment to inhibit ACE when the patient is suffering from hypertension, chronic congestive heart failure, hyperaldosteronemia or cognitive disorders. Inhibition of ACE reduces levels of angiotensin II and thus inhibits the vasopressor, hypertensive and hyperaldosteronemic effects caused thereby. An effective ACE inhibitory amount of a compound of Formula (I) is that amount which is effective in inhibiting ACE in a patient in need thereof which results, for example, in a hypotensive effect. An effective ACE inhibitory amount and an effective ACE inhibitory dose are the same as that described above for an effective enkephalinase inhibitory amount and dose.

In effecting treatment of a patient, compounds of Formula (I) can be administered in any form or mode which makes the compound bioavailable in effective amounts, including oral and parenteral routes. For example, the compound can be administered orally, subcutaneously, intramuscularly, intravenously, transdermally, intranasally, rectally, and the like. Oral administration is generally preferred. One skilled in the art of preparing Formulations can readily select the proper form and mode of administration depending upon the disease state to be treated, the stage of the disease, and other relevant circumstances.

Compounds of Formula (I) can be administered in the form of pharmaceutical compositions or medicaments which are made by combining the compounds of Formula (I) with pharmaceutically acceptable carriers or excipients, the proportion and nature of which are determined by the chosen route of administration, and standard pharmaceutical practice.

In another embodiment, the present invention provides compositions comprising a compound of Formula (I) in admixture or otherwise in association with one or more inert carriers. These compositions are useful, for example, as assay standards, as convenient means of making bulk shipments, or as pharmaceutical compositions. An assayable amount of a compound of Formula (I) is an amount which is readily measurable by standard assay procedures and techniques as are well known and appreciated by those skilled in the art. Assayable amounts of a compound of Formula (I) will generally vary from about 0.001% to about 75% of the composition by weight. Inert carriers can be any material which does not degrade or otherwise covalently react with a compound of Formula (I). Examples of suitable inert carriers are water; aqueous buffers, such as those which are generally useful in High Performance Liquid Chromatography (HPLC) analysis; organic solvents, such as acetonitrile, ethyl acetate, hexane and the like; and pharmaceutically acceptable carriers or excipients.

More particularly, the present invention provides pharmaceutical compositions comprising an effective amount of a compound of Formula (I) in admixture or otherwise in association with one or more pharmaceutically acceptable carriers or excipients.

The pharmaceutical compositions or medicaments are prepared in a manner well known in the pharmaceutical art. The carrier or excipient may be a solid, semi-solid, or liquid material which can serve as a vehicle or medium for the active ingredient. Suitable carriers or excipients are well known in the art. The pharmaceutical composition may be adapted for oral or parenteral use and may be administered to the patient in the form of tablets, capsules, suppositories, solutions, suspensions, or the like.

The pharmaceutical compositions may be administered orally, for example, with an inert diluent or with an edible carrier. They may be enclosed in gelatin capsules or compressed into tablets. For the purpose of oral therapeutic administration, the compounds of Formula (I) may be incorporated with excipients and used in the form of tablets, troches, capsules, elixirs, suspensions, syrups, wafers, chewing gums and the like. These preparations should contain at least 4% of the compound of Formula (I), the active ingredient, but may be varied depending upon the particular form and may conveniently be between 4% to about 70% of the weight of the unit. The amount of the active ingredient present in compositions is such that a unit dosage form suitable for administration will be obtained.

The tablets, pills, capsules, troches and the like may also contain one or more of the following adjuvants: binders, such as microcrystalline cellulose, gum tragacanth or gelatin; excipients, such as starch or lactose, disintegrating agents such as alginic acid, Primogel, corn starch and the like; lubricants, such as magnesium stearate or Sterotex; glidants, such as colloidal silicon dioxide; and sweetening agents, such as sucrose or saccharin may be added or flavoring agents, such as peppermint, methyl salicylate or orange flavoring. When the dosage unit form is a capsule, it may contain, in addition to materials of the above type, a liquid carrier such as polyethylene glycol or a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active ingredient, sucrose as a sweetening agent and certain preservatives, dyes and colorings and flavors. Materials used in preparing these various compositions should be pharmaceutically pure and non-toxic in the amounts used.

For the purpose of parenteral administration, the compounds of Formula (I) may be incorporated into a solution or suspension. These preparations should contain at least 0.1% of a compound of the invention, but may be varied to be between 0.1 and about 50% of the weight thereof. The amount of the active ingredient present in such compositions is such that a suitable dosage will be obtained.

The solutions or suspensions may also include one or more of the following adjuvants: sterile diluents such as water for injection, saline solution, fixed oils, polyethylene glycols, glycerine, propylene glycol or other synthetic solvents; antibacterial agents such as benzvl alcohol or methyl paraben; antioxidants such as ascorbic acid or sodium bisulfite; chelating agents such as ethylene diaminetetraacetic acid; buffers such as acetates, citrates or phosphates and agents for the adjustment of toxicity such as sodium chloride or dextrose. The parenteral preparation can be enclosed in ampules, disposable syringes or multiple dose vials made of glass or plastic.

As with any group of structurally related compounds which possess a particular generic utility, certain groups and configurations are preferred for compounds of Formula (I) in their end-use application.

Compounds of Formula (I) wherein Z is -O- are preferred.

It is, of course, understood that the compounds of Formula (I) may exist in a variety of isomeric configurations including structural as well as stereo isomers. It is further understood that the present invention encompasses those compounds of Formula (I) in each of their various structural and stereo isomeric configurations as individual isomers and as mixtures of isomers.

The following specific compounds of Formula (I) are particularly preferred in the end-use application of the compounds of the present invention:
[4S-[4α, 7α(R*), 12bβ]]-7-[(1-oxo-2(S)-thio-3-phenylpropyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2(R)-thio-3-phenylpropyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2-thio-ethyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2(R)-thio-2-phenylethyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2(S)-thio-2-phenylethyl)amino]-1,2,3,4,6,7,8,12b-octahydro-6-oxo-pyrido[2,1-a][2]benzazepine-4-carboxylic acid;
[6α(R*), 11bβ]-6-[(S)-(1-oxo-2(S)-thio-3-phenylpropyl)amino]-1,2,3,5,6,7,11b-heptahydro-5-oxo-pyrrolo[2,1-a][2]benzazepine-3(S)-carboxylic acid;
[6α(R*), 11bβ]-6-[(S)-(1-oxo-2(R)-thio-3-phenylpropyl)amino]-1,2,3,5,6,7,11b-heptahydro-5-oxo-pyrrolo[2,1-a][2]benzazepine-3(S)-carboxylic acid;
[4S-[4α, 7α(R*), 12bβ]]-7-[(1-oxo-2(S)-acetylthio-3-phenylpropyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2(R)-acetylthio-3-phenylpropyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2-acetylthio-ethyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2(R)-acetylthio-2-phenylethyl)amino]-1,2,3,4,6,7,8,12b-octahydro-6-oxo-pyrido[2,1-a][2]benzazepine-4-carboxylic acid;
[4S-[4α, 7α(R*), 12bβ]]-7-[(1-oxo-2(S)-acetylthio-2-phenylethyl)amino]-1,2,3,4,6,7,8,12b-octahydro-6-oxo-pyrido[2,1-a][2]benzazepine-4-carboxylic acid;
[6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-3(S)-carboxylic acid;
[6α(R*), 11bβ]-6-[(S)-(1-oxo-2(R)-acetylthio-3-phenylpropyl)amino]-1,2,3,5,6,7,11b-heptahydro-5-oxo-pyrrolo[2,1-a][2]benzazepine-3(S)-carboxylic acid;
[4S-[4α, 7α(R*), 12bβ]]-7-[(1-oxo-2(S)-acetylthio-3-phenylpropyl)amino]-1,2,3,4,6,7,8,12b-octahydro-6-oxo-pyrido[2,1-a][2]benzazepine-4-carboxylic acid, pivaloyloxymethyl ester;
[4S-[4α, 7α(R*), 12bβ]]-7-[(1-oxo-2(R)-acetylthio-3-phenylpropyl)amino]-1,2,3,4,6,7,8,12b-octahydro-6-oxo-pyrido[2,1-a][2]benzazepine-4-carboxylic acid, pivaloyloxymethyl ester;
[4S-[4α, 7α(R*), 12bβ]]-7-[(1-oxo-2-acetylthio-ethyl)amino]-1,2,3,4,6,7,8,12b-octahydro-6-oxo-pyrido[2,1-a][2]benzazepine-4-carboxylic acid, pivaloyloxymethyl ester;
[4S-[4α, 7α(R*), 12bβ]]-7-[(1-oxo-2(R)-acetylthio-2-phenylethyl)amino]-1,2,3,4,6,7,8,12b-octahydro-6-oxo-pyrido[2,1-a][2]benzazepine-4-carboxylic acid, pivaloyloxymethyl ester;
[4S-[4α, 7α(R*), 12bβ]]-7-[(1-oxo-2(S)-acetylthio-2-phenylethyl)amino]-1,2,3,4,6,7,8,12b-octahydro-6-oxo-pyrido[2,1-a][2]benzazepine-4-carboxylic acid, pivaloyloxymethyl ester;
[6α(R*), 11bβ]-6-[(S)-(1-oxo-2(S)-acetylthio-3-phenylpropyl)amino]-1,2,3,5,6,7,11b-heptahydro-5-oxo-pyrrolo[2,1-a][2]benzazepine-3(S)-carboxylic acid, pivaloyloxymethyl ester;
[6α(R*), 11bβ]-6-[(S)-(1-oxo-2(R)-acetylthio-3-phenylpropyl)amino]-1,2,3,5,6,7,11b-heptahydro-5-oxo-pyrrolo[2,1-a][2]benzazepine-3(S)-carboxylic acid, pivaloyloxymethyl ester;
[4S-[4α, 7α(R*), 12bβ]]-7-[(1-Oxo-2(S)-thio-3-phenylpropyl)amino]-1,2,3,4,6,7,8,12b-hexahydro-6-oxo-1H-[1,4]-oxazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-oxazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-oxazino[3,4-a][2]benzazepine-4-carboxylic acid, pivaloyloxymethyl ester;
[4S-[4α, 7α(R*), 12bβ]]-7-[(1-Oxo-2(S)-thio-3-phenylpropyl)amino]-1,2,3,4,6,7,8,12b-hexahydro-6-oxo-1H-[1,4]-thiazino[3,4-a][2]benzazepine-4-carboxylic acid;
[4S-[4α, 7α(R*), 12bβ]]-7-[(1-Oxo-2(S)-acetylthio-3-phenylpropyl)amino]-l,2,3,4,6,7,8,l2b-hexahydro-6-oxo-1H-[1,4]-thiazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-thiazino[3,4-a][2]benzazepine-4-carboxylic acid, pivaloyloxymethyl ester;
[4S-[4α, 7α(R*), 12bβ]]-7-[(1-Oxo-2(S)-thio-3-phenylpropyl)amino]-1,2,3,4,6,7,8,12b-hexahydro-6-oxo-1H-[1,4]-azazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-azazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-azazino[3,4-a][2]benzazepine-4-carboxylic acid, pivaloyloxymethyl ester;
[4S-[4α, 7α(R*), 12bβ]]-7-[(1-Oxo-2(S)-thio-3-phenylpropyl)amino]-1,2,3,4,6,7,8,12b-hexahydro-6-oxo-1H-[1,4]-N⁴-benzyl-azazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-N⁴-benzyl-azazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-N⁴-benzyl-azazino[3,4-a][2]benzazepine-4-carboxylic acid, pivaloyloxymethyl ester;
[4S-[4α, 7α(R*), 12bβ]]-7-[(1-Oxo-2(S)-thio-3-phenylpropyl)amino]-1,2,3,4,6,7,8,12b-hexahydro-6-oxo-1H-[1,4]-N⁴-benzoyl-azazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-N⁴-benzoyl-azazino[3,4-a][2]benzazepine-4-carboxylic acid;
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-N⁴-benzoyl-azazino[3,4-a][2]benzazepine-4-carboxylic acid, pivaloyloxymethyl ester;

The following studies illustrate the utility of the compounds of the present invention as enkephalinase inhibitors and as ACE inhibitors.

Enkephalinase is partially purified from rat kidney. The enzyme is extracted from the microvilli fraction by using Triton X-100 according to the method of Malfroy and Schwartz [*J.Biol.Chem.* 259, 14365-14370 (1984)] or by using proteolytic treatment according to the method of Almenoff and Orlowski [*Biochem.* 22, 590-599 (1983)]. The enzyme is further purified by anion exchange chromatography (Mono Q™ column, Pharmacia) using a Pharmacia FPLC system. The enzyme activity may be measured by the fluorometric methods of Florentin et al. [*Anal.Biochem*. 141, 62-69 (1984)] or of Almenoff and Orlowski [*J.Neurochemistry* 42, 151-157 (1984)]. The enzyme is assayed in 50mM HEPES buffer (pH 7.4) in a 3.0 mL reaction volume containing 12 µM of the substrate dansyl-D-AlaGly(p-nitro)PheGly, (Kₘ=40µM) at 25°C. The substrate (and inhibitor) is added from a concentrated stock soluticn in DMSO (up to 0.1 mL DMSO final volume). The enzyme in a small volume (approximately 0.1 µg of FPLC purified protein) is added to initiate the reaction and the rate of fluorescense increase is recorded continuously using a fluorometer (excitation at 339nm, emission at 562nm).

The enzymatic activity of ACE is monitored using the spectrophotometric substrate described by Holmquist et al. [*Anal.Biochem*. 95, 540-548 (1979)] and the buffer system described by Ryan *[Methods of Enzymatic Analysis,* 3rd ed., H. U. Bergmeyer, editor; vol. V, Verlag Chemie, Weinheim, 1983, pp. 20-34].

The results of the analysis of enzymatic activity as described in Table 1 indicate that the compounds of the present invention are inhibitors of enkephalinase as well as inhibitors of ACE.

**Table 1**

| Kᵢ's of Compounds of Formula (I) as Inhibitors of Enkephalinase and of ACE | | |
|---|---|---|
| Compound of Formula (I) | Enkephalinase, Kᵢ(nM) | ACE, Kᵢ (nM) |
| 100,173 | < 1 | 0.74 |
| 101,628 | < 1 | 6.7 |
| 27,855 | 8 | 5 |
| 101,804 | 1 | 2.1 |
| 100,919 | < 1 | 1 |
| 101,519 | < 1 | < 7 |
| 102,179 | < 1 | < 7 |
| 100,173 = [4S-[4α, 7α(R*), 12bβ]]-7-[(1-oxo-2(S)-thio-3-phenylpropyl)amino]-1,2,3,4,6,7,8,12b-octahydro-6-oxo-pyrido[2,1-a][2]benzazepine-4-carboxylic acid | | |
| 101,628 = [4S-[4α, 7α(R*), 12bβ]]-7-[(1-Oxo-2(R)-thio-3-phenylpropyl)amino]-1,2,3,4,6,7,8,12b-octahydro-6-oxo-pyrido[2,1-a][2]benzazepine-4-carboxylic acid | | |
| 27,855 = [4S-[4α, 7α(R*), 12bβ]]-7-[(1-oxo-2-thio-ethyl)amino]-1,2,3,4,6,7,8,12b-octahydro-6-oxo-pyrido[2,1-a][2]benzazepine-4-carboxylic acid | | |
| 101,804 = [4S-[4α, 7α(R*), 12bβ]]-7-[(1-Oxo-2-thio-2-phenylethyl)amino]-1,2,3,4,6,7,8,12b-octahydro-6-oxo-pyrido[2,1-a][2]benzazepine-4-carboxylic acid | | |
| 100,919 = [6α(R*), 11bβ]-6-[(S)-(1-oxo-2(R)-thio-3-phenylpropyl)amino]-1,2,3,5,6,7,11b-heptahydro-5-oxo-pyrrolo[2,1-a][2]benzazepine-3(S)-carboxylic acid | | |
| 101,519 = [4S-[4α, 7α(R*), 12bβ]]-7-[(1-Oxo-2(S)-thio-3-phenylpropyl)amino]-1,2,3,4,6,7,8,12b-hexahydro-6-oxo-1 H-[1,4]-oxazino[3,4-a][2]benzazepine-4-carboxylic acid | | |
| 102,179 = [4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]oxazino[3,4-a][2]benzazepine-4-carboxylic acid | | |

## Claims

1. A compound of the formula (I) 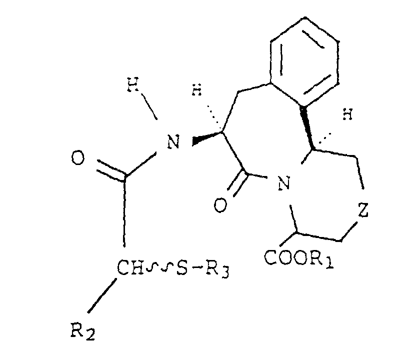 wherein
R₁ is hydrogen; -CH₂O-C(O)C(CH₃)₃; a C₁-C₄ alkyl; an Ar-Y-group wherein Ar is aryl and Y is a single bond or a C₁-C₄ alkyl; or diphenylmethyl;
R₂ is hydrogen, C₁-C₈ alkyl, -CH₂OCH₂CH₂OCH₃ or an Ar-Y-group;
R₃ is hydrogen, acetyl, -CH₂O-C(O)C(CH₃)₃ or benzoyl;
Z is a group (CH₂)ₙ, -O-, S, NR₆ or N-C(O)R₇ wherein n is an integer 0 or 1, R₆ is hydrogen, a C₁-C₄ alkyl or an Ar-Y-group and R₇ is -CF₃, C₁-C₁₀ alkyl or an Ar-Y-group,
wherein
"Ar" refers to a phenyl or naphthyl group unsubstituted or substituted with from one to three substituents selected from the group consisting of methylenedioxy, hydroxy, C₁-C₄ alkoxy, fluoro and chloro.

2. A compound according to Claim 1 wherein n is 1.

3. A compound according to Claim 1 wherein n is 0.

4. A compound according to Claims 1-3 wherein R₂ is phenylmethyl.

5. A compound according to Claim 1, namely
[4S-[4α, 7α(R*), 12bβ]]-7-[(1-oxo-2(S)-thio-3-phenylpropyl)amino]-1,2,3,4,6,7,8,12b-octahydro-6-oxo-pyrido[2,1-a][2]benzazepine-4-carboxylic acid,
[4S-[4α, 7α(R*), 12bβ]]-7-[(1-oxo-2(S)-acetylthio-3-phenylpropyl )amino)-1,2,3,4,6,7,8,12b-octahydro-6-oxo-pyrido[2,1-a][2]benzazepine-4-carboxylic acid,
[4S-[4α, 7α(R*), 12bβ]]-7-[(1-Oxo-2(S)-acetylthio-3-phenylpropyl)amino]-1,2,3,4,6,7,8,12b-octahydro-6-oxo-pyrido[2,1-a][2]benzazepine-4-carboxylic acid, pivaloyloxymethyl ester,
[4S-[4α, 7α(R*), 12bβ]]-7-[(1-oxo-2(R)-thio-3-phenylpropyl )amino]-1,2,3,4,6,7,8,12b-octahydro-6-oxo-pyrido[2,1-a][2]benzazepine-4-carboxylic acid,
[4S-[4α, 7α(R*), 12bβ]]-7-[(1-oxo-2-thio-ethyl)amino]-1,2,3,4,6,7,8,12b-octahydro-6-oxo-pyrido[2,1-a][2]benzazepine-4-carboxylic acid,
[4S-[4α, 7α(R*), 12bβ]]-7-[(1-oxo-2-thio-2-phenylethyl)amino]-1,2,3,4,6,7,8,12b-octahydro-6-oxo-pyrido[2,1-a][2]benzazepine-4-carboxylic acid,
[6α(R*), 11bβ]-6-[(S)-(1-oxo-2(R)-thio-3-phenylpropyl)amino]-1,2,3,5,6,7,11b-heptahydro-5-oxo-pyrrolo[2,1-a][2]benzazepine-3(S)-carboxylic acid, or
[6α(R*), 11bβ]-6-[(S)-(1-oxo-2(S)-thio-3-phenylpropyl)amino]-1,2,3,5,6,7,11b-heptahydro-5-oxo-pyrrolo[2,1-a][2]benzazepine-3(S)-carboxylic acid.

6. A compound according to Claim 1, namely
[4S-[4α, 7α(R*), 12bβ]]-7- [(1-Oxo-2(S)-acetylthio-3-phenylpropyl)amino]-1,2,3,4,6,7,8,12b-hexahydro-6-oxo-1H-[1,4]-oxazino[3,4-a][2]benzazepine-4-carboxylic acid,
[4S-[4α, 7α(R*), 12bβ]]-7-[(1-Oxo-2(S)-thio-3-phenylpropyl)amino]-1,2,3,4,6,7,8,12b-hexahydro-6-oxo-1H-[1,4]-oxazino[3,4-a][2]benzazepine-4-carboxylic acid,
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-oxazino[3,4-a][2]benzazepine-4-carboxylic acid,
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-oxazino[3,4-a][2]benzazepine-4-carboxylic acid, pivaloyloxymethyl ester.

7. A compound according to Claim 1 wherein Z is -O-.

8. A compound according to Claim 1 wherein Z is -S-.

9. A compound according to Claim 1 wherein Z is -NH-.

10. A process for preparing a compound of the Formula (I') according to anyone of claims 1-5 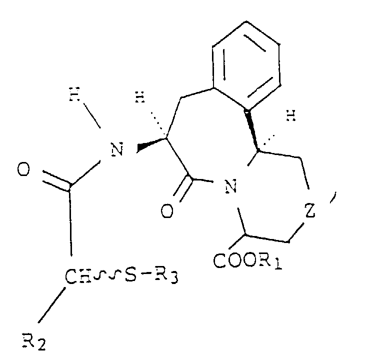 wherein R₁,R₂ and R₃ are as defined in claim 1 and Z' represents (CH₂)n wherein n is an integer 0 or 1,
which comprises
a) if
R₁ is diphenylmethyl and
R₃ is acetyl or benzoyl;
reacting a compound of the formula 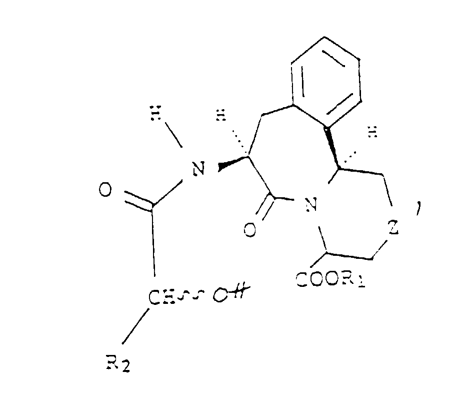 wherein Z', R₁, n and R2 are as defined above with thiolacetic acid or thiolbenzoic acid in the presence of triphenylphosphine and an oxidizing agent;
b) if
R₁ is hydrogen;
R₃ is acetyl or benzoyl; and R₂ and n are as defined above,
reacting a compound of the formula 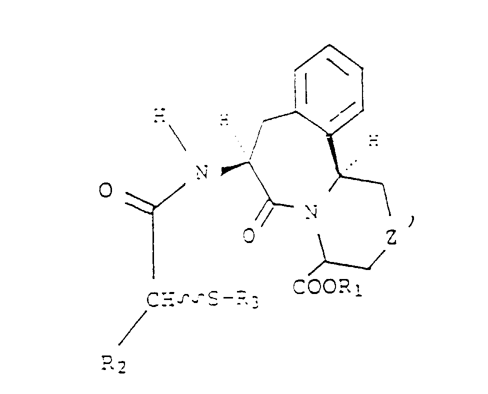 wherein
R₁ is diphenylmethyl;
R₃, Z', n and R₂ are as defined above, with an acid,
c) if
R₁ is hydrogen;
R₃ is hydrogen; and Z',
R₂ and n are as defined above,
reacting a compound of the formula 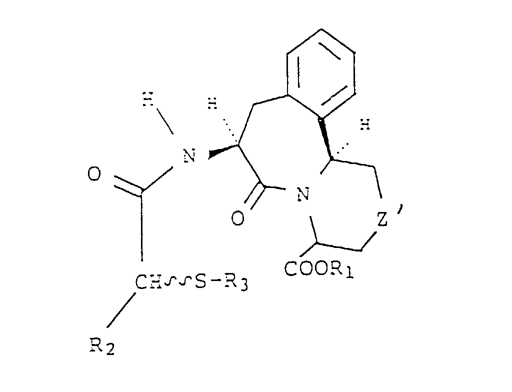 wherein
R₃ is acetyl or benzoyl; and
Z', R₁, n and R₂ are as defined above, with a base,
d) if
R₁ is -CH₂O-C(O)C(CH₃)₃; a C₁-C₄ alkyl; an Ar-Y- group wherein Ar is aryl and Y is a C₀-C₄ alkyl;
R₃ is acetyl or benzoyl; and
Z', R₂ and n are as defined above,
reacting a compound of the formula 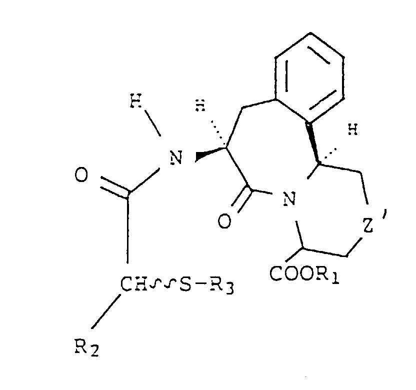 wherein
R₁ is hydrogen;
Z', n, R₃ and R₂ are as defined above, in the presence of base,
with the appropriate C₁-C₄ alkyl halide, chloromethyl pivalate or Ar-Y halide wherein Ar and Y are defined as above,
e) if
R₁ is -CH₂O-C(O)C(CH₃)₃; a C₁-C₄ alkyl, an Ar-Y- group wherein Ar is aryl and Y is a C₀-C₄ alkyl or diphenylmethyl;
R₃ is hydrogen; and
Z', R₂ and n are as defined above,
reacting a compound of the formula 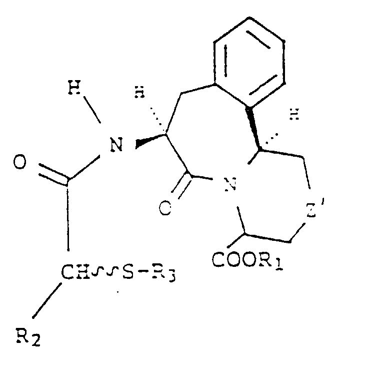 wherein
R₃ is acetyl or benzoyl and R₁, Z', n, and R₂ are as defined above with ammonia, or
f) if
R₄ is hydrogen; -CH₂O-C(O)C(CH₃)₃; a C₁-C₄ alkyl; an Ar-Y- group wherein Ar is aryl and Y is a C₀-C₄ alkyl or diphenylmethyl;
R₃ is -CH₂O-C(O)C(CH₃)₃; and
Z', R₂ and n are as defined above,
reacting a compound of the formula 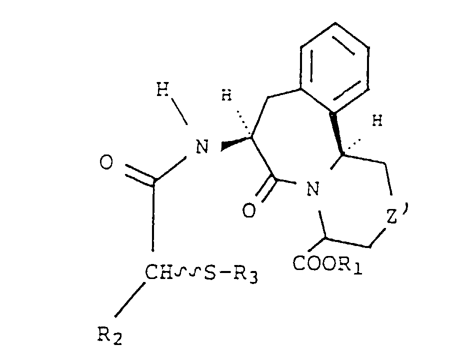 wherein
R₃ is hydrogen; and
R₁, Z', n, and R₂ are as defined above, in the presence of base, with chloromethyl pivalate.

11. A process for preparing a compound of the Formula I" according to any one of claims 1 and 6-9 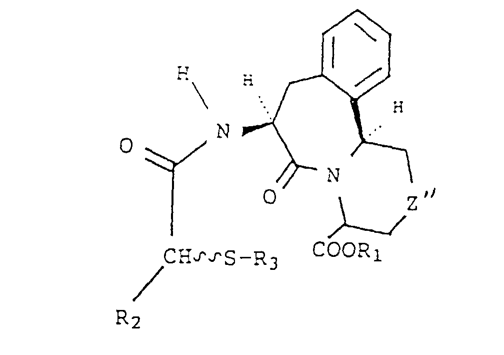 wherein
R₁, R₂ and R₃ are as defined in claim 1 and
Z" is -O-, -S-, 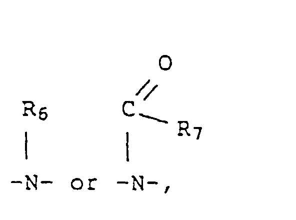 wherein R₆ is hydrogen, a C₁-C₄ alkyl or an Ar-Y-group and R₇ is -CF₃, a C₁-C₁₀ alkyl or an Ar-Y-group,
which comprises
a) if
R₃ is acetyl or benzoyl;
R₁ is diphenylmethyl; and
Z is -O-, -S-, 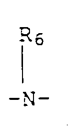 wherein
R₆ is hydrogen
and R₂ is as defined above,
reacting a compound of the formula 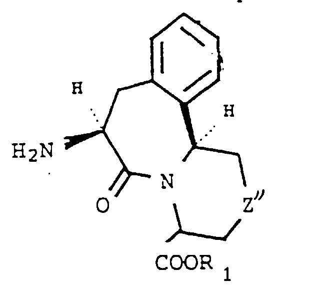 wherein
R₁ and Z'' are defined as above with a compound of the formula 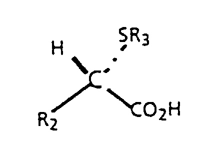 wherein
R₃ and R₂ are defined as above in the presence of a coupling reagent,
b) if
R₃ is acetyl or benzoyl;
R₁ is hydrogen; and
Z'' is -O-, -S-, 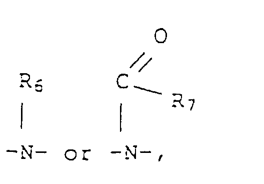 wherein
R₆ is hydrogen, a C₁-C₄ alkyl or an Ar-Y- group; and
R₇ is -CF₃, C₁-C₁₀ alkyl or an Ar-Y- group
and R₂ is as defined above,
reacting a compound of the formula 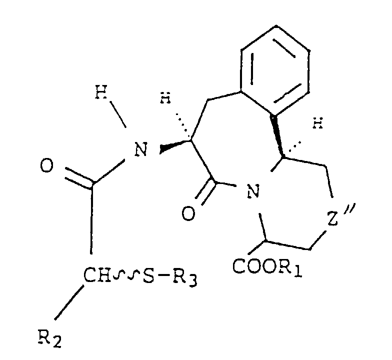 wherein
R₂, R₃ and Z'' are defined as above; and
R₁, is diphenylmethyl with an acid,
c) if
R₃ is hydrogen;
R₁ is hydrogen; and
Z'' is -O-, -S-, 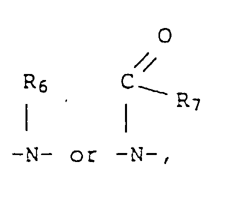 wherein
R₆ is hydrogen, a C₁-C₄ alkyl or an Ar-Y- group; and
R₇ is -CF₃, C₁-C₁₀ alkyl or an Ar-Y group
and R₂ is as defined above,
reacting a compound of the formula 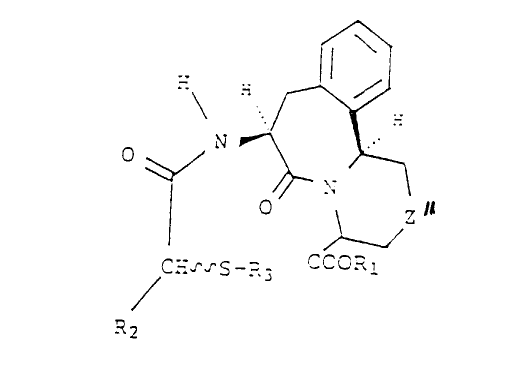 wherein
R₂, R₁ and Z'' are defined as above; and
R₃ is acetyl with a base,
d) if
R₃ is acetyl or benzoyl;
R₁ is -CH₂O-C(O)(CH₃)₃; a C₁-C₄ alkyl; an Ar-Y- group wherein Ar is aryl and Y is a C₀-C₄ alkyl; and
Z'' is -O-, -S-, 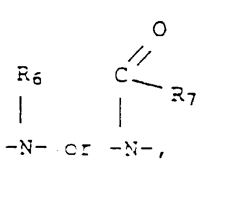 wherein
R₅ is hydrogen, a C₁-C₄ alkyl or an Ar-Y- group; and
R₇ is -CF₃, C₁-C₁₀ alkyl or an Ar-Y- group
and R₂ if as defined above,
reacting a compound of the formula 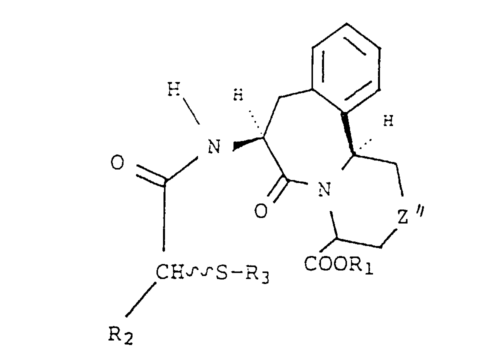 wherein
R₂, R₃ and Z'' are defined as above; and
R₁ is hydrogen, in the presence of base, with the appropriate C₁-C₄ alkyl halide, chloromethyl pivalate or Ar-Y halide wherein Ar and Y are defined as above,
e) if
R₃ is hydrogen;
R₁ is -CH₂O-C(O)(CH₃)₃; diphenylmethyl; a C₁-C₄ alkyl; an Ar-Y- group wherein Ar is aryl and Y is a C₀-C₄ alkyl; and
Z'' is -O-, -S-, 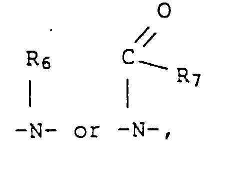 wherein
R₆ is hydrogen, a C₁-C₄ alkyl or an Ar-Y- group; and
R₇ is -CF₃, C₁-C₁₀ alkyl or an Ar-Y- group
and R₂ is as defined above,
reacting a compound of the formula 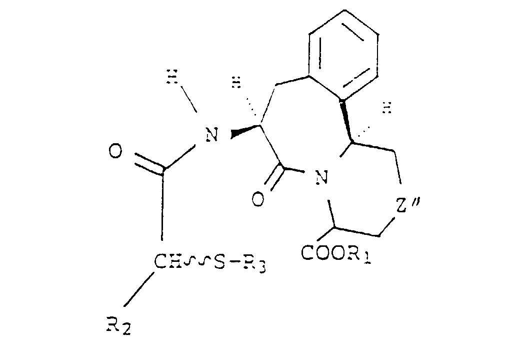 wherein
R₂, R₁ and Z'' are defined as above; and
R₃ is acetyl or benzoyl with ammonia,
f) if
R₃ is -CH₂O-C(O)C(CH₃)₃;
R₁ is hydrogen, -CH₂O-C(O)(CH₃)₃; diphenylmethyl; a C₁-C₄ alkyl; an Ar-Y- group wherein Ar is aryl and Y is a C₀-C₄ alkyl; and
Z'' is -O-, -S-, 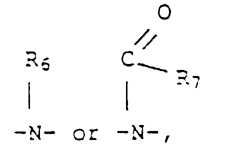 wherein
R₆ is hydrogen, a C₁-C₄ alkyl or an Ar-Y- group; and
R₇ is -CF₃, C₁-C₁₀ alkyl or an Ar-Y- group
and R₂ is as defined above,
reacting a compound of the formula 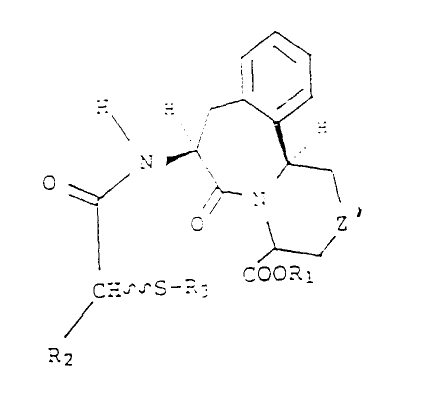 wherein
R₂, R₁ and Z' are defined as above; and R₃ is hydrogen, in the presence of base, with chloromethyl pivalate.

12. A compound according to any one of claims 1-9 for use as a pharmaceutical agent.

13. A composition comprising an assayable amount of a compound according to any one of claims 1-9 in admixture or otherwise in association with an inert carrier.

14. A pharmaceutical composition containing a compound according to any one of claims 1-9 or a mixture thereof and optionally a pharmaceutically acceptable carrier and/or diluent.

15. The use of a compound according to any one of claims 1-9 for the preparation of a pharmaceutical composition for use as an enkephalinase inhibitor.

16. The use of a compound according to any one of claims 1-9 for the preparation of a pharmaceutical composition for use as an ACE inhibitor.

17. The use of a compound according to any one of claims 1-9 for the preparation of a pharmaceutical composition for use in the treatment of acute or chronic pain, as an antihypertensive agent, for the treatment of congestive heart failure, for the treatment of cardiac hypertrophy or as a diuretic.

18. A method for the preparation of a pharmaceutical composition as defined in claim 14 comprising combining a compound as defined in any one of claims 1-9 with a pharmaceutically acceptable carrier.

## Patentansprüche

1. Verbindung der Formel (I) 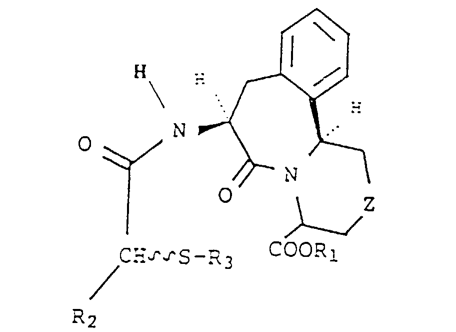
in der R₁ ein Wasserstoffatom, eine Gruppe -CH₂O-C(O)C(CH₃)₃, einen C₁-C₄-Alkyl-rest, einen Ar-Y-Rest, wobei Ar einen Arylrest bedeutet und Y eine Einfachbindung oder einen C₁-C₄-Alkyirest darstellt, oder eine Diphenylmethylgruppe bedeutet;
R₂ ein Wasserstoffatom, einen C₁-C₈-Alkylrest, eine Gruppe -CH₂OCH₂CH₂OCH₃ oder einen Ar-Y-Rest darstellt;
R₃ ein Wasserstoffatom, eine Acetylgruppe, eine Gruppe -CH₂O-C(O)C(CH₃)₃ oder eine Benzoylgruppe bedeutet;
Z eine Gruppe (CH₂)ₙ, -O-, S, NR₆ oder N-C(O)R₇ darstellt, wobei n eine ganze Zahl 0 oder 1 ist, R₆ ein Wasserstoffatom, einen C₁-C₄-Alkylrest oder einen Ar-Y-Rest bedeutet und R₇ eine Gruppe -CF₃, einen C₁-C₁₀-Alkylrest oder einen Ar-Y-Rest darstellt, wobei
"Ar" einen Phenyl- oder Naphthylrest bedeutet, der unsubstituiert oder mit einem bis drei Substituenten substituiert ist, die aus der Gruppe ausgewählt sind, die aus einer Methylendioxygruppe, einer Hydroxygruppe, einem C₁-C₄-Alkoxyrest, einem Fluor- und Chloratom besteht.

2. Verbindung nach Anspruch 1, wobei n 1 ist.

3. Verbindung nach Anspruch 1, wobei n 0 ist.

4. Verbindung nach den Ansprüchen 1 bis 3, wobei R₂ eine Phenylmethylgruppe ist.

5. Verbindung nach Anspruch 1, nämlich [4S-[4α,7α(R*), 12bβ]]-7-[(1-Oxo-2(S)-thio-3-phenylpropyl)amino]-1,2,3,4,6,7,8,12b-octahydro-6-oxopyrido[2,1-a][2]benzazepin-4-carbonsäure,
[4S-[4α, 7α(R*), 12bβ]]-7-[(1-Oxo-2(S)-acetylthio-3-phenylpropyl)amino]-1,2,3,4,6,7,8,12b-octahydro-6-oxopyrido[2, 1 -a][2]benzazepin-4-carbonsäure,
[4S-[4α, 7α(R*), 12bβ]]-7-[(1-Oxo-2(S)-acetylthio-3-phenylpropyl)amino]-1,2,3,4,6,7,8,12b-octahydro-6-oxopyrido[2,1-a][2]benzazepin-4-carbonsäurepivaloyioxymethyiester,
[4S-[4α, 7α(R*), 12bβ]]-7-[( 1 -Oxo-2(R)-thio-3-phenylpropyl)amino]-1,2,3,4,6,7,8,12b-octahydro-6-oxopyrido[2,1 -a][2}benzazepin-4-carbonsäure,
[4S-[4α,7α(R*), 12bβ]]-7-[(1-Oxo-2-thioethyl)amino]-1,2,3,4,6,7,8,12b-octahydro-6-oxo-pyrido[2,1-a][2]benzazepin-4-carbonsäure,
[4S-[4α,7α(R*), 12bβ]]-7-[(1-Oxo-2-thio-2-phenylethyl)amino] 1,2,3,4,6,7,8,12b-octahydro-6-oxopyrido[2,1-a][2]benzazepin-4-carbonsäure,
[6α(R*),11bβ]-6-[(S)-(1-Oxo-2(R)-thio-3-phenylpropyl)amino]-1,2,3,5,6,7,11b-heptahydro-5-oxopyrrolo[2,1-a][2]benzazepin-3(S)-carbonsäure oder
[6α(R*),11bβ]-6-[(S)-(1-Oxo-2(S)-thio-3-phenylpropyl)amino]-1,2,3,5,6,7,11b-heptahydro-5-oxopyrrolo[2,1-a][2]benzazepin-3(S)-carbonsäure.

6. Verbindung nach Anspruch 1, nämlich [4S-[4α,7α(R*),12bβ]]-7-[(1-Oxo-2(S)-acetylthio-3-phenylpropyl)amino]-1,2,3,4,6,7,8,12b-hexahydro-6-oxo-1H-[1,4]-oxazino-[3,4-a][2]benzazepin-4-carbonsäure,
[4S-[4α,7α(R*), 12bβ]]-7-[(1-Oxo-2(S)-thio-3-phenylpropyl)amino]-1,2,3,4,6,7,8,12b-hexahydro-6-oxo-1H-[1,4]-oxazino[3,4-a][2]benzazepin-4-carbonsäure,
[4S-[4α, 7α(R*), 12bβ]]-7-[(1-Oxo-2(S)-acetylthio-3-phenylpropyl)amino]-1,2,3,4,6,7,8,12b-hexahydro-6-oxo-1H-[1,4]-oxazino[3,4-a][2]benzazepin-4-carbonsäure,
[4S-[4α,7α(R*), 12bβ]]-7-[(1-Oxo-2(S)-acetylthio-3-phenylpropyl)amino]-1,2,3,4,6,7,8,12b-hexahydro-6-oxo-1H-[1,4]-oxazino[3,4-a][2]benzazepin-4-carbonsäure-pivaloyloxymethylester.

7. Verbindung nach Anspruch 1, wobei Z -O- bedeutet.

8. Verbindung nach Anspruch 1, wobei Z -S- darstellt.

9. Verbindung nach Anspruch 1, wobei Z -NH- bedeutet.

10. Verfahren zum Herstellen einer Verbindung der Formel (I') nach einem der Ansprüche 1 - 5 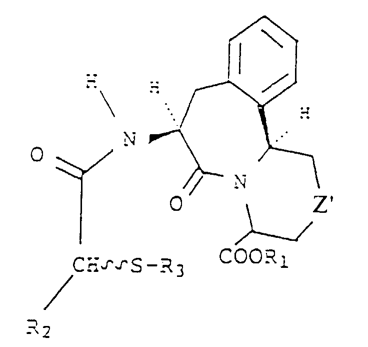 in der R₁, R₂ und R₃ wie in Anspruch 1 definiert sind und Z' eine Einheit (CH₂)ₙ wiedergibt, wobei n eine ganze Zahl 0 oder 1 ist, das umfaßt
a) falls
R₁ eine Diphenylmethylgruppe bedeutet und
R₃ eine Acetyl- oder Benzoylgruppe darstellt;
Umsetzen einer Verbindung der Formel 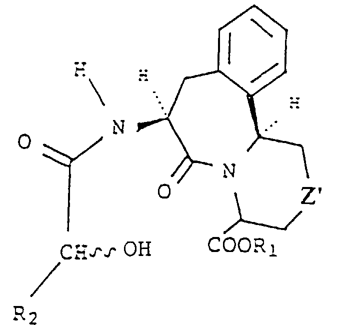 in der Z', R₁, n und R₂ wie vorstehend definiert sind, mit Thioessigsäure oder Thiobenzoesäure in Gegenwart von Triphenylphosphin und einem Oxidationsmittel;
b) falls
R₁ ein Wasserstoffatom bedeutet;
R₃ eine Acetyl- oder Benzoylgruppe darstellt und R₂ und n wie vorstehend definiert sind;
Umsetzen einer Verbindung der Formel 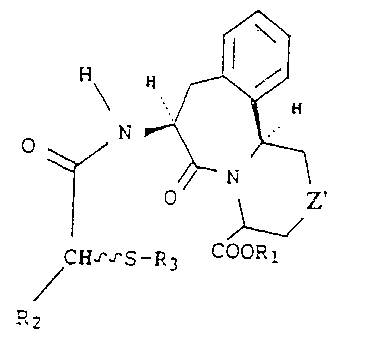
in der R₁ eine Diphenylmethylgruppe bedeutet;
R₃, Z', n und R₂ wie vorstehend definiert sind, mit einer Säure;
c) falls
R₁ ein Wasserstoffatom bedeutet
R₃ ein Wasserstoffatom darstellt und Z', R₂ und n wie vorstehend definiert sind,
Umsetzen einer Verbindung der Formel 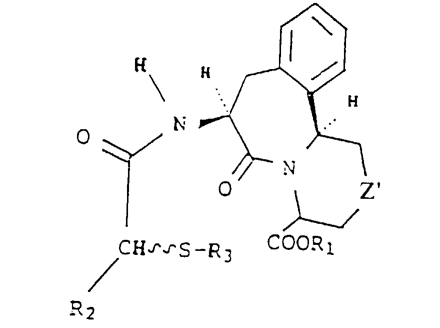
in der R₃ eine Acetyl- oder Benzoylgruppe darstellt; und
Z', R₁, n und R₂ wie vorstehend definiert sind, mit einer Base;
d) falls
R₁ eine Gruppe -CH₂O-C(O)C(CH₃)₃, einen C₁-C₄-Alkylrest, einen Ar-Y-Rest darstellt, wobei Ar einen Arylrest bedeutet und Y einen C₀-C₄-Alkylrest darstellt;
R₃ eine Acetyl- oder Benzoylgruppe bedeutet und
Z', R₂ und n wie vorstehend definiert sind;
Umsetzen einer Verbindung der Formel 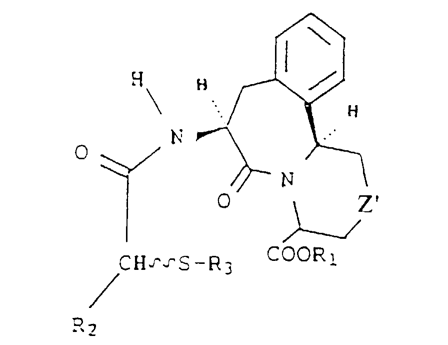 in der
R₁ ein Wasserstoffatom bedeutet,
Z', n, R₃ und R₂ wie vorstehend definiert sind, in Gegenwart einer Base,
mit dem entsprechenden C₁-C₄-Alkylhalogenid, Pivalinsäurechlormethylester oder einem Ar-Y-halogenid, wobei Ar und Y wie vorstehend definiert sind,
e) falls
R₁ eine Gruppe -CH₂O-C(O)C(CH₃)₃, einen C₁-C₄-Alkylrest, einen Ar-Y-Rest, wobei Ar einen Arylrest darstellt und Y einen C₀-C₄-Alkylrest bedeutet, oder eine Diphenylmethylgruppe darstellt;
R₃ ein Wasserstoffatom darstellt und
Z', R₂ und n wie vorstehend definiert sind,
Umsetzen einer Verbindung der Formel 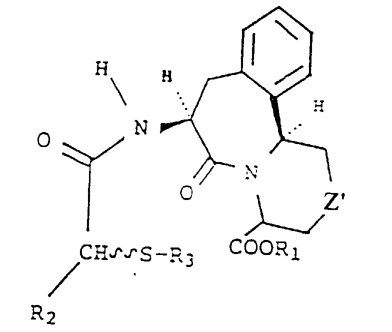 in der R₃ eine Acetyl- oder Benzoylgruppe darstellt und R₁, Z', n und R₂ wie vorstehend definiert sind mit Ammoniak, oder
f) falls
R₄ ein Wasserstoffatom, eine Gruppe -CH₂O-C(O)C(CH₃)₃, einen C₁-C₄-Alkylrest, einen Ar-Y-Rest, wobei Ar eine Arylgruppe bedeutet und Y einen C₀-C₄-Alkylrest darstellt, oder eine Diphenylmethylgruppe bedeutet;
R₃ eine Gruppe -CH₂O-C(O)C(CH₃)₃ darstellt und
Z', R₂ und n wie vorstehend definiert sind,
Umsetzen einer Verbindung der Formel 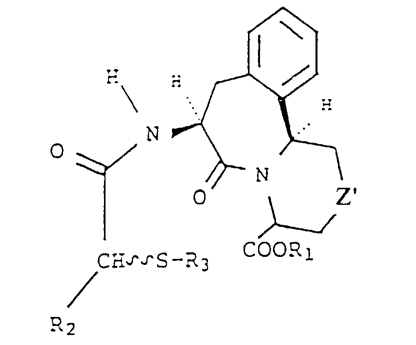 in der
R₃ ein Wasserstoffatom bedeutet und
R₁, Z', n und R₂ wie vorstehend definiert sind, in Gegenwart einer Base mit Pivalinsäurechlormethylester.

11. Verfahren zum Herstellen einer Verbindung der Formel (I'') nach einem der Ansprüche 1 und 6 - 9 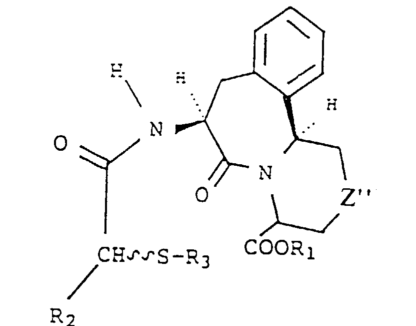
in der R₁, R₂ und R₃ wie in Anspruch 1 definiert sind und
Z" -O-, -S-, 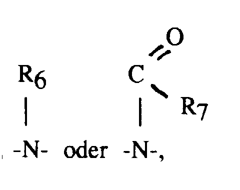
wobei R₆ ein Wasserstoffatom, einen C₁-C₄-Alkylrest oder einen Ar-Y-Rest darstellt und
R₇ eine Gruppe -CF₃, einen C₁-C₁₀-Alkylrest oder einen Ar-Y-Rest bedeutet,
das umfaßt
a) falls
R₃ eine Acetyl- oder Benzoylgruppe darstellt;
R₁ eine Diphenylmethylgruppe bedeutet und
Z'' eine Gruppe -O-, -S-, 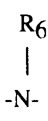 darstellt,
wobei R₆ ein Wasserstoffatom bedeutet und R₂ wie vorstehend definiert ist,
Umsetzen einer Verbindung der Formel 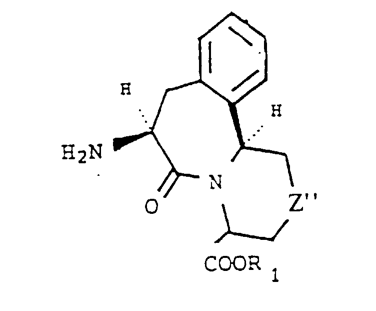 in der R₁ und Z" wie vorstehend definiert sind, mit einer Verbindung der Formel 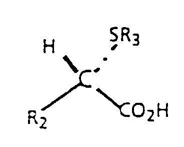 wobei R₃ und R₂ wie vorstehend definiert sind, in Gegenwart eines Kupplungsreagens,
b) falls
R₃ eine Acetyl- oder Benzoylgruppe darstellt;
R₁ ein Wasserstoffatom bedeutet und
Z'' eine Gruppe -O-, -S-, 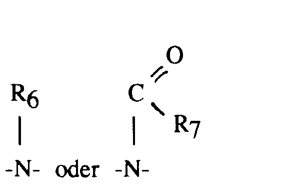 darstellt,
wobei R₆ ein Wasserstoffatom, einen C₁-C₄-Alkylrest oder einen Ar-Y-Rest bedeutet
und R₇ eine Gruppe -CF₃, einen C₁-C₁₀-Alkylrest oder einen Ar-Y-Rest darstellt
und R₂ wie vorstehend definiert ist,
Umsetzen einer Verbindung der Formel 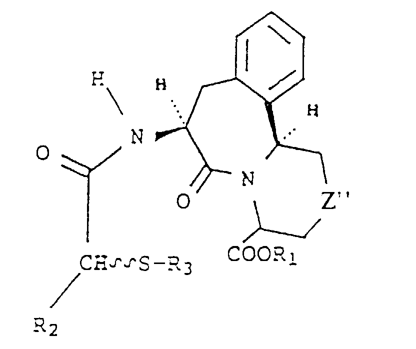 in der
R₂, R₃ und Z" wie vorstehend definiert sind und
R₁ eine Diphenylmethylgruppe bedeutet, mit einer Säure,
c) falls
R₃ ein Wasserstoffatom bedeutet;
R₁ ein Wasserstoffatom darstellt und
Z'' eine Gruppe -O-, -S-, 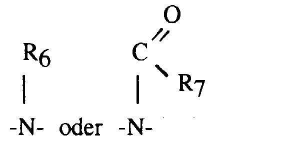 bedeutet,
wobei R₆ ein Wasserstoffatom, einen C₁-C₄-Alkylrest oder einen Ar-Y-Rest darstellt
und R₇ eine Gruppe -CF₃, einen C₁-C₁₀-Alkylrest oder einen Ar-Y-Rest bedeutet
und R₂ wie vorstehend definiert ist,
Umsetzen einer Verbindung der Formel 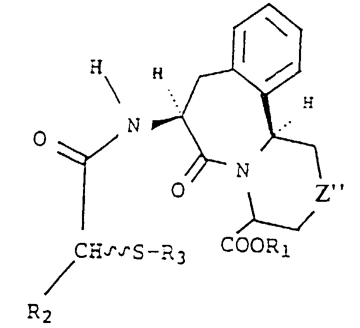
in der R₂, R₁ und Z" wie vorstehend definiert sind und
R₃ eine Acetylgruppe bedeutet, mit einer Base,
d) falls
R₃ eine Acetyl- oder Benzoylgruppe darstellt;
R₁ eine Gruppe -CH₂O-C(O)(CH₃)₃, einen C₁-C₄-Alkylrest, einen Ar-Y-Rest bedeutet, wobei Ar eine Arylgruppe darstellt und Y einen C₀-C₄-Alkylrest bedeutet; und
Z'' eine Gruppe -O-, -S-, 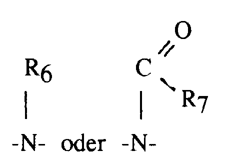 darstellt,
wobei R₆ ein Wasserstoffatom, einen C₁-C₄-Alkylrest oder einen Ar-Y-Rest bedeutet
und R₇ eine Gruppe -CF₃, einen C₁-C₁₀-Alkylrest oder einen Ar-Y-Rest darstellt
und R₂ wie vorstehend definiert ist,
Umsetzen einer Verbindung der Formel 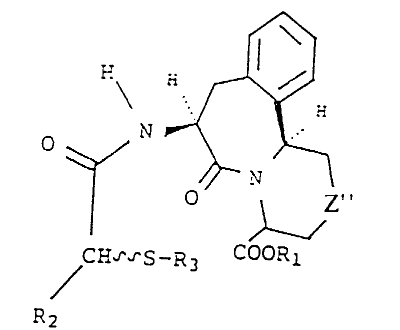
in der R₂, R₃ und Z'' wie vorstehend definiert sind und
R₁ ein Wasserstoffatom darstellt, in Gegenwart einer Base,
mit dem entsprechenden C₁-C₄-Alkylhalogenid, Pivalinsäurechlormethylester oder Ar-Y-halogenid, wobei Ar und Y wie vorstehend definiert sind,
e) falls
R₃ ein Wasserstoffatom bedeutet,
R₁ eine Gruppe -CH₂O-C(O)(CH₃)₃,eine Diphenylmethylgruppe, einen C₁-C₄-Alkylrest, einen Ar-Y-Rest darstellt, wobei Ar eine Arylgruppe bedeutet und Y einen C₀-C₄-Alkylrest darstellt; und
Z'' eine Gruppe -O-, -S-, 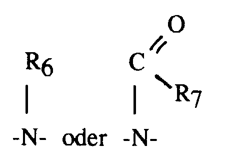 bedeutet,
wobei R₆ ein Wasserstoffatom, einen C₁-C₄-Alkylrest oder einen Ar-Y-Rest darstellt
und R₇ eine Gruppe -CF₃, einen C₁-C₁₀-Alkylrest oder einen Ar-Y-Rest bedeutet
und R₂ wie vorstehend definiert ist,
Umsetzen einer Verbindung der Formel 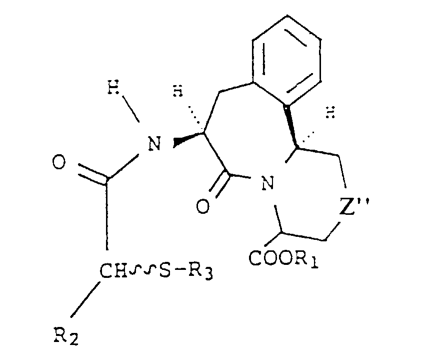 in der R₂, R₁ und Z" wie vorstehend definiert sind, und R₃ eine Acetyl- oder Benzoylgruppe bedeutet, mit Ammoniak,
f) falls
R₃ eine Gruppe -CH₂O-C(O)C(CH₃)₃ darstellt,
R₁ ein Wasserstoffatom, eine Gruppe -CH₂O-C(O)(CH₃)₃, eine Diphenylmethylgruppe, einen C₁-C₄-Alkylrest, einen Ar-Y-Rest bedeutet, wobei Ar eine Arylgruppe darstellt und Y einen C₀-C₄-Alkylrest bedeutet und
Z'' eine Gruppe -O-, -S-, 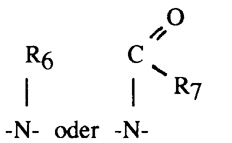 bedeutet,
wobei R₆ ein Wasserstoffatom, einen C₁-C₄-Alkylrest oder einen Ar-Y-Rest darstellt
und R₇ eine Gruppe -CF₃, einen C₁-C₁₀-Alkylrest oder einen Ar-Y-Rest bedeutet
und R₂ wie vorstehend definiert ist,
Umsetzen einer Verbindung der Formel 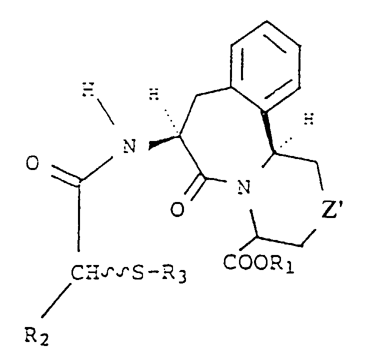 in der
R₂, R₁ und Z' wie vorstehend definiert sind und
R₃ ein Wasserstoffatom bedeutet, in Gegenwart einer Base mit Pivalinsäurechlormethylester.

12. Verbindung nach einem der Ansprüche 1-9 zur Verwendung als Arzneimittel.

13. Zusammensetzung, die eine prüfbare Menge einer Verbindung nach einem der Ansprüche 1-9 in Beimengung oder anderweitig in Verbindung mit einem inerten Träger umfaßt.

14. Arzneimittel, das eine Verbindung nach einem der Ansprüche 1 - 9 oder ein Gemisch davon und gegebenenfalls ein(en) pharmazeutisch verträglichen/s Träger und/oder Verdünnungsmittel enthält.

15. Verwendung einer Verbindung nach einem der Ansprüche 1 - 9 zur Herstellung eines Arzneimittels zur Verwendung als Enkephalinase-Hemmstoff.

16. Verwendung einer Verbindung nach einem der Ansprüche 1 - 9 zur Herstellung eines Arzneimittels zur Verwendung als ACE-Hemmstoff.

17. Verwendung einer Verbindung nach einem der Ansprüche 1 - 9 zur Herstellung eines Arzneimittels zur Verwendung bei der Behandlung von akuten oder chronischen Schmerzen, als Antihypertonikum, zur Behandlung einer Stauungsinsuffizienz des Herzens, zur Behandlung einer Herzhypertrophie oder als Diuretikum.

18. Verfahren zur Herstellung eines Arzneimittels, wie in Anspruch 14 definiert, das das Kombinieren einer Verbindung, wie in einem der Ansprüche 1 - 9 definiert, mit einem pharmazeutisch verträglichen Träger umfaßt.

## Revendications

1. Composé de formule (I) 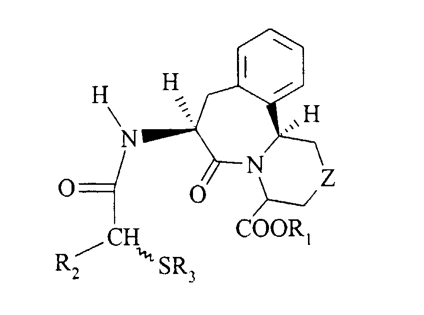
dans laquelle dans laquelle R₁ est hydrogène; -CH₂O-C(O)C(CH₃)₃; un groupe alkyle en C₁-C₄; un groupe Ar-Y-, dans lequel Ar est aryle et Y est une liaison simple ou un groupe alkyle en C₁-C₄; ou diphénylméthyle;
R₂ est hydrogène. alkyle en C₁-C₈, - CH₂OCH₂CH₂OCH₃ ou un groupe Ar-Y-; R₃ est hydrogène, acétyle, -CH₂O-C(O)C(CH₃)₃ ou benzoyle;
Z est un groupe (CH₂)ₙ, -O-, S, NR₆ ou N-C(O)R₇, dans lequel n est un entier égal à 0 ou 1, R₆ est hydrogène, un groupe alkyle en C₁-C₄ ou un groupe Ar-Y- et R₇ est -CF₃, alkyle en C₁-C₁₀ ou un groupe Ar-Y-,
dans laquelle
« Ar » se rapporte à un groupe phényle ou naphtyle substitué ou non avec de un à trois substituants choisis parmi le groupe constitué de méthylènedioxy, hydroxy, alcoxy en C₁-C₄, fluoro et chloro.

2. Composé selon la revendication 1, dans lequel n est 1.

3. Composé selon la revendication 1, dans lequel n est 0.

4. Composé selon les revendications 1 à 3, dans lequel R₂ est phénylméthyle.

5. Composé selon la revendication 1, à savoir
acide [4S-[4α,7α(R*), 12bβ]]-7-[(1-oxo-2(S)-thio-3-phénylpropyl)amino]-1,2,3,4,6,7,8,12b-octahydro-6-oxo-pyrido[2,1-a][2]benzazépine-4-carboxylique,
acide [4S-[4α,7α(R*),12bβ]]-7-[(1-oxo-2(S)-acétylthio-3-phénylpropyl)amine]-1,2,3,4,6,7,8,12b-octahydro-6-oxo-pyrido[2,1-a][2]benzazépine-4-carboxylique,
ester pivaloyloxyméthyle de l'acide [4S-[4α,7α(R*), 12bβ]]-7-[(1-oxo-2(S)-acétylthio-3-phénylpropyl)amino]-1,2,3,4,6,7,8,12b-octahydro-6-oxo-pyrido[2,1-a] [2]benzazépine-4-carboxylique,
acide [4S-[4α,7α(R*], 12bβ]]-7-[(1-oxo-2(R)-thio-3-phénylpropyl)amino]-1,2,3,4,6,7,8,12b-octahydro-6-oxo-pyrido[2,1-a][2]benzazépine-4-carboxylique,
acide [4S-[4α,7α(R*),12bβ]]-7-[(1-oxo-2-thioéthyl)amino]-1,2,3,4,6,7,8,12b-octahydro-6-oxo-pyrido[2,1-a][2]benzazépine-4-carboxylique,
acide [4S-[4α,7α(R*), 12bβ]]-7-[(1-oxo-2-thio-2-phényléthyl)amino]-1,2,3,4,6,7,8,12b-octahydro-6-oxo-pyrido[2,1-a][2]henzazépine-4-carboxylique
acide [6α(R*),11bβ]-6-[(S)-(1-oxo-2(R)-thio-3 -phénylpropyl)amino]-1,2,3,5,6,7,11b-heptahydro-5-oxo-pyrrolo[2,1-a] [2]benzazépine-3(S)-carboxylique ou
acide [6α(R*),11bβ]-6-[(S)-(1-oxo-2(S)-thio-3-phénylpropyl)amino]-1,2,3,5,6,7,11b-heptahydro-5-oxo-pyrrolo[2,1-a] [2]benzazépine-3(S)-carboxylique

6. Composé selon la revendication 1, à savoir
acide [4S-[4α,7α(R*),12bβ]]-7-[(1-oxo-2(S)-acétylthio-3-phénylpropyl)amino]-1,2,3,4,6,7,8,12b-hexahydro-6-oxo-1H-[1,4]-oxazino[3,4-a][2]benzazépine-4-carboxylique,
acide [4S-[4α,7α(R*), 12bβ]]-7-[(1-oxo-2(S)-thio-3-phénylpropyl)amino]-1,2,3,4,6,7,8,12b-hexahydro-6-oxo-1H-[1,4]-oxazino[3,4-a][2]benzazépine-4-carboxylique,
acide [4S-[4α,7α(R*), 12bβ]]-7-[(1-oxo-2(S)-acétylthio-3-phénylpropyl)amino]-1,2,3,4,6,7,8,12b-hexahydro-6-oxo-1H-[1,4]-oxazino[3,4-a][2]benzazépine-4-carboxylique,
ester pivaloyloxyméthylique de l'acide [4S-[4α,7α(R*),12bβ]]-7-[(1-oxo-2(S)-acétylthio-3-phénylpropyl)amino]- 1,2,3,4,6,7,8,12b-hexahydro-6-oxo-1H-[1,4]-oxazino[3,4-a] [2]benzazépine-4-carboxylique.

7. Composé selon la revendication 1, dans lequel Z est -O-.

8. Composé selon la revendication 1, dans lequel Z est -S-.

9. Composé selon la revendication 1, dans lequel Z est -NH-.

10. Procédé pour la préparation d'un composé de formule (I'), selon l'une quelconque des revendications 1 à 5 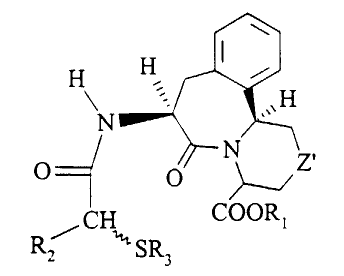 dans laquelle R₁, R₂ et R₃ sont tels que définis dans la revendication 1 et Z' représente (CH₂)ₙ dans laquelle n est un entier égal à 0 ou 1, qui comprend
a) si
R₁ est diphénylméthyle et
R₃ est acétyle ou benzoyle;
la réaction d'un composé de formule 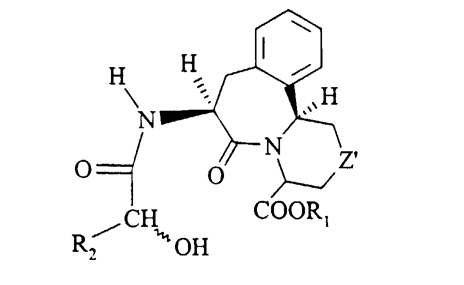 dans laquelle Z', R₁, n et R₂ sont tels que définis ci-dessus avec l'acide thioacétique ou l'acide thiobenzoïque en présence de triphénylphosphine et d'un agent oxydant;
b) si
R₁ est hydrogène et
R₃ est acétyle ou benzoyle; et R₂ et n sont tels que définis ci-dessus,
la réaction d'un composé de formule 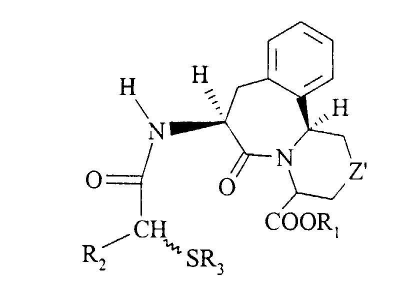 dans laquelle
R₁ est diphénylméthyle;
R₃, Z', n et R₂ sont tels que définis ci-dessus, avec un acide,
c) si
R₁ est hydrogène;
R₃ est hydrogène; et Z', R₂ et n sont tels que définis ci-dessus,
la réaction d'un composé de formule 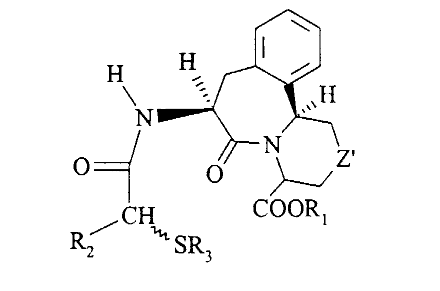 dans laquelle
R₃ est acétyle ou benzoyle; et
Z', R₁, n et R₂ sont tels que définis ci-dessus, avec une base,
d) si
R₁ est -CH₂O-C(O)C(CH₃)₃; alkyle en C₁-C₄; un groupe Ar-Y-, dans lequel Ar est aryle et Y est alkyle en C₀-C₄;
R₃ est acétyle ou benzoyle; et Z', R₂ et n sont tels que définis ci-dessus,
la réaction d'un composé de formule 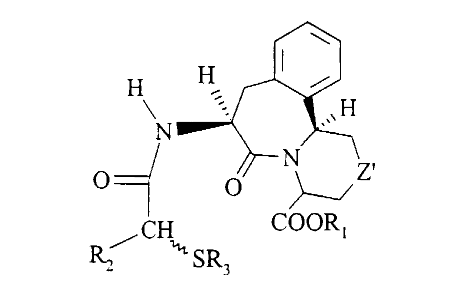 dans laquelle
R₁ est hydrogène;
Z', n, R₃ et R₂ sont tels que définis ci-dessus, en présence d'une base, avec un halogénure d'alkyle en C₁-C₄ approprié, le pivalate de chlorométhyle ou un halogénure de Ar-Y, dans lequel Ar et Y sont tels que définis ci-dessus,
e) si
R₁ est -CH₂O-C(O)C(CH₃)₃; alkyle en C₁-C₄; un groupe Ar-Y-, dans lequel Ar est aryle et Y est alkyle en C₀-C₄ ou diphénylméthyle;
R₃ est hydrogène; et Z', R₂ et n sont tels que définis ci-dessus,
la réaction d'un composé de formule 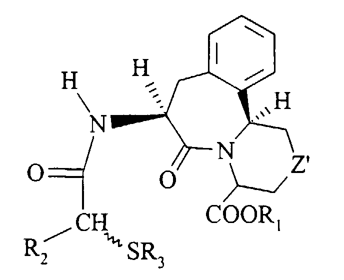 dans laquelle R₃ est acétyle ou benzoyle et R₁, Z', n et R₂ sont tels que définis ci-dessus avec de l'ammoniac ou
f) si
R₄ est hydrogène; -CH₂O-C(O)C(CH₃)₃; alkyle en C₁-C₄; un groupe Ar-Y-, dans lequel Ar est aryle et Y est alkyle en C₀-C₄ ou diphénylméthyle;
R₃ est -CH₂O-C(O)C(CH₃)₃; et Z', R₂ et n sont tels que définis ci-dessus,
la réaction d'un composé de formule 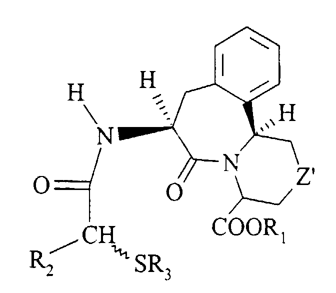 dans laquelle R₃ est hydrogène; et R₁, Z', n et R₂ sont tels que définis ci-dessus, en présence d'une base, avec le pivalate de chlorométhyle.

11. Procédé de préparation d'un composé de formule I" selon une quelconque des revendications 1 et 6 à 9 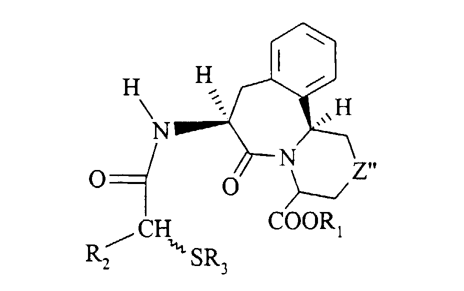
dans laquelle R₁, R₂ et R₃ sont tels que définis dans la revendication 1 et Z" est -O-, -S-, -NR₆- ou -N(COR₇)-,
dans laquelle R₆ est hydrogène, alkyle en C₁-C₄ ou un groupe Ar-Y- et R₇ est -CF_{3 ,} alkyle en C₁-C₁₀ ou un groupe Ar-Y-,
qui comprend
a) si
R₃ est acétyle ou benzoyle ;
R₁ est diphénylméthyle ; et
Z" est -O-, -S-, -NR₆-,
dans lequel R₆ est hydrogène et R₂ est tel que défini ci-dessus,
la réaction d'un composé de formule 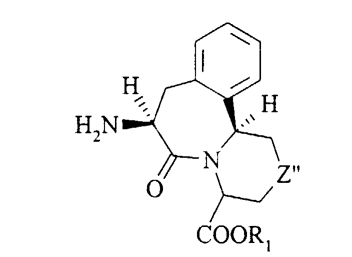 dans laquelle R₁ et Z" sont tels définis ci-dessus avec un composé de formule 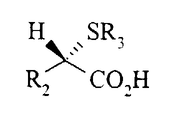 dans laquelle R₃ et R₂ sont tels que définis ci-dessus en présence d'un réactif de couplage,
b) si
R₃ est acétyle ou benzoyle;
R₁ est hydrogène et
Z" est -O-, -S-, 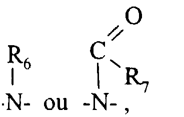 dans lequel R₆ est hydrogène, alkyle en C₁-C₄ ou un groupe Ar-Y-; et R₇ est -CF₃, alkyle en C₁-C₁₀ ou un groupe Ar-Y- et R₂ est tel que défini ci-dessus,
la réaction d'un composé de formule 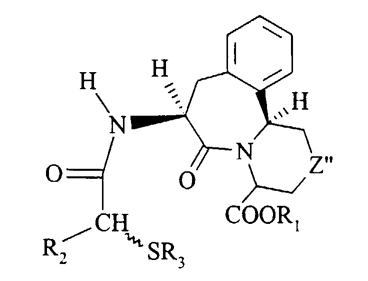 dans laquelle R₂, R₃ et Z'' sont tels que définis ci-dessus; et R₁ est diphénylméthyle avec un acide,
c) si
R₃ est hydrogène;
R₁ est hydrogène et
Z" est -O-, -S-, 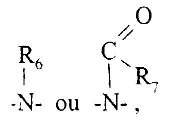 dans lequel R₆ est hydrogène, alkyle en C₁-C₄ ou un groupe Ar-Y-; et R₇ est -CF₃, alkyle en C₁-C₁₀ ou un groupe Ar-Y- et R₂ est tel que défini ci-dessus,
la réaction d'un composé de formule 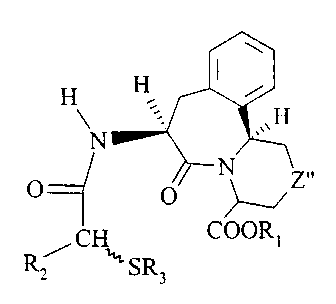 dans laquelle R₂, R₁ et Z" sont tels que définis ci-dessus; et R₃ est acétyle, avec une base,
d) si
R₃ est acétyle ou benzoyle;
R₁ est -CH₂O-C(O)(CH₃)₃; alkyle en C₁-C₄; un groupe Ar-Y-, dans lequel Ar est aryle et Y est alkyle en C₀-C₄ et
Z'' est -O-, -S-, 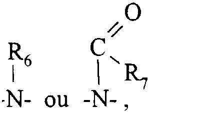 dans lequel R₆ est hydrogène, alkyle en C₁-C₄ ou un groupe Ar-Y-; et R₇ est -CF₃, alkyle en C₁-C₁₀ ou un groupe Ar-Y- et R₂ est tel que défini ci-dessus,
la réaction d'un composé de formule 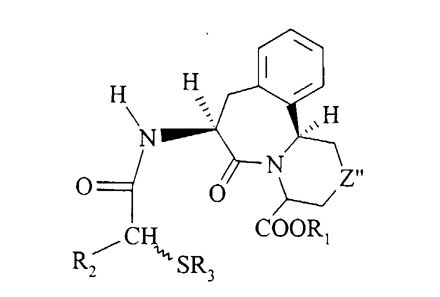 dans laquelle R₂, R₃ et Z" sont tels que définis ci-dessus; et R₁ est hydrogène, en présence d'une base, avec l'halogénure d'alkyle en C₁-C₄ approprié, le pivalate de chlorométhyle ou un halogénure de Ar-Y, dans lequel Ar et Y sont tels que définis ci-dessus,
e) si
R₃ est hydrogène;
R₁ est -CH₂O-C(O)(CH₃)₃; diphénylméthyle; alkyle en C₁-C₄; un groupe Ar-Y-, dans lequel Ar est aryle et Y est alkyle en C₀-C₄ et
Z" est -O-, -S-, 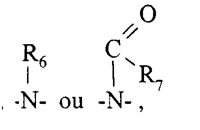 dans lequel R₆ est hydrogène, alkyle en C₁-C₄ ou un groupe Ar-Y-; et R₇ est -CF₃, alkyle en C₁-C₁₀ ou un groupe Ar-Y- et R₂ est tel que défini ci-dessus,
la réaction d'un composé de formule 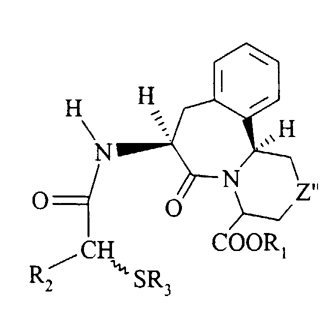 dans laquelle R₂, R₁ et Z" sont tels que définis ci-dessus; et R₃ est acétyle ou benzoyle avec de l'ammoniac,
f) si
R₃ est -CH₂O-C(O)C(CH₃)₃;
R₁ est hydrogène, -CH₂O-C(O)(CH₃)₃; diphénylméthyle; alkyle en C₁-C₄; un groupe Ar-Y-, dans lequel Ar est aryle et Y est alkyle en C₀-C₄ et
Z" est -O-, -S-, 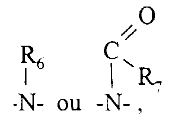 dans lequel R₆ est hydrogène, alkyle en C₁-C₄ ou un groupe Ar-Y-; et R₇ est -CF₃, alkyle en C₁-C₁₀ ou un groupe Ar-Y- et R₂ est tel que défini ci-dessus,
la réaction d'un composé de formule 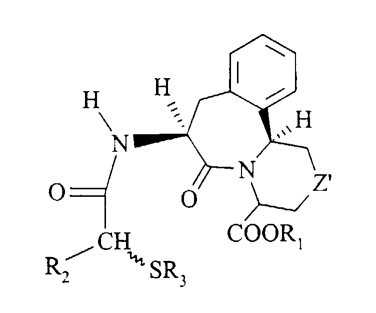 dans laquelle R₂, R₁ et Z' sont tels que définis ci-dessus; et R₃ est hydrogène, en présence d'une base, avec le pivalate de chlorométhyle.

12. Composé selon l'une quelconque des revendications 1 à 9 pour une utilisation en tant qu'agent pharmaceutique.

13. Composition comprenant une quantité dosable d'un composé selon l'une quelconque des revendications 1 à 9 en mélange ou en association avec un support inerte.

14. Composition pharmaceutique contenant un composé selon l'une quelconque des revendications 1 à 9 ou un mélange de ces composés et éventuellement un support et/ou diluant pharmaceutiquement acceptable.

15. Utilisation d'un composé selon l'une quelconque des revendications 1 à 9 pour la préparation d'une composition pharmaceutique pour une utilisation comme inhibiteur de l'enképhalinase.

16. Utilisation d'un composé selon l'une quelconque des revendications 1 à 9 pour la préparation d'une composition pharmaceutique pour une utilisation comme inhibiteur de l'ACE.

17. Utilisation d'un composé selon l'une quelconque des revendications 1 à 9 pour la préparation d'une composition pharmaceutique pour une utilisation dans le traitement de la douleur chronique ou aiguë, comme agent antihypertenseur, pour le traitement de l'insuffisance cardiaque congestive, pour le traitement de l'hypertrophie cardiaque ou comme diurétique.

18. Procédé pour la préparation d'une composition pharmaceutique telle que définie dans la revendication 14 comprenant la combinaison d'un composé tel que défini dans l'une quelconque des revendication 1 à 9 avec un support pharmaceutiquement acceptable.
